(19) Europäisches Patentamt
European Patent Office
Office européen des brevets

(11) EP 2 003 123 A2

(12) **EUROPEAN PATENT APPLICATION**

(43) Date of publication:
**17.12.2008 Bulletin 2008/51**

(21) Application number: **08010623.0**

(22) Date of filing: **11.06.2008**

(51) Int Cl.:
*C07D 235/14* (2006.01) *C07D 235/16* (2006.01)
*C07D 235/20* (2006.01) *C08G 61/12* (2006.01)
*C08G 73/06* (2006.01) *C08G 73/18* (2006.01)
*C08J 5/18* (2006.01) *C09D 165/00* (2006.01)
*C09D 179/04* (2006.01) *H01B 3/30* (2006.01)
*H01L 21/312* (2006.01) *H01L 23/532* (2006.01)
*H01L 21/768* (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **14.06.2007 JP 2007158015**
**21.06.2007 JP 2007164379**
**09.08.2007 JP 2007208644**
**25.06.2007 JP 2007167012**
**09.08.2007 JP 2007208645**

(71) Applicant: **Daicel Chemical Industries, Ltd.**
**Kita-ku,**
**Osaka-shi**
**Osaka 530-0001 (JP)**

(72) Inventors:
- **Funaki, Yoshinori**
**Himeji-shi**
**Hyogo 671-1283 (JP)**
- **Itaya, Ryo**
**Himeji-shi**
**Hyogo 671-1283 (JP)**
- **Takaragi, Akira**
**Himeji-shi**
**Hyogo 671-1283 (JP)**
- **Okamoto, Kazuki**
**Himeji-shi**
**Hyogo 671-1283 (JP)**
- **Torieda, Mayumi**
**Himeji-shi**
**Hyogo 671-1283 (JP)**

(74) Representative: **Grünecker, Kinkeldey, Stockmair & Schwanhäusser**
**Anwaltssozietät**
**Leopoldstrasse 4**
**80802 München (DE)**

(54) **Thin-film materials, thin films and producing method thereof**

(57) An N-substituted benzimidazole-containing bridged alicyclic compound is provided. The compound is represented by following Formula (1-1):

$$\left[ \left( R^a \right)_{m3} Z^3 \left\{ Y^{11} - A^3 - Y^2 \left( X^3 \right)_{k2} \right\}_{n4} \right] \quad (1-1)$$

In the formula, $Z^3$ represents a bridged alicyclic skeleton; $Y^{11}$ represents a single bond or a divalent organic group; $Y^2$ represents a single bond or a di- or tri-valent organic group; $X^3$ represents a hydrogen atom or a reactive functional group; $R^a$ represents a hydrogen atom or a hydrocarbon group; $A^3$ represents a group represented by one of following Formulae (a) and (b) :

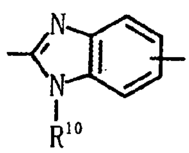
(a)

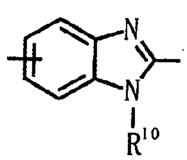
(b)

EP 2 003 123 A2

wherein $R^{10}$ represents a monovalent organic group, wherein, in each of Formulae (a) and (b), the left side is to be bonded to $Y^{11}$, and the right side is to be bonded to $Y^2$; "n4" denotes an integer of 2 to 7; "m3" denotes an integer of 0 to 5; and "k2" denotes an integer of 0 to 2, wherein the total of "n4" and "m3" equals 2 to 7, and wherein two or more $Y^{11}$s, $Y^2$s, $X^3$s, $A^3$s, and $R^{10}$s per molecule, and two or more $X^3$s and $R^a$s, if present per molecule, may be the same as or different from one another, respectively.

## Figure 1

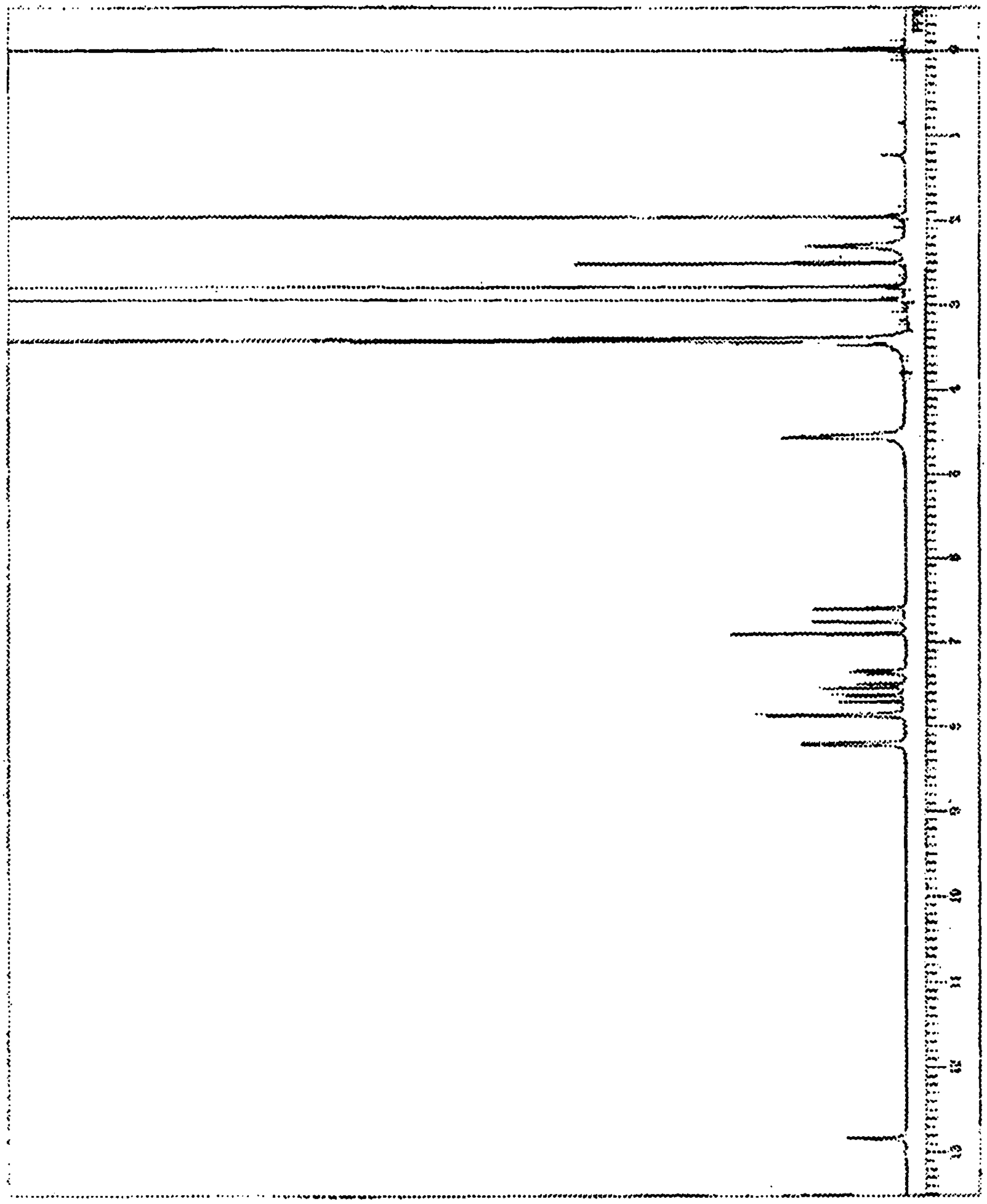

## Description

BACKGROUND OF THE INVENTION

1. Field of the Invention

The present invention relates to thin films such as insulating films for use typically in manufacture of semiconductors, particularly to thin films such as insulating films that excel in thermal stability or mechanical strength or have low moisture absorptivity, and exhibit a low relative dielectric constant; producing methods of these thin films; monomers useful for the production of the insulating films; polymers obtained from the monomers; polymerizable compounds; and materials for forming thin films such as insulating films and polymers having a pore structure, which contain these.

2. Description of the Related Art

Finer circuit patterns in recent semiconductor processes require lower dielectric constants of interlayer dielectric films. It is believed that construction of a pore structure is effective to allow interlayer dielectric films to have a lower dielectric constant. Typically, there has been proposed introduction of a pore structure typically with a foaming agent (blowing agent) into a silicon oxide interlayer dielectric film. This technique gives pores in the film, but inevitably causes binding of pores (connection or communication of pores), whereby the resulting film is inferior in mechanical strength and thermal stability. This causes serious problems, such as film disruption, in interconnection processes of semiconductor manufacture.

The present inventors found that an insulating film, if formed by polymerization of a polyfunctional crosslinkable monomer, contains pores at the molecular level and thereby has both a lower dielectric constant and a high mechanical strength (for example, see Japanese Unexamined Patent Application Publication (JP-A) No. 2004-307804). Insulating films formed according to this technique, however, often have varying dielectric constants, because large amounts of unreacted terminals remain therein. In addition, it is desirable to provide further lower relative dielectric constants in order to permit higher levels of integration of semiconductors.

SUMMARY OF THE INVENTION

An object of the present invention is to provide: thin films, such as insulating films, which have high thermal stability and a low relative dielectric constant, are thereby useful for manufacturing of semiconductors, and have a pore structure with low moisture absorptivity; producing methods of these thin films; monomers and polymers capable of forming these thin films; and materials for film production, containing these monomers or polymers.

Another object of the present invention is to provide: polymers and insulating films, each of which has a pore structure, has high thermal stability and a very low relative dielectric constant, thereby is useful for manufacturing of semiconductors, and shows less variation in relative dielectric constant; producing methods of these polymers and insulating films; and materials and polymerizable compounds capable of forming these insulating films and polymers.

After intensive investigations to achieve the above objects, the present inventors found that compounds and polymers each having a bridged alicyclic skeleton and an N-substituted benzimidazole ring give thin films having a low relative dielectric constant and exhibiting very low moisture absorptivity.

They also found that insulating films that have a very low relative dielectric constant and exhibit less variation in relative dielectric constant can be efficiently obtained by polymerization of an ethynyl-containing bridged alicyclic compound having a specific structure.

In addition, they found that insulating films that have a very low relative dielectric constant and exhibit less variation in relative dielectric constant can also be efficiently obtained by polymerization of two compounds which have two or more functional groups or moieties capable of binding with each other to form, for example, a heterocyclic ring and are capable of forming a polymer having a pore structure as a result of the reaction between the functional groups or moieties, in which at least one of the two compounds has a flexible unit with a specific structure; and/or polymerization of a compound which has, per molecule, two or more functional groups or moieties capable of reacting with each other to form, for example, a heterocyclic ring, which is capable of forming a polymer having a pore structure as a result of the reaction between the functional groups or moieties, and which intramolecularly has a flexible unit with a specific structure. The present invention has been made based on these findings.

Specifically, according to the present invention, an N-substituted benzimidazole-containing bridged alicyclic compound is provided. The compound is represented by following Formula (1-1):

$$\left( R^a \right)_{m3} Z^3 \left[ Y^{11} - A^3 - Y^2 \left( X^3 \right)_{k2} \right]_{n4} \qquad (1-1)$$

In Formula (1-1), $Z^3$ represents a bridged alicyclic skeleton; $Y^{11}$ represents a single bond or a divalent organic group; $Y^2$ represents a single bond or a di- or tri-valent organic group; $X^3$ represents a hydrogen atom or a reactive functional group; $R^a$ represents a hydrogen atom or a hydrocarbon group; $A^3$ represents a group represented by one of following Formulae (a) and (b):

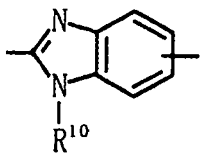
(a)

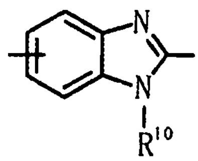
(b)

In the Formulae (a) and (b), $R^{10}$ represents a monovalent organic group, in each of Formulae (a) and (b), the left side is to be bonded to $Y^{11}$, and the right side is to be bonded to $Y^2$; "n4" denotes an integer of 2 to 7; "m3" denotes an integer of 0 to 5; and "k2" denotes an integer of 0 to 2. The total of "n4" and "m3" equals 2 to 7. Two or more $Y^{11}$s, $Y^2$s, $X^3$s, $A^3$s, and $R^{10}$s per molecule, and two or more $X^3$s and $R^a$s, if present per molecule, may be the same as or different from one another, respectively.

Preferably, a bridged alicyclic ring constituting the bridged alicyclic skeleton as $Z^3$ include rings represented by the following formulae, and rings each composed of two or more of these rings bonded to each other:

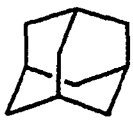
(2a)

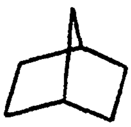
(2b)

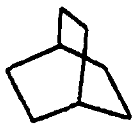
(2c)

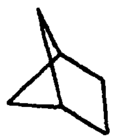
(2d)

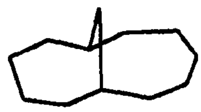
(2e)

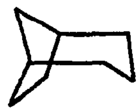
(2f)

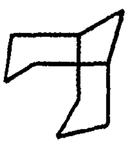
(2g)

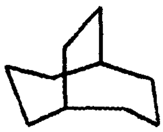
(2h)

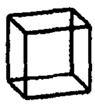
(2i)

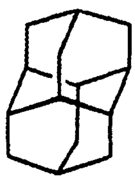
(2j)

Preferably, the monovalent organic group as $R^{10}$ includes an aliphatic hydrocarbon group, an alicyclic hydrocarbon group, an aromatic hydrocarbon group, and a group composed of two or more of these groups bonded to each other with or without the interposition of at least one of oxygen atom and sulfur atom. Preferably, the monovalent organic group as $R^{10}$ includes groups represented by the following formulae:

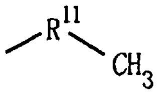
(31a)

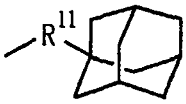
(31b)

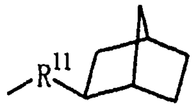
(31c)

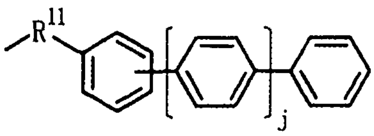
(31d)

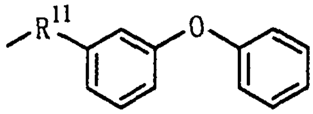
(31e)

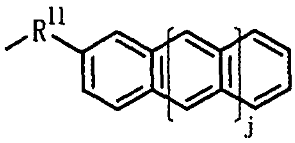
(31f)

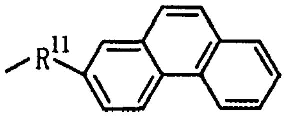
(31g)

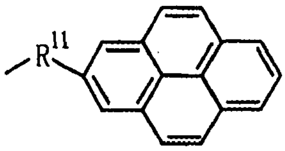
(31h)

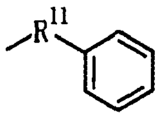
(31i)

wherein $R^{11}$ represents a single bond or a divalent aliphatic hydrocarbon group having one to fifty carbon atoms; and "j" denotes an integer of 0 to 3.

Preferably, the divalent organic groups as $Y^{11}$ and $Y^2$ include an alkylene group, an alkenylene group, an alkynylene group, a divalent alicyclic hydrocarbon group, an arylene group, a divalent heterocyclic group, a group composed of two or more of these divalent organic groups bonded to each other, and a group composed of one or more of these divalent organic groups bonded to at least one atom selected from oxygen atom (-O-) and sulfur atom (-S-). Preferably, the divalent organic groups as $Y^{11}$ and $Y^2$ include divalent groups represented by the following formulae, and divalent groups each composed of two or more of these groups bonded to each other:

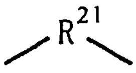 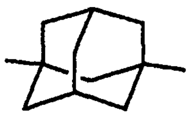 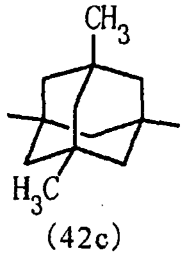 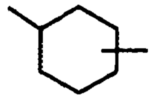 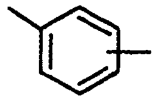

(42a) (42b) (42c) (42d) (42e)

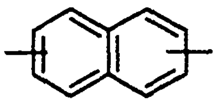 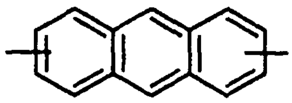 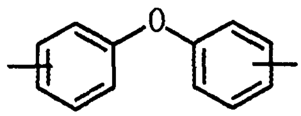

(42f) (42g) (42h)

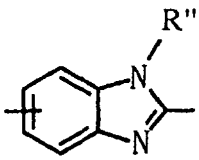 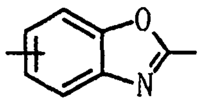 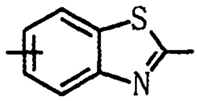

(42i) (42j) (42k)

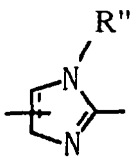 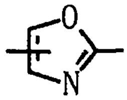 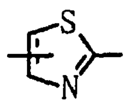

(42l) (42m) (42n)

wherein $R^{21}$ represents a divalent aliphatic hydrocarbon group having one to fifty carbon atoms; and R" represents a hydrogen atom or a monovalent organic group, wherein the left and right bonds in these formulae may direct to the left and right sides or to the right and left sides, respectively, in Formula (1-1).

Preferably, the reactive functional group as $X^3$ include a substituted or unsubstituted ethynyl group, a substituted or unsubstituted vinyl group, a halogen atom, an unsubstituted or mono-substituted amino group, a haloformyl group, acid anhydride group, acid azido group, hydrazido group, cyano group, an acyl group, carboxyl group, a substituted oxycarbonyl group, hydroxyl group, mercapto group, an imino group, and an alkoxysilyl group.

Additionally, according to the present invention, an N-substituted benzimidazole-containing polymer is provided as a polymerization product of any one of the above-mentioned N-substituted benzimidazole-containing bridged alicyclic compounds with "k2" in Formula (1-1) being 1 or 2. The N-substituted benzimidazole-containing bridged alicyclic compounds with "k2" in Formula (1-1) being 1 or 2 is referred to a compound A'.

Additionally, according to the present invention, an N-substituted benzimidazole-containing polymer is provided as a reaction product between the compound A' and a compound B'. The compound B' is a polyfunctional compound containing two or more functional groups or moieties capable of reacting with the reactive functional group $X^3$ of the compound A'.

Additionally, according to the present invention, there is provided an N-substituted benzimidazole-containing polymer including a repeating unit represented by any one of following Formulae (51a), (51b) and (51c):

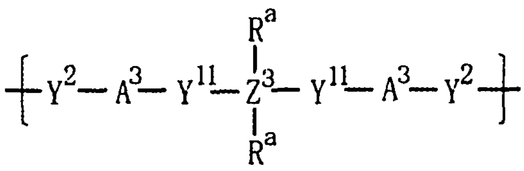

(51a)

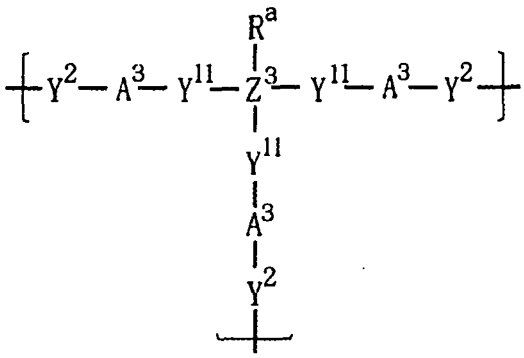

(51b)

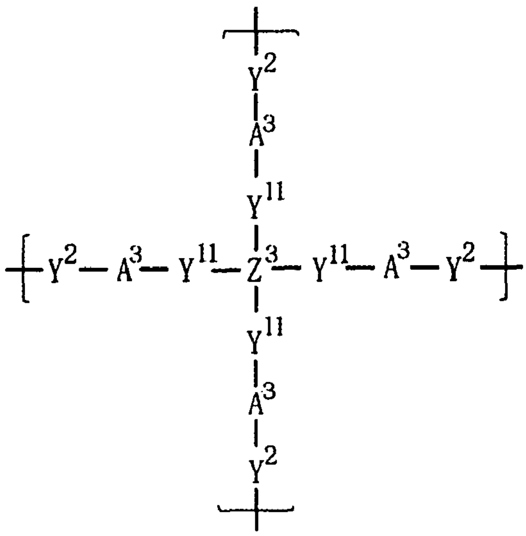

(51c)

wherein $Z^3$ represents a bridged alicyclic skeleton; $Y^{11}$ represents a single bond or a divalent organic group; $Y^2$ represents a single bond or a divalent organic group; $R^a$ represents a hydrogen atom or a hydrocarbon group; and $A^3$ represents a group represented by one of following Formulae

(a) and (b) :

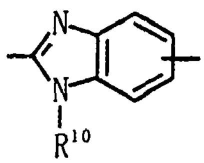

(a)

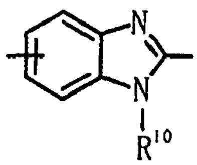

(b)

wherein $R^{10}$ represents a monovalent organic group, wherein the left side is to be bonded to $Y^{11}$, and the right side is

to be bonded to $Y^2$ in each of Formulae (a) and (b).

Preferably, a weight-average molecular weight of the polymer is about 200 to 100000.

Additionally, in the present invention, a material for producing a film, comprising the N-substituted benzimidazole-containing bridged alicyclic compounds, is provided. The N-substituted benzimidazole-containing bridged alicyclic compound is preferably dissolved in a solvent.

Additionally, in the present invention, a material for producing a film comprises a compound A' and B' both dissolved in a solvent. The compound A' is any of the above-mentioned N-substituted benzimidazole-containing bridged alicyclic compounds with "k2" in Formula (1-1) being 1 or 2, and the compoumd B' is a polyfunctional compound containing two or more functional groups or moieties capable of reacting with the reactive functional group $X^3$ of the compound A'.

Additionally, in the present invention, a material for producing a film, having the above-mentioned N-substituted benzimidazole-containing polymer dissolved in a solvent, is provided.

Additionally, in the present invention, a method of producing a thin film is provided. The method includes a step of applying any one of the above-mentioned materials to a substrate and a step of drying the applied material or carrying out a reaction of the applied material by heating, to give a thin film.

Additionally, in the present invention, a thin film produced by the above-mentioned method is provided.

Additionally, in the present invention, an ethynyl-containing bridged alicyclic compound is provided. The compound is represented by following Formula (1):

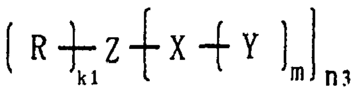

(1)

In Formula (1), Z represents a bridged alicyclic skeleton; X represents a divalent or higher-valent organic group containing a heterocyclic ring or a precursor structure thereof; Y represents a substituted or unsubstituted ethynyl-containing group; R represents a hydrogen atom or a hydrocarbon group; "m" denotes an integer of 1 to 5; "n3" denotes an integer of 2 to 7; and "k1" denotes an integer of 0 to 5, wherein the total of "n3" and "k1" equals 2 to 7, and wherein two or more Xs and Ys per molecule, and two or more Rs, if present per molecule, may be the same as or different from each other, respectively.

Preferably, a bridged alicyclic ring constituting the bridged alicyclic skeleton as Z includes rings represented by the following formulae, and rings are each composed of two or more of these rings bonded to each other:

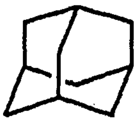

(2a)

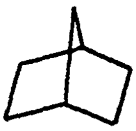

(2b)

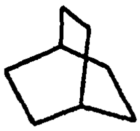

(2c)

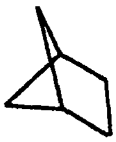

(2d)

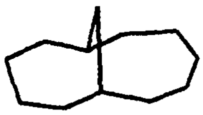

(2e)

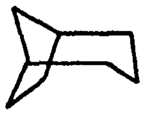

(2f)

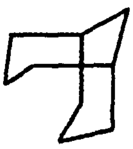

(2g)

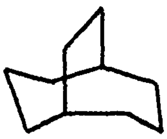

(2h)

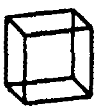

(2i)

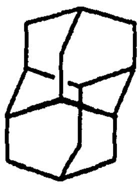

(2j)

Preferably, the organic group represented by X includes an imidazolyl group, benzimidazolyl group, oxazolyl group, benzoxazolyl group, thiazolyl group, benzothiazolyl group, a precursor group of any of these heterocyclic groups, a group composed of two or more of these heterocyclic groups or their precursor groups bonded to each other, and a group composed of one or more of these heterocyclic groups or their precursor groups bonded to one or more aromatic hydrocarbon groups.

Preferably, the organic group represented by X includes groups represented by following formulae, and groups

each composed of two or more of these groups bonded to each other:

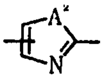
(3a)
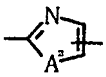
(3b)
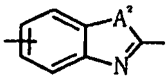
(3c)
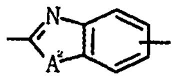
(3d)
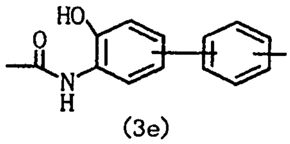
(3e)

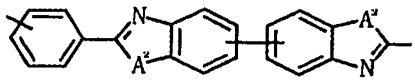
(3f)
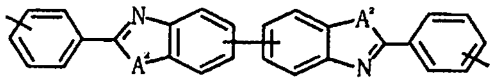
(3g)

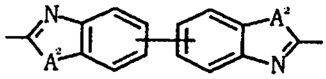
(3h)
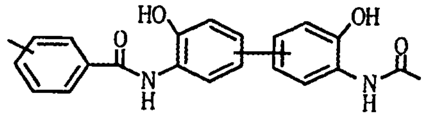
(3i)

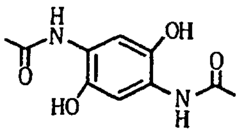
(3j)
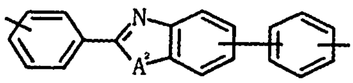
(3k)
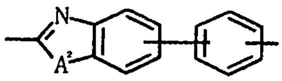
(3l)

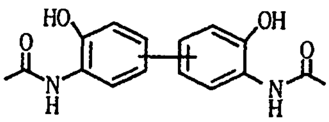
(3m)
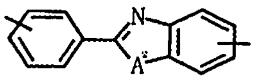
(3n)
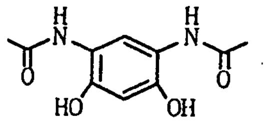
(3o)

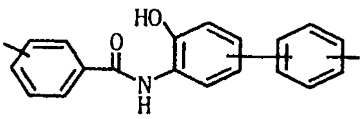
(3p)
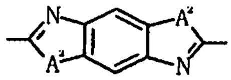
(3q)
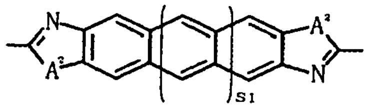
(3r)

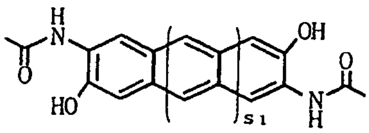
(3s)
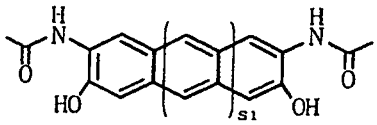
(3t)

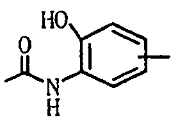
(3u)
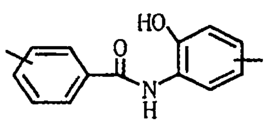
(3v)

wherein $A^2$ represents -NH-, oxygen atom, or sulfur atom; and "s1" denotes an integer of 0 to 5, and wherein each of rings in the formulae may have one or more substituents.

Additionally, in the present invention, a material for producing an insulating film is provided. The material contains any of the above-mentioned ethynyl-containing bridged alicyclic compounds.

Preferably, the material may further contain one or more other ethynyl-containing compounds, in addition to the ethynyl-containing bridged alicyclic compound.

Preferably, the material may be a solution including the ethynyl-containing bridged alicyclic compound dissolved in an organic solvent.

Additionally, in the present invention, there is provided a polymer obtained by a polymerization of any of the materials for producing the insulating film. The polymer has a pore structure.

Additionally, in the present invention, an insulating film including the polymer, having a pore structure, is provided.

Additionally, in the present invention, a producing method of an insulating film includes the steps of applying the above-mentioned material for producing the insulating film to a substrate; and carrying out polymerization of the applied material to form an insulating film containing a polymer having a pore structure.

Further, in the present invention, a material for producing an insulating film includes a pair of compounds A and B, and/or a compound C. The pair of the compounds A and B each contain two or more functional groups or moieties per molecule and form a polymer having a pore structure as a result of polymerization through binding of the functional group or moiety of one compound with the functional group or moiety of the other compound. The compound C contains two or more functional groups or moieties per molecule and forms a polymer having a pore structure as a result of polymerization through binding of one of the functional group or moiety with the other of the functional group or moiety.

The pair of the compounds A and B, and/or the compound C satisfy the following condition (i) or (ii):

(i) at least one of the compounds A and B contains a bridged alicyclic skeleton or an aromatic skeleton as a central skeleton,

at least one of the compounds A and B has a thermally stable skeleton positioned between the central skeleton and the functional groups or moieties and the thermally stable skeleton is composed of an aromatic-ring-containing divalent organic group,
at least one of the compounds A and B intramolecularly has a flexible unit composed of an organic group containing at least an alkylene group or ether bond and having a total of two to twenty atoms, and
the functional groups or moieties of the compound A and the functional groups or moieties of the compound B constitute a pair of functional groups or moieties capable of reacting with each other to form a heterocyclic ring, or the functional groups or moieties of the compound A and the functional groups or moieties of the compound B are both substituted or unsubstituted ethynyl-containing groups; or

(ii) the compound C contains a bridged alicyclic skeleton or an aromatic skeleton as a central skeleton,

the compound C has a thermally stable skeleton composed of an aromatic-ring-containing divalent organic group and positioned between the central skeleton and the one of the functional group or moiety and/or between the central skeleton and the other of the functional group or moiety,
the compound C has a flexible unit between the central skeleton and the one of the functional group or moiety and/or between the central skeleton and the other of the functional group or moiety, the flexible unit composed of an organic group containing at least an alkylene group or ether bond and having a total of two to twenty atoms, and
the one the functional group or moiety and the other of the functional group or moiety of the compound C constitute a pair of functional groups or moieties capable of reacting with each other to form a heterocyclic ring, or are both substituted or unsubstituted ethynyl-containing groups.

Preferably, a bridged alicyclic ring or aromatic ring constituting the bridged alicyclic skeletons or aromatic skeletons as the central skeleton of at least one of the compounds A and B and that of the compound C include rings represented by the following formulae, and rings each composed of two or more of these rings bonded to each other:

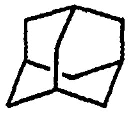
(4a)

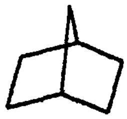
(4b)

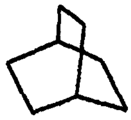
(4c)

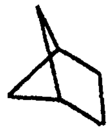
(4d)

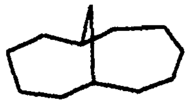
(4e)

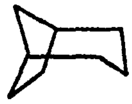
(4f)

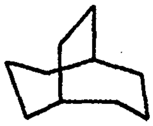
(4g)

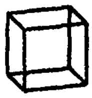
(4h)

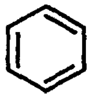
(4i)

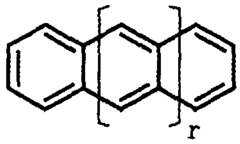
(4j)

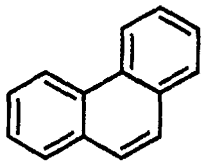
(4k)

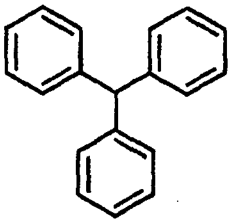
(4l)

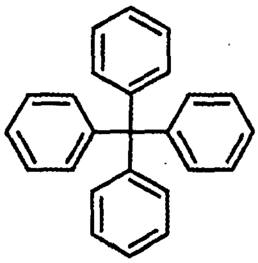
(4m)

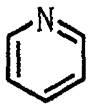
(4n)

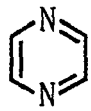
(4o)

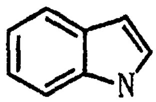
(4p)

wherein "r" denotes an integer of 0 to 5.

Preferably, the thermally stable skeleton of at least one of the compounds A and B, or the thermally stable skeleton of the compound C include groups represented by the following formulae, or groups each composed of two or more of these groups bonded to each other:

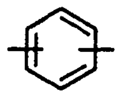

(5a)

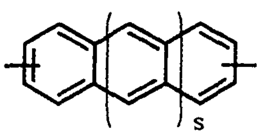

(5b)

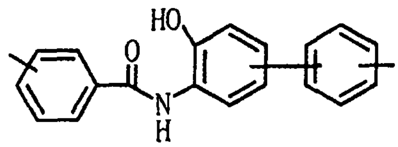

(5c)

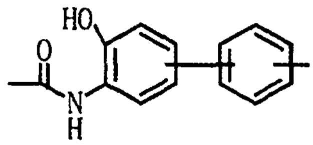

(5d)

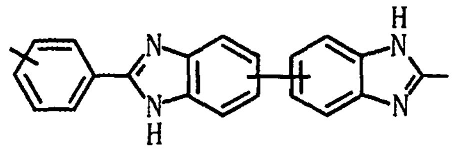

(5e)

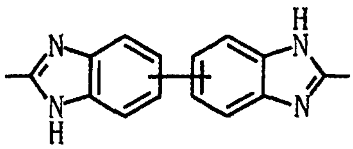

(5f)

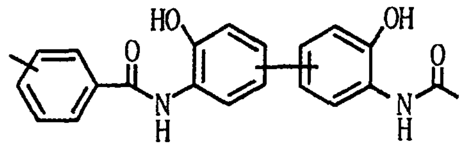

(5g)

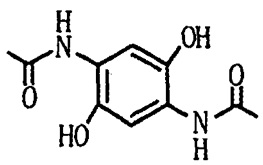

(5h)

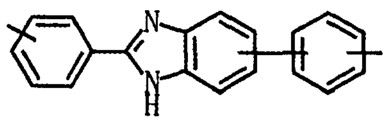

(5i)

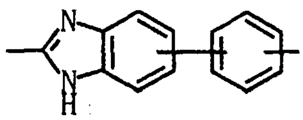

(5j)

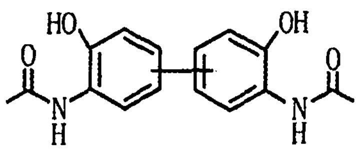

(5k)

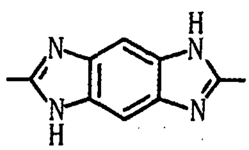

(5l)

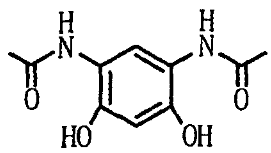

(5m)

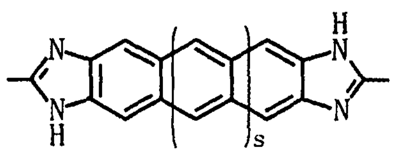

(5n)

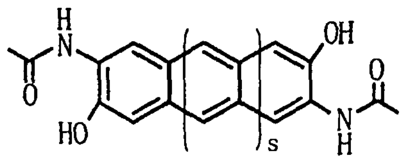

(5o)

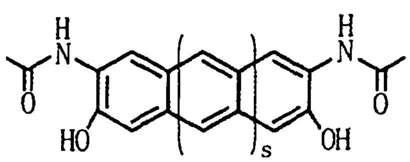

(5p)

wherein "s" denotes an integer of 0 to 5.

Preferably, the flexible unit of at least one of the compounds A and B, and the flexible unit of the compound C are each preferably a flexible unit composed of a group represented by any one of the following formulae:

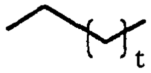

(6a)

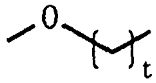

(6b)

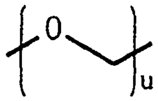

(6c)

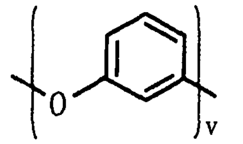

(6d)

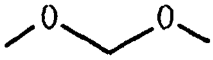

(6e)

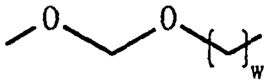

(6f)

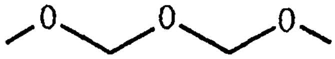

(6g)

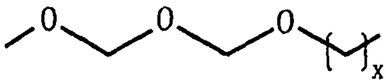

(6h)

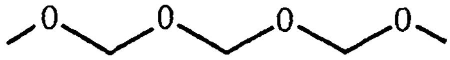

(6i)

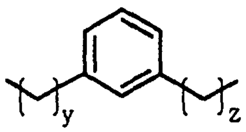

(6j)

wherein "t" denotes an integer of 1 to 19; "u" denotes an integer of 1 to 10; "v" denotes an integer of 1 to 3; "w" denotes an integer of 1 to 16; "x" denotes an integer of 1 to 14; and each of "y" and "z" independently denotes an integer of 0 to 6, wherein both of "y" and "z" are not simultaneously zero (0).

Preferably, the heterocyclic ring formed as a result of a reaction between the functional groups or moieties of the compound A and the functional groups or moieties of the compound B, or the heterocyclic ring formed as a result of a reaction between the one of the functional group or moiety and the other of the functional group or moiety of the compound C include, for example, benzimidazole ring, benzoxazole ring, and benzothiazole ring.

Preferably, the compounds A, B and C satisfy the following condition (iii) or (iv):

(iii) the compound A is a compound represented by following Formula (1a) or Formula (1b), and the compound B is a compound represented by following Formula (2):

Formula (1a):

$$\left[R^1\right]_{n2} X^1 \left[Y^{1a} - W^1 - Y^{1b} - Z^1\right]_{n1} \qquad (1a)$$

wherein $X^1$ represents a di-, tri-, or tetra-valent bridged alicyclic group or aromatic group; $Y^{1a}$ and $Y^{1b}$ are the same as or different from each other and each represent a single bond, a divalent aromatic hydrocarbon group, a divalent heteroaromatic group, a divalent group corresponding to a precursor of the divalent heteroaromatic group, or a divalent group composed of two or more of these groups bonded to each other; $W^1$ represents a flexible unit composed of a divalent group containing at least an alkylene group or ether bond and having a total of two to twenty atoms; $Z^1$ represents a functional group or moiety capable of reacting with $Z^2$ in following Formula (2) to form a heterocyclic ring, or, only when $Z^2$ in Formula (2) is a substituted or unsubstituted ethynyl-containing group, $Z^1$ may represent a substituted or unsubstituted ethynyl-containing group; $R^1$ represents a hydrogen atom or a hydrocarbon

group; "n1" denotes an integer of 2 to 4; and "n2" denotes an integer of 0 to 2, wherein the total of "n1" and "n2" equals 2 to 4, and wherein two or more $Y^{1a}$s, $Y^{1b}$s, $W^1$s, and $Z^1$s per molecule and two or more $R^1$s, if present per molecule, may be the same as or different from each other, respectively, or

Formula (1b):

$$W\left(Y^1-Z^1\right)_n \qquad (1b)$$

wherein $Y^1$ represents a single bond, a divalent aromatic hydrocarbon group, a divalent heteroaromatic group, a divalent group corresponding to a precursor of the divalent heteroaromatic group, or a divalent group composed of two or more of these groups bonded to each other; W represents a flexible unit composed of a di-, tri-, or tetra-valent group containing at least an alkylene group or ether bond and having a total of two to twenty atoms; $Z^1$ represents a functional group or moiety capable of reacting with $Z^2$ in following Formula (2) to form a heterocyclic ring, or, only when $Z^2$ in Formula (2) is a substituted or unsubstituted ethynyl-containing group, $Z^1$ may represent a substituted or unsubstituted ethynyl-containing group; and "n" denotes an integer of 2 to 4, wherein two or more $Y^1$s and $Z^1$s per molecule may be the same as or different from each other, respectively, and

Formula (2):

$$\left(\left(R^2\right)_{m2}X^2\right)_i\left(Y^{2a}\left(W^2-Y^{2b}\right)_k Z^2\right)_{m1} \qquad (2)$$

wherein $X^2$ represents a di-, tri-, or tetra-valent bridged alicyclic group or aromatic group; $Y^{2a}$ and $Y^{2b}$ are the same as or different from each other and each represent a single bond, a divalent aromatic hydrocarbon group, a divalent heteroaromatic group, a divalent group corresponding to a precursor of the divalent heteroaromatic group, or a divalent group composed of two or more of these groups bonded to each other; $W^2$ represents a flexible unit composed of a divalent group containing at least an alkylene group or ether bond and having a total of two to twenty atoms; $Z^2$ represents a functional group or moiety capable of reacting with $Z^1$ in Formula (1a) or (1b) to form a heterocyclic ring, or, only when $Z^1$ in Formula (1a) or (1b) is a substituted or unsubstituted ethynyl-containing group, $Z^2$ may represent a substituted or unsubstituted ethynyl-containing group; $R^2$ represents a hydrogen atom or a hydrocarbon group; "m1" denotes an integer of 2 to 4; "m2" denotes an integer of 0 to 2, wherein the total of "m1" and "m2" equals 2 to 4; "i" denotes 0 or 1; and "k" denotes 0 or 1, wherein two or more $Y^{2a}$s, $Y^{2b}$s, $W^2$s, and $Z^2$s per molecule and two or more $R^2$s, if present per molecule, may be the same as or different from each other, respectively,

(iv) the compound C is a compound represented by following Formula (3):

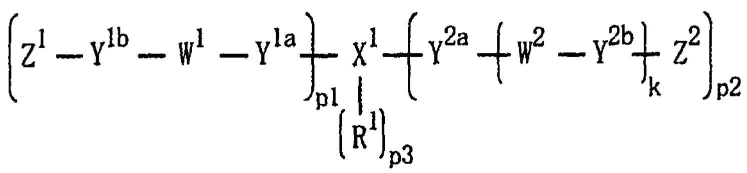

(3)

wherein $X^1$ represents a di-, tri-, or tetra-valent bridged alicyclic group or aromatic group; $Y^{1a}$, $Y^{1b}$, $Y^{2a}$, and $Y^{2b}$ are the same as or different from one another and each represent a single bond, a divalent aromatic hydrocarbon group, a divalent heteroaromatic group, a divalent group corresponding to a precursor of the divalent heteroaromatic group, or a divalent group composed of two or more of these groups bonded to each other; $W^1$ and $W^2$ are the same as or different from each other and each represent a flexible unit composed of a divalent group containing at least an alkylene group or ether bond and having a total of two to twenty atoms; $Z^1$ and $Z^2$ are a pair of functional groups or moieties capable of reacting with each other to form a heterocyclic ring, or $Z^1$ and $Z^2$ are both substituted or unsubstituted ethynyl-containing groups; $R^1$ represents a hydrogen atom or a hydrocarbon group; "k" denotes 0 or 1; each of "p1" and "p2" independently

denotes an integer of 1 to 3; and "p3" denotes an integer of 0 to 2, wherein the total of "p1", "p2", and "p3" equals 2 to 4, and wherein two or more $Y^{1a}$s, $Y^{1b}$s, $Y^{2a}$s, $Y^{2b}$s, $W^1$s, $W^2$s, $Z^1$s, $Z^2$s, and $R^1$s, if present per molecule, may be the same as or different from each other, respectively.

$X^1$ in Formula (1a) and Formula (3) may be a di-, tri-, or tetra-valent alicyclic group or aromatic group.

Preferably, the alicyclic ring or aromatic cyclic ring constituting the di-, tri-, or tetra-valent alicyclic group or aromatic group as $X^1$ include rings represented by the following formulae, and rings each composed of two or more of these rings bonded to each other:

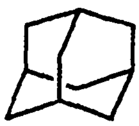
(4a)

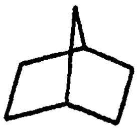
(4b)

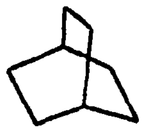
(4c)

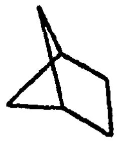
(4d)

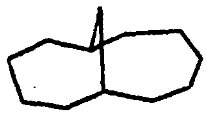
(4e)

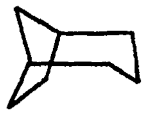
(4f)

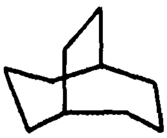
(4g)

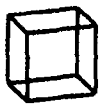
(4h)

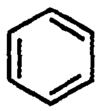
(4i)

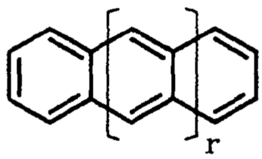
(4j)

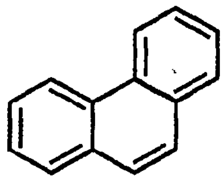
(4k)

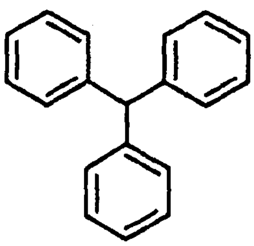
(4l)

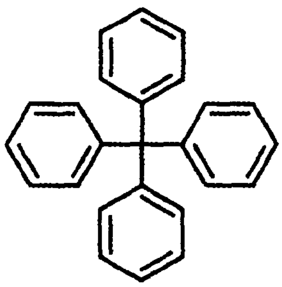
(4m)

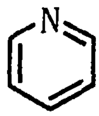
(4n)

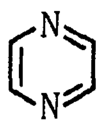
(4o)

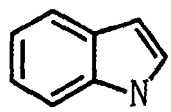
(4p)

wherein "r" denotes an integer of 0 to 5.

Each of $Y^{1a}$, $Y^{1b}$, $Y^1$, $Y^{2a}$, and $Y^{2b}$ in Formulae (1a), (1b), (2), and (3) is preferably independently a single bond, or a group represented by any one of the following formulae, or a group composed of two or more of these groups bonded to each other:

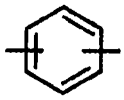
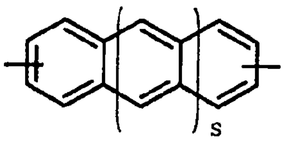
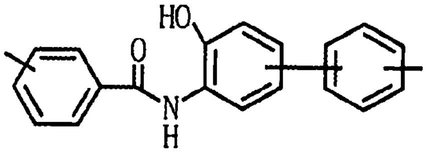

(5a) (5b) (5c)

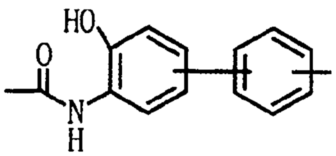
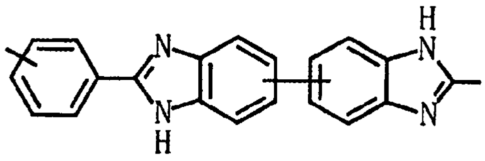

(5d) (5e)

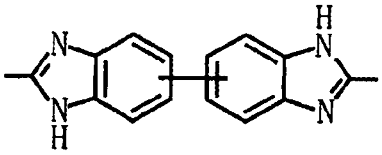
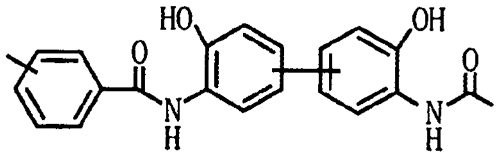

(5f) (5g)

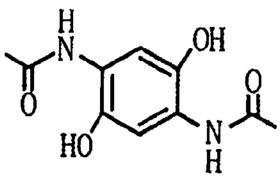
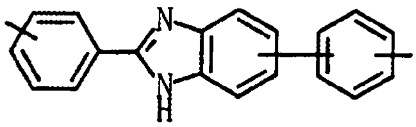
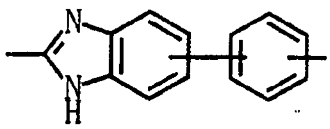

(5h) (5i) (5j)

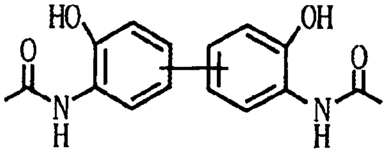
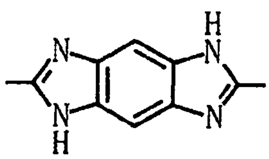
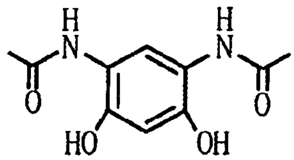

(5k) (5l) (5m)

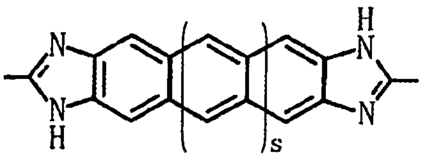
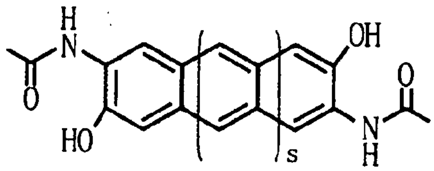

(5n) (5o)

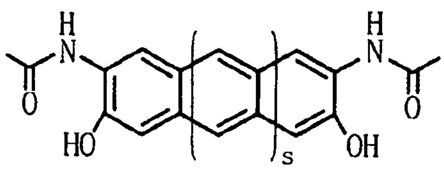

(5p)

wherein "s" denotes an integer of 0 to 5.

Preferably, each of $W^1$, W, and $W^2$ in Formulae (1a), (1b), (2), and (3) is independently a flexible unit including a group represented by any one of the following formulae:

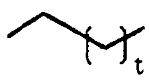

(6a)

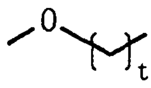

(6b)

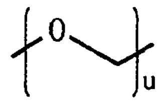

(6c)

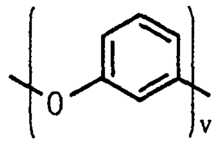

(6d)

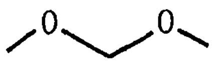

(6e)

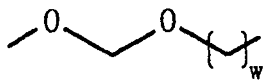

(6f)

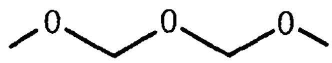

(6g)

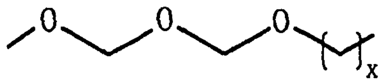

(6h)

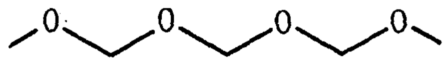

(6i)

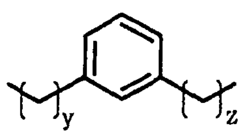

(6j)

wherein "t" denotes an integer of 1 to 19; "u" denotes an integer of 1 to 10; "v" denotes an integer of 1 to 3; "w" denotes an integer of 1 to 16; "x" denotes an integer of 1 to 14; and each of "y" and "z" independently denotes an integer of 0 to 6, wherein both of "y" and "z" are not simultaneously zero (0).

Preferably, the heterocyclic ring formed as a result of a reaction between $Z^1$ in Formula (1a) or (1b) and $Z^2$ in Formula (2), and/or the heterocyclic ring formed as a result of a reaction between $Z^1$ in Formula (3) and $Z^2$ in Formula (3) include, for example, benzimidazole ring, benzoxazole ring, and benzothiazole ring.

Preferably, one of $Z^1$ in Formula (1a) or (1b) and $Z^2$ in Formula (2), or one of $Z^1$ and $Z^2$ in Formula (3) is a carboxyl group, a substituted oxycarbonyl group, formyl group, a haloformyl group, or a substituted or unsubstituted ethynyl-containing group, and the other of $Z^1$ in Formula (1a) or (1b) and $Z^2$ in Formula (2), or one of $Z^1$ and $Z^2$ in Formula (3) is 3,4-diaminophenyl group, 3-amino-4-hydroxyphenyl group, 4-amino-3-hydroxyphenyl group, 3-amino-4-mercaptophenyl group, 4-amino-3-mercaptophenyl group, or a substituted or unsubstituted ethynyl-containing group, wherein, when the one is a substituted or unsubstituted ethynyl-containing group, the other is also a substituted or unsubstituted ethynyl-containing group.

Preferably, the material for producing insulating film include a solution of the compounds A and B, and/or the compound C dissolved in an organic solvent.

Additionally, in the present invention, a polymer obtained by a polymerization of any of the materials for producing the insulating film, and having a pore structure, is provided.

Additionally, in the present invention, an insulating film, including the polymer having a pore structure, is provided.

Additionally, in the present invention, a method of producing an insulating film is provided. The method includes the steps of applying the material for producing the insulating film to a substrate; and carrying out polymerization of the applied material to form an insulating film composed of a polymer having a pore structure.

Additionally, in the present invention, a polymerizable compound is represented by following Formula (7):

$$\left[R^1\right]_{n2} X^1 \left[Y^{1a} - W^1 - Y^{1b} - Z^1\right]_{n1} \qquad (7)$$

wherein $X^1$ represents a di-, tri-, or tetra-valent aromatic or non-aromatic cyclic group; $Y^{1a}$ and $Y^{1b}$ are the same as or different from each other and each represent a single bond, a divalent aromatic hydrocarbon group, a divalent heteroaromatic group, a divalent group corresponding to a precursor of the divalent heteroaromatic group, or a divalent group composed of two or more of these groups bonded to each other, wherein at least one of $Y^{1a}$ and $Y^{1b}$ is a divalent heteroaromatic group or a group containing a divalent group corresponding to a precursor of the divalent heteroaromatic group; $W^1$ represents a flexible unit composed of a divalent group containing at least an alkylene group or ether bond and having a total of two to twenty atoms; $Z^1$ represents carboxyl group, a substituted oxycarbonyl group, formyl group, a haloformyl group, a substituted or unsubstituted ethynyl-containing group, 3,4-diaminophenyl group, 3-amino-4-hydroxyphenyl group, 4-amino-3-hydroxyphenyl group, 3-amino-4-mercaptophenyl group, or 4-amino-3-mercaptophenyl group; $R^1$ represents a hydrogen atom or a hydrocarbon group; "n1" denotes an integer of 2 to 4; and "n2" denotes an integer of 0 to 2, wherein the total of "n1" and "n2" equals 2 to 4, and wherein two or more $Y^{1a}$s, $Y^{1b}$s, $W^1$s, and $Z^1$s per molecule and two or more $R^1$s, if present per molecule, may be the same as or different from each other, respectively.

Prefererably, at least one of $Y^{1a}$ and $Y^{1b}$ is a divalent heteroaromatic group containing at least one of benzimidazole ring, benzoxazole ring, and benzothiazole ring, or a divalent group corresponding to a precursor of the divalent heteroaromatic group.

Additionally, in the present invention, a polymerizable compound is represented by following Formula (8):

$$W \left[Y^1 - Z^1\right]_n \qquad (8)$$

wherein $Y^1$ represents a divalent heteroaromatic group or a divalent group corresponding to a precursor of the divalent heteroaromatic group; W represents a flexible unit composed of a di-, tri-, or tetra-valent group containing at least an alkylene group or ether bond and having a total of two to twenty atoms; $Z^1$ represents a carboxyl group, a substituted oxycarbonyl group, formyl group, a haloformyl group, a substituted or unsubstituted ethynyl-containing group, 3,4-diaminophenyl group, 3-amino-4-hydroxyphenyl group, 4-amino-3-hydroxyphenyl group, 3-amino-4-mercaptophenyl group, or 4-amino-3-mercaptophenyl group; and "n" denotes an integer of 2 to 4, wherein two or more $Y^1$s and $Z^1$s per molecule may be the same as or different from each other, respectively.

Preferably, $Y^1$s are each a divalent heteroaromatic group containing at least one of benzimidazole ring, benzoxazole ring, and benzothiazole ring, or a divalent group corresponding to a precursor of the divalent heteroaromatic group.

Additionally, in the present invention, a polymerizable compound is represented by following Formula (9):

$$\left[Z^1 - Y^{1b} - W^1 - Y^{1a}\right]_{p1} \underset{\left[R^1\right]_{p3}}{\overset{}{X^1}} \left[Y^{2a} \left[W^2 - Y^{2b}\right]_k Z^2\right]_{p2} \qquad (9)$$

wherein $X^1$ represents a di-, tri-, or tetra-valent organic group; $Y^{1a}$, $Y^{1b}$, $Y^{2a}$, and $Y^{2b}$ are the same as or different from one another and each represent a single bond, a divalent aromatic hydrocarbon group, a divalent heteroaromatic group, a divalent group corresponding to a precursor of the divalent heteroaromatic group, or a divalent group composed of two or more of these groups bonded to each other, wherein at least one of $Y^{1a}$ and $Y^{1b}$ is a divalent heteroaromatic group or a group containing a divalent group corresponding to a precursor of the divalent heteroaromatic group; $W^1$ and $W^2$ are the same as or different from each other and each represent a flexible unit composed of a divalent group containing at least an alkylene group or ether bond and having a total of two to twenty atoms; each of $Z^1$ and $Z^2$ independently represents carboxyl group, a substituted oxycarbonyl group, formyl group, a haloformyl group, a substituted or unsubstituted ethynyl-containing group, 3,4-diaminophenyl group, 3-amino-4-hydroxyphenyl group, 4-amino-3-hydroxyphenyl

group, 3-amino-4-mercaptophenyl group, or 4-amino-3-mercaptophenyl group; $R^1$ represents a hydrogen atom or a hydrocarbon group; "k" denotes 0 or 1; each of "p1" and "p2" independently denotes an integer of 1 to 3; and "p3" denotes an integer of 0 to 2, wherein the total of "p1", "p2", and "p3" equals 2 to 4, and wherein two or more $Y^{1a}$s, $Y^{1b}$s, $Y^{2a}$s, $Y^{2b}$s, $W^1$s, $W^2$s, $Z^1$s, $Z^2$s, and $R^1$s, if present per molecule, may be the same as or different from each other, respectively.

Preferably, at least one of $Y^{1a}$ and $Y^{1b}$ is a divalent heteroaromatic group containing at least one of benzimidazole ring, benzoxazole ring, and benzothiazole ring, or a divalent group corresponding to a precursor of the divalent heteroaromatic group.

Preferably, at least one of $Y^{2a}$ and $Y^{2b}$ is a divalent heteroaromatic group containing at least one of benzimidazole ring, benzoxazole ring, and benzothiazole ring, or a divalent group corresponding to a precursor of the divalent heteroaromatic group.

The N-Substituted benzimidazole-containing polymer of the present invention has a bridged alicyclic skeleton as a central skeleton and has a structural unit containing N-substituted benzimidazole ring as a repeating unit. Because the substituent(s) bonded to the nitrogen atom suppresses the polarity and exhibits steric exclusion effect, the polymer thereby gives a thin film exhibiting very low moisture absorptivity and having a low relative dielectric constant. The thin film excels in thermal stability and mechanical strength.

Additionally, the ethynyl-containing bridged alicyclic compound of the present invention has a bridged alicyclic skeleton as a central skeleton. Because a substituted or unsubstituted ethynyl-containing group is bonded to the bridged alicyclic skeleton through a divalent or higher-valent organic group containing a heterocyclic ring, polymerization of the compound gives a polymer and/or a insulating film having a pore structure and a low relative dielectric constant. In addition, the ethynyl group, even if remains unreacted, does not adversely affect the dielectric constant, whereby the insulating film shows less variation in relative dielectric constant. Further, the insulating film, obtained by a polymerization of the ethynyl-containing bridged alicyclic compound of the present invention, excels in thermal stability and mechanical strength.

Further, in the material for producing the insulating film of the present invention, at least one of two compounds capable of forming a polymer having a pore structure, or one compound capable of forming a polymer having a pore structure has a flexible unit with a specific structure. Therefore, a molecular chain in the compound may easily move, and functional groups or moieties surely react with each other upon polymerization, to give polymers and insulating films having a pore structure. The polymers and insulating films have a low dielectric constant and show less variation in dielectric constant. The insulating films thus obtained has high thermal stability and high mechanical strength. The polymerizable compound of the present invention is usable as a constitutional component (monomer component) of the advantageous material for producing the insulating film, having excellent characteristic properties.

BRIEF DESCRIPTION OF THE DRAWINGS

FIG. 1 depicts an NMR spectrum of an amino-containing adamantane derivative prepared in Preparation Example A1;
FIG. 2 depicts an infrared absorption spectrum of an amino-containing adamantane derivative prepared in Preparation Example A1;
FIG. 3 depicts an NMR spectrum of an ethynyl-containing adamantane derivative prepared in Preparation Example A6;
FIG. 4 depicts an NMR spectrum of an N-substituted ethynylbenzimidazole-containing bridged alicyclic compound prepared in Example A5;
FIG. 5 depicts an NMR spectrum of an N-substituted ethynylbenzimidazole-containing bridged alicyclic compound prepared in Example A6;
FIG. 6 depicts an NMR spectrum of an N-substituted ethynylbenzimidazole-containing bridged alicyclic compound prepared in Example A7;
FIG. 7 is a graph showing leak current characteristics of thin films prepared from the ethynyl-containing adamantane derivative prepared in Preparation Example A6 and from the N-substituted ethynylbenzimidazole-containing bridged alicyclic compounds prepared in Example A5 to A7, respectively;
FIG. 8 depicts an NMR spectrum of an ethynyl-containing adamantane derivative prepared in Example B2;
FIG. 9 is a graph showing intra-sample variations in relative dielectric constant of thin films prepared in Example 1 and Comparative Example 1;
FIG. 10 is a graph showing intra-sample variations in relative dielectric constant of thin films prepared in Examples 2, 3 and 4; and
FIG. 11 is a graph showing how the leak current varies depending on the applied field, of thin films prepared in Example 1 and Comparative Example 1.

DESCRIPTION OF THE PREFERRED EMBODIMENTS

All numbers are herein assumed to be modified by the term "about."

An N-substituted benzimidazole-containing bridged alicyclic compound according to the present invention is represented by Formula (1-1). In Formula (1-1), $Z^3$ represents a bridged alicyclic skeleton; $Y^{11}$ represents a single bond or a divalent organic group; $Y^2$ represents a single bond or a di- or tri-valent organic group; $X^3$ represents a reactive functional group; and $R^a$ represents a hydrogen atom or a hydrocarbon group. The group $A^3$ represents a group of Formula (a) or (b). In Formula (a) or (b), $R^{10}$ represents a monovalent organic group. In Formulae (a) and (b), the left side is to be bonded to $Y^{11}$, and the right side is to be bonded to $Y^2$.

Representative examples of the bridged alicyclic skeleton as central skeleton $Z^3$ include rings of Formulae (2a) to (2j); and rings each composed of two or more (e.g., two or three) of these rings bonded to each other.

Preferred examples of the bridged alicyclic skeleton include adamantane skeletons (e.g., adamantane-1,3,5,7-tetrayl group), biadamantane skeleton, tetraphenyladamantane skeleton, norbornane skeleton, tetramethylnorbornane skeleton, norbornene skeleton, and tetramethylnorbornene skeleton. The molecular weight of the central skeleton moiety is, for example, about 40 to 1460, and preferably about 60 to 500.

Examples of the divalent organic groups as $Y^{11}$ and $Y^2$ include an alkylene group, an alkenylene group, an alkynylene group, a divalent alicyclic hydrocarbon group, an arylene group, a divalent heterocyclic group, a group composed of two or more (e.g., two to five) of these groups bonded to each other, and a group composed of one or more (e.g., one to five) of these divalent organic groups bonded to at least one atom selected from oxygen atom (-O-) and sulfur atom (-S-). Examples of the trivalent organic group as $Y^2$ include trivalent organic groups corresponding to these divalent organic groups.

Examples of the alkylene group include linear or branched alkylene groups having one to ten carbon atoms, such as methylene, ethylene, ethylidene, trimethylene, isopropylidene, propylene, tetramethylene, pentamethylene, hexamethylene, octamethylene, and decamethylene groups, of which linear alkylene groups are preferred. Examples of the alkenylene group include linear or branched alkenylene groups having two to ten carbon atoms, such as vinylene and propenylene groups, of which linear alkenylene groups are preferred. Example of the alkynylene group include linear or branched alkynylene groups having two to ten carbon atoms, such as ethynylene and propynylene groups, of which linear alkynylene groups are preferred.

Examples of the divalent alicyclic hydrocarbon group include cycloalkylene groups and cycloalkylidene groups, such as cyclopentylene, cyclohexylene, cyclopentylidene, and cyclohexylidene groups; and divalent bridged alicyclic groups such as adamantane-1,3-diyl group and 1,3-dimethyladamantane-5,7-diyl group. Examples of the arylene group include phenylene, benzylidene, naphthylene, and anthranylene groups.

Examples of a heterocyclic ring constituting the divalent heterocyclic group include benzimidazole ring (whose nitrogen atom may be substituted), benzoxazole ring, benzothiazole ring, imidazole ring (whose nitrogen atom may be substituted), oxazole ring, and thiazole ring.

Examples of the divalent group composed of these divalent organic groups bonded to oxygen atom include oxyalkylene groups having about one to ten carbon atoms, such as oxyethylene group; polyoxyalkylene groups having about two to ten carbon atoms; and divalent groups derived from diphenyl ether by removing two hydrogen atoms from the diphenyl ether.

Representative examples of the divalent organic groups as $Y^{11}$ and $Y^2$ include divalent groups of Formulae (42a) to (42n), and divalent groups each composed of two or more (e.g., two to five) of these groups bonded to each other. Each of the rings in Formulae (42a) to (42n) may have one or more substituents. Examples of such substituents include hydrocarbon groups having one to fifty carbon atoms.

In Formulae (42a) to (42n), $R^{21}$ represents a divalent aliphatic hydrocarbon group having one to fifty carbon atoms (preferably one to twenty carbon atoms). Examples of the divalent aliphatic hydrocarbon group include linear or branched alkylene groups, alkenylene groups, and alkynylene groups, such as methylene, ethylene, ethylidene, trimethylene, propylene, isopropylidene, tetramethylene, ethylethylene, hexamethylene, decamethylene, dodecamethylene, vinylene, propenylene, hexenylene, octenylene, and propynylene groups.

R" represents a hydrogen atom or a monovalent organic group. Examples of the monovalent organic group are as with monovalent organic groups listed as after-mentioned $R^{10}$. Representative examples of the trivalent organic group as $Y^2$ include trivalent organic groups corresponding to the above-listed representative examples of the divalent organic group.

Examples of the reactive functional group as $X^3$ include a substituted or unsubstituted ethynyl group, a substituted or unsubstituted vinyl group, a halogen atom, an unsubstituted or mono-substituted amino group, a haloformyl group, acid anhydride group, acid azido group, hydrazido group, cyano group, an acyl group, carboxyl group, a substituted oxycarbonyl group, hydroxyl group, mercapto group, an imino group, and an alkoxysilyl group.

Examples of the substituent(s) in the substituted or unsubstituted ethynyl group include alkyl groups such as methyl, ethyl, propyl, isopropyl, butyl, and isobutyl groups, of which alkyl groups having about one to ten carbon atoms

are preferred; and aryl groups such as phenyl and naphthyl groups, of which aryl groups having about six to twenty carbon atoms are preferred; and substituted silyl groups such as trimethylsilyl and triethylsilyl group, of which trialkylsilyl groups are preferred.

Examples of the substituent(s) in the substituted or unsubstituted vinyl group include alkyl groups such as methyl, ethyl, propyl, isopropyl, butyl, and isobutyl groups, of which alkyl groups having about one to ten carbon atoms are preferred; and aryl groups such as phenyl and naphthyl groups, of which aryl groups having about six to twenty carbon atoms are preferred.

Examples of the mono-substituted amino group include alkylamino groups such as methylamino group and ethylamino group; arylamino groups such as phenylamino group; and aralkylamino groups such as benzylamino group.

Examples of the haloformyl group include -COCl, -COBr, and -COI. Examples of the acyl group include acyl groups having about one to ten carbon atoms, such as formyl group, acetyl group, propionyl group, and benzoyl group. The substituted oxycarbonyl group includes, for example, alkoxycarbonyl groups such as methoxycarbonyl group and ethoxycarbonyl group, of which alkoxy-carbonyl groups whose alkoxy moiety has one to four carbon atoms are preferred; aryloxycarbonyl groups such as phenoxycarbonyl group; and aralkyloxycarbonyl groups such as benzyloxycarbonyl group. Examples of the imino group include groups each formed as a result of a reaction between the acyl group and ammonia or an amine. The amine may be an amine having about one to ten carbon atoms, such as methylamine, ethylamine, or aniline. Examples of the alkoxysilyl group include trialkylsilyl groups such as trimethylsilyl group and triethylsilyl group.

Examples of the monovalent organic group as $R^{10}$ include an aliphatic hydrocarbon group, an alicyclic hydrocarbon group, an aromatic hydrocarbon group, and a group composed of two or more (e.g., two to four) of these groups bonded to each other with or without the interposition of at least one of oxygen atom and sulfur atom. The monovalent organic group has a total of, for example, one to fifty, and preferably two to forty carbon atoms.

Examples of the aliphatic hydrocarbon group include linear or branched alkyl groups having about one to forty carbon atoms, such as methyl, ethyl, propyl, isopropyl, butyl, isobutyl, s-butyl, t-butyl, pentyl, hexyl, decyl, and dodecyl groups, of which those having about one to thirty carbon atoms are preferred, and those having about one to twenty-five carbon atoms are more preferred; linear or branched alkenyl groups having about two to forty carbon atoms, such as vinyl, allyl, 1-butenyl, and 3-methyl-4-pentenyl groups, of which those having about two to thirty carbon atoms are preferred, and those having about two to twenty-five carbon atoms are more preferred; and linear or branched alkynyl groups having about two to forty carbon atoms, such as ethynyl, propynyl, 1-butynyl, and 2-butynyl groups, of which those having about two to thirty carbon atoms are preferred, and those having about two to twenty-five carbon atoms are more preferred.

Examples of the alicyclic hydrocarbon group include monocyclic alicyclic hydrocarbon groups including cycloalkyl groups having about three to twenty members, such as cyclopropyl, cyclobutyl, cyclopentyl, cyclohexyl, and cyclooctyl groups (of which those having about three to fifteen members are preferred, and those having about three to twelve members are more preferred), and cycloalkenyl groups having about three to twenty members, such as cyclopropenyl, cyclobutenyl, cyclopentenyl, and cyclohexenyl groups (of which those having about three to fifteen members are preferred, and those having about three to ten members are more preferred); and bridged alicyclic hydrocarbon groups (bridged hydrocarbon groups) typically having a bridged alicyclic ring including about two to four rings, such as adamantane ring, perhydroindene ring, decalne ring, perhydrofluorene ring, perhydroanthracene ring, perhydrophenanthrene ring, tricyclodecane ring, tricycloundecane ring, tetracyclododecane ring, perhydroacenaphthene ring, perhydrophenalene ring, norbornane ring, or norbornene ring. Examples of the aromatic hydrocarbon group include aromatic hydrocarbon groups having about six to twenty carbon atoms, such as phenyl, biphenyl, naphthyl, anthranyl, phenanthryl, and pyrenyl groups, of which those having about six to fourteen carbon atoms are preferred.

Examples of hydrocarbon groups each composed of an aliphatic hydrocarbon group bonded to an alicyclic hydrocarbon group include cycloalkyl-alkyl groups (of which $C_{3-20}$ cycloalkyl - $C_{1-4}$ alkyl groups are preferred) such as cyclopentylmethyl, cyclohexylmethyl, and 2-cyclohexylethyl groups; and bridged alicyclic group-alkyl groups, such as adamantylmethyl, adamantylethyl, norbornylmethyl, and norbornylethyl groups. Examples of hydrocarbon groups each composed of an aliphatic hydrocarbon group and an aromatic hydrocarbon group bonded to each other include aralkyl groups (of which $C_{7-18}$ aralkyl groups are preferred) such as benzyl, 2-phenylethyl, and biphenylmethyl groups; and alkyl-substituted aryl groups, such as phenyl group or naphthyl group substituted with about one to four alkyl groups each having one to four carbon atoms.

The aliphatic hydrocarbon group, alicyclic hydrocarbon group, aromatic hydrocarbon group, and the group composed of these groups may each have one or more substituents. Such substituents are not particularly limited, as long as they do not adversely affect the reaction and properties of high-molecular-weight polymers as polymerization products.

Representative examples of the monovalent organic group as $R^{10}$ include groups of Formulae (31a) to (31i). In these formulae, $R^{11}$ represents a single bond or a divalent aliphatic hydrocarbon group having one to fifty carbon atoms (preferably one to thirty-nine carbon atoms); and "j" denotes an integer of 0 to 3. Examples of the divalent aliphatic

hydrocarbon group having one to fifty carbon atoms as $R^{11}$ are as with the above-listed divalent aliphatic hydrocarbon groups having one to fifty carbon atoms as $R^{21}$. In these formulae, each of rings may have one or more substituents. Examples of such substituents include hydrocarbon groups having one to fifty carbon atoms.

Examples of the hydrocarbon group as $R^a$ include aliphatic hydrocarbon groups, alicyclic hydrocarbon groups, aromatic hydrocarbon groups, and groups each composed of two or more of these groups bonded to each other. Examples of the aliphatic hydrocarbon groups, alicyclic hydrocarbon groups, aromatic hydrocarbon groups, and groups each composed of two or more of these groups bonded to each other are as with the groups listed in $R^{10}$.

In Formula (1-1), "n4" denotes an integer of 2 to 7, and is preferably 3 or 4, and more preferably 4; "m3" denotes an integer of 0 to 5; and "k2" denotes an integer of 0 to 2. The total of "n4" and "m3" equals 2 to 7. Two or more $Y^{11}$s, $Y^2$s, $X^3$s, $A^3$s, and $R^{10}$s per molecule, and two or more $X^3$s and $R^a$s, if present per molecule, may be the same as or different from one another, respectively.

Compounds of Formula (1-1) can be synthetically prepared from known compounds or derivatives of known compounds as starting materials, using reactions such as condensation reactions, substitution reactions, addition reactions, oxidation reactions, and cyclization reactions. Typically, a compound of Formula (1-1) can be prepared by reacting a corresponding compound of Formula (a) or Formula (b), wherein $R^{10}$ is a hydrogen atom, with a halogen compound represented by $R^{10}X^4$, wherein $X^4$ represents a halogen atom; and $R^{10}$ is as defined above, in the presence of a base. The base may be, for example, sodium hydride. The reaction may be conducted in a solvent at a temperature of about -20°C to 120°C. Examples of the solvent are amides such as N,N-dimethylacetamide (DMAc) and N-methyl-2-pyrrolidone; and cyclic aminoacetals such as dimethylimidazolidine and dimethylimidazolidinone (dimethylimidazolidine-dione). A benzimidazole skeleton of the compound used as a starting material in this reaction can be formed, for example, by reacting an aldehyde compound and a diamine compound in a solvent at a temperature of about -30°C to 150°C in an oxygen atmosphere, in which the diamine compound has benzene ring to which two amino groups are bonded at the ortho positions. Examples of the solvent herein are as with the above listed solvents.

N-Substituted benzimidazole-containing polymers according to the present invention include (i) polymers as polymerization products of any of the N-substituted benzimidazole-containing bridged alicyclic compounds wherein "k2" in Formula (1-1) is 1 or 2 (the compound is hereinafter also simply referred to as Compound A'); (ii) polymers as reaction products between Compound A' and a polyfunctional compound having two or more functional groups or moieties capable of reacting with the reactive functional group $X^3$ of Compound A' (this compound is hereinafter also simply referred to as Compound B'); and (iii) N-substituted benzimidazole-containing polymers having repeating units of at least one of Formulae (51a), (51b) and (51c). N-Substituted benzimidazole-containing polymers according to the present invention have a weight-average molecular weight of, for example, about 200 to 100000, preferably about 1000 to 80000, and more preferably about 5000 to 60000.

The polymers (i) are obtained when $X^3$ in Compound A' is a self-reactive functional group, i.e., a functional group capable of reacting with each other, such as a substituted or unsubstituted ethynyl group. Each of different Compounds A' can be used alone or in combination.

In preparation of the polymers (ii), the functional group of Compound B' having reactivity with the reactive functional group $X^3$ of Compound A' can be selected from known functional groups. Typically, when the reactive functional group $X^3$ of Compound A' is a substituted or unsubstituted ethynyl group, the reactive functional group of Compound B' may be, for example, a substituted or unsubstituted ethynyl group. When the reactive functional group $X^3$ of Compound A' is a halogen atom, a haloformyl group, acid anhydride group, carboxyl group, or a substituted oxycarbonyl group, examples of the reactive functional group of Compound B' include an unsubstituted or mono-substituted amino group, hydroxyl group, and mercapto group. When the reactive functional group $X^3$ of Compound A' is an unsubstituted or mono-substituted amino group, hydroxyl group, or mercapto group, examples of the reactive functional group of Compound B' include a halogen atom, a haloformyl group, acid anhydride group, carboxyl group, a substituted oxycarbonyl group, and an acyl group. When the reactive functional group $X^3$ of Compound A' is an acyl group, the reactive functional group of Compound B' may be, for example, an amino group. When the reactive functional group $X^3$ of Compound A' is an alkoxysilyl group, the reactive functional group of Compound B' may be, for example, an alkoxysilyl group.

A heterocyclic ring is formed as a result of a reaction when the reactive functional group $X^3$ of Compound A' and the reactive functional group of Compound B' are in the following specific combinations. Specifically, an imidazole ring, for example, is formed when one of the reactive functional group of Compound A' and the reactive functional group of Compound B' is carboxyl group, a substituted oxycarbonyl group, a haloformyl group, or formyl group, and the other is two amino groups bonded to adjacent carbon atoms. In this case, a benzimidazole ring is formed when the other is two amino groups bonded to carbon atoms at the ortho positions of benzene ring. An oxazole ring, for example, is formed when one is carboxyl group, a substituted oxycarbonyl group, a haloformyl group, or formyl group, and the other is amino group and hydroxyl group bonded to adjacent carbon atoms, and in this case, a benzoxazole ring is formed when the other is amino group and hydroxyl group bonded to carbon atoms at the ortho positions of benzene ring. A thiazole ring, for example, is formed when one is carboxyl group, a substituted oxycarbonyl group, a haloformyl group, or formyl group, and the other is amino group and mercapto group bonded to adjacent carbon atoms. In this case, a benzothiazole ring

is formed when the other is amino group and mercapto group bonded to carbon atoms at the ortho positions of benzene ring.

Preferably, Compound B' is a bifunctional, trifunctional, or tetrafunctional compound. Representative examples of Compound B' include 1,2,4,5-tetraaminobenzene, 1,4-diamino-2,5-dihydroxybenzene, 1,5-diamino-2,4-dihydroxybenzene, 1,4-diamino-2,5-dimercaptobenzene, 1,5-diamino-2,4-dimercaptobenzene, and 3,3'-diaminobenzidine. Each of these compounds can be used when the reactive functional group $X^3$ of Compound A' is, for example, carboxyl group, a substituted oxycarbonyl group, a haloformyl group, or formyl group, and the reactions of these compounds give the heterocyclic ring. Examples of Compound B' also include polymers having, in their principle chain or side chain, a functional group capable of reacting with the reactive functional group $X^3$ of Compound A'. In this case, Compound A' acts as a crosslinking agent.

In Formulae (51a), (51b), and (51c) in the polymers (iii), $Z^3$ represents a bridged alicyclic skeleton; $Y^{11}$ represents a single bond or a divalent organic group; $Y^2$ represents a single bond or a divalent organic group; $R^a$ represents a hydrogen atom or a hydrocarbon group; and $A^3$ represents a group of Formula (a) or (b). In Formulae (a) and (b), $R^{10}$ represents a monovalent organic group. In Formulae (a) and (b), the left side is to be bonded to $Y^{11}$, and the right side is to be bonded to $Y^2$. The number of repeating units of at least one of Formulae (51a), (51b), and (51c) in a polymer is, for example, about 5 to 25, and preferably about 10 to 20.

The bridged alicyclic skeleton as $Z^3$, the divalent organic groups as $Y^{11}$ and $Y^2$, the hydrocarbon group as $R^a$, and the monovalent organic group as $R^{10}$ are as above.

Of polymers having repeating units of at least one of Formulae (51a), (51b), and (51c), a polymer wherein $A^3$ is a group of Formula (a) can be prepared in the following manner. Initially, a formyl-containing bridged alicyclic compound represented by following Formula (7-2):

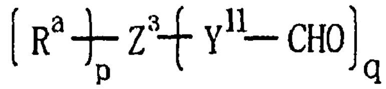

(7 − 2)

wherein $Z^3$, $Y^{11}$, and $R^a$ are as defined above; "q" denotes an integer of 2 to 4; and "p" denotes an integer of 0 to 2, wherein the total of "p" and "q" equals 2 to 4, is reacted (polymerized) with a polyfunctional amine compound represented by following Formula (8-1):

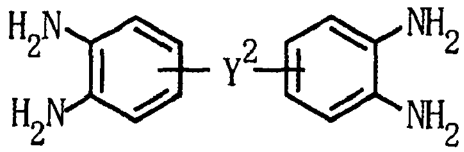

(8 − 1)

wherein $Y^2$ is as defined above. The terminal diaminophenyl group of the formed polymer is generally reacted and capped with an aldehyde compound such as benzaldehyde, and the capped compound is reacted with $R^{10}X^4$, wherein $X^4$ represents a halogen atom; and $R^{10}$ is as defined above, to introduce $R^{10}$ into the nitrogen atom of NH group of the formed benzimidazole ring. When "p" is 2 and "q" is 2, a polymer having a repeating unit of Formula (51a) is prepared; when "p" is 1 and "q" is 3, a polymer having a repeating unit of Formula (51b) is prepared; and when "p" is 0 and "q" is 4, a polymer having a repeating unit of Formula (51c) is prepared.

Of polymers having a repeating unit of Formulae (51a), (51b), or (51c), a polymer wherein $A^3$ is a group of Formula (b) can be prepared in the following manner. Initially, an amino-containing bridged alicyclic compound represented by following Formula (9-1):

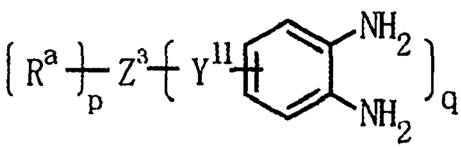

(9 − 1)

wherein $Z^3$, $Y^{11}$, $R^a$ is as defined above; "q" denotes an integer of 2 to 4; and "p" denotes an integer of 0 to 2, wherein

the total of "p" and "q" equals 2 to 4, is reacted (polymerized) with a polyfunctional aldehyde compound represented by following Formula (10):

$$OHC-Y^2-CHO \qquad (10)$$

wherein $Y^2$ is as defined above. The terminal formyl group of the formed polymer is generally reacted and capped with a diamine compound having two amino groups bonded to adjacent carbon atoms, such as o-diaminobenzene, and the capped compound is reacted with $R^{10}X^4$, wherein $X^4$ represents a halogen atom; and $R^{10}$ is as defined above, to introduce $R^{10}$ into the nitrogen atom of NH group of the formed benzimidazole ring. When "p" is 2 and "q" is 2, a polymer having a repeating unit of Formula (51a) is prepared; when "p" is 1 and "q" is 3, a polymer having a repeating unit of Formula (51b) is prepared; and when "p" is 0 and "q" is 4, a polymer having a repeating unit of Formula (51c) is prepared.

The reaction between the formyl-containing bridged alicyclic compound of Formula (7-2) and the polyfunctional amine compound of Formula (8-1), the reaction between the amino-containing bridged alicyclic compound of Formula (9-1) and the polyfunctional aldehyde compound of Formula (10), and the reactions for capping terminals can be carried out in a solvent at temperatures of about -30°C to 150°C in an oxygen atmosphere. Examples of the solvent include amides such as N,N-dimethylacetamide (DMAc) and N-methyl-2-pyrrolidone; cyclic aminoacetals such as dimethylimidazolidine and dimethylimidazolidinone (dimethylimidazolidine-dione). The "alkylation" reaction with $R^{10}X^4$ can be carried out in the presence of a base, such as sodium hydride, in a solvent, such as the above solvent, at temperatures of about -20°C to 120°C. The capping of polymer terminal is conducted so as to hydrophobilize the terminal of polymer to thereby reduce moisture absorptivity.

An ethynyl-containing bridged alicyclic compound according to the present invention is represented by Formula (1). In Formula (1), Z represents a bridged alicyclic skeleton; X represents a divalent or higher-valent organic group containing a heterocyclic ring or a precursor structure thereof; Y represents a substituted or unsubstituted ethynyl-containing group; and R represents a hydrogen atom or a hydrocarbon group.

Representative examples of the bridged alicyclic skeleton as the central skeleton Z include rings of Formulae (2a) to (2j), and rings each composed of two or more (e.g., two or three) of these rings bonded to each other.

Preferred examples of the bridged alicyclic skeleton include adamantane skeleton such as adamantane-1,3,5,7-tetrayl group, biadamantane skeleton, tetraphenyladamantane skeleton, norbornane skeleton, tetramethylnorbornane skeleton, norbornene skeleton, and tetramethylnorbornene skeleton. The molecular weight of the central skeleton moiety is, for example; about 40 to 1460, and preferably about 60 to 500.

Examples of the heterocyclic ring in the divalent or higher-valent organic group containing a heterocyclic ring or a precursor structure thereof as X include imidazole ring, benzimidazole ring, oxazole ring, benzoxazole ring, thiazole ring, and benzothiazole ring. Among them, benzimidazole ring, benzoxazole ring, and benzothiazole ring are preferred, for better thermal stability.

X may contain a heterocyclic ring or a precursor structure thereof alone or may further contain an aromatic carbon ring in addition to the heterocyclic ring or a precursor structure thereof. Preferred examples of X include imidazolyl group, benzimidazolyl group, oxazolyl group, benzoxazolyl group, thiazolyl group, benzothiazolyl group, a precursor group of any of these heterocyclic groups, a group composed of two or more of these heterocyclic groups or their precursor groups bonded to each other, and a group composed of one or more of these heterocyclic groups or their precursor groups bonded to one or more aromatic hydrocarbon groups. X may contain, where necessary, an organic group containing at least an alkylene group or ether bond and having a total of two to twenty carbon atoms (preferably two to ten carbon atoms).

Representative examples of X include groups of Formulae (3a) to (3v), and groups each composed of two or more of these groups bonded to each other. The groups of Formulae (3e), (3i), (3j), (3m), (3o), (3p), (3s), (3t), (3u), and (3v) are groups each containing a precursor structure of benzoxazole ring. In Formulae (3a) to (3v), $A^2$ represents - NH-, oxygen atom, or sulfur atom; and "s1" denotes an integer of 0 to 5, wherein each of rings in the formulae may have one or more substituents.

Examples of such substituents which the rings in the formulae may have include aliphatic hydrocarbon groups, alicyclic hydrocarbon groups, aromatic hydrocarbon groups, and groups each composed of two or more (e.g., two to four) of these groups bonded to each other with or without the interposition of oxygen atom and/or sulfur atom. The substituents which each ring may have a total of, for example, one to fifty carbon atoms.

Examples of the aliphatic hydrocarbon groups include linear or branched alkyl groups having about one to twenty carbon atoms, such as methyl, ethyl, propyl, isopropyl, butyl, isobutyl, s-butyl, t-butyl, pentyl, hexyl, decyl, and dodecyl groups, of which those having about one to ten carbon atoms are preferred, and those having about one to six carbon atoms are more preferred; linear or branched alkenyl groups having about two to twenty carbon atoms, such as vinyl, allyl, 1-butenyl, and 3-methyl-4-pentenyl groups, of which those having about two to ten carbon atoms are preferred,

and those having about two to five carbon atoms are more preferred; and linear or branched alkynyl groups having about two to twenty carbon atoms, such as ethynyl, propynyl, 1-butynyl, and 2-butynyl groups, of which those having about two to ten carbon atoms are preferred, and those having about two to five carbon atoms are more preferred.

Examples of the alicyclic hydrocarbon groups include monocyclic alicyclic hydrocarbon groups including cycloalkyl groups having about three to twenty members, such as cyclopropyl, cyclobutyl, cyclopentyl, cyclohexyl, and cyclooctyl groups (of which those having about three to fifteen members are preferred, and those having about three to twelve members are more preferred), and cycloalkenyl groups having about three to twenty members, such as cyclopropenyl, cyclobutenyl, cyclopentenyl, and cyclohexenyl groups (of which those having about three to fifteen members are preferred, and those having about three to ten members are more preferred); and bridged alicyclic hydrocarbon groups (bridged hydrocarbon groups) typically having a bridged alicyclic ring containing about two to four rings, such as adamantane ring, perhydroindene ring, decalne ring, perhydrofluorene ring, perhydroanthracene ring, perhydrophenanthrene ring, tricyclodecane ring, tricycloundecane ring, tetracyclododecane ring, perhydroacenaphthene ring, perhydrophenalene ring, norbornane ring, and norbornene ring. Examples of the aromatic hydrocarbon groups include aromatic hydrocarbon groups having about six to twenty carbon atoms, such as phenyl, biphenyl, naphthyl, anthranyl, phenanthryl, and pyrenyl groups, of which those having about six to fourteen carbon atoms are preferred.

Examples of hydrocarbon groups each composed of an aliphatic hydrocarbon group and an alicyclic hydrocarbon group bonded to each other include cycloalkyl-alkyl groups such as cyclopentylmethyl, cyclohexylmethyl, and 2-cyclohexylethyl groups, of which $C_{3-20}$ cycloalkyl - $C_{1-4}$ alkyl groups are preferred; and bridged alicyclic group-alkyl groups, such as adamantylmethyl, adamantylethyl, norbornylmethyl, and norbornylethyl groups. Examples of hydrocarbon groups each composed of an aliphatic hydrocarbon group and an aromatic hydrocarbon group bonded to each other include aralkyl groups such as benzyl, 2-phenylethyl, and biphenylmethyl groups, of which aralkyl groups having seven to eighteen carbon atoms are preferred; and alkyl-substituted aryl groups, such as phenyl group or naphthyl group substituted with about one to four alkyl groups each having one to four carbon atoms. The aliphatic hydrocarbon groups, alicyclic hydrocarbon groups, aromatic hydrocarbon groups, and the groups composed of these groups may have one or more substituents. Such substituents are not particularly limited, as long as they do not adversely affect the reaction and properties of high-molecular-weight polymers as polymerization products.

Examples of substituents in the substituted or unsubstituted ethynyl-containing group as Y include alkyl groups such as methyl, ethyl, propyl, isopropyl, butyl, and isobutyl groups, of which the alkyl groups having about one to ten carbon atoms are preferred; aryl groups such as phenyl and naphthyl groups, of which the aryl groups having about six to twenty carbon atoms are preferred; and substituted silyl groups such as trimethylsilyl and triethylsilyl groups, of which trialkylsilyl groups are preferred.

Preferred examples of Y include substituted or unsubstituted ethynyl groups and substituted or unsubstituted ethynylphenyl groups.

Representative examples of Y include groups represented by following Formulae (41a) to (41d):

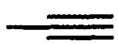

(41a)

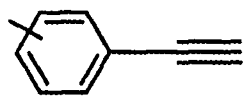

(41b)

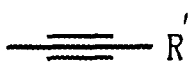

(41c)

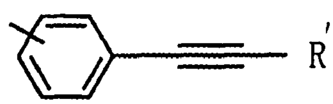

(41d)

wherein R' represents an alkyl group, an aryl group, or a trialkylsilyl group.

Examples of the alkyl group as R' include alkyl groups having about one to ten carbon atoms, such as methyl, ethyl, propyl, isopropyl, butyl, and isobutyl groups. Examples of the aryl group include aryl groups having about six to twenty carbon atoms, such as phenyl and naphthyl groups. Examples of the trialkylsilyl group include trimethylsilyl and triethylsilyl groups.

Examples of the hydrocarbon group as R include an aliphatic hydrocarbon group, an alicyclic hydrocarbon group, an aromatic hydrocarbon group, and a group composed of these groups bonded to each other. Examples of the aliphatic hydrocarbon group include linear or branched alkyl groups having about one to twenty carbon atoms, such as

methyl, ethyl, propyl, isopropyl, butyl, isobutyl, s-butyl, t-butyl, pentyl, hexyl, decyl, and dodecyl groups, of which those having about one to ten carbon atoms are preferred, and those having about one to six carbon atoms are more preferred; linear or branched alkenyl groups having about two to twenty carbon atoms, such as vinyl, allyl, 1-butenyl, and 3-methyl-4-pentenyl groups, of which those having about two to ten carbon atoms are preferred, and those having about two to five carbon atoms are more preferred; and linear or branched alkynyl groups having about two to twenty carbon atoms, such as ethynyl, propynyl, 1-butynyl, and 2-butynyl groups, of which those having about two to ten carbon atoms are preferred, and those having about two to five carbon atoms are more preferred.

Examples of the alicyclic hydrocarbon group include monocyclic alicyclic hydrocarbon groups including cycloalkyl groups having about three to twenty members, such as cyclopropyl, cyclobutyl, cyclopentyl, cyclohexyl, and cyclooctyl groups (of which those having about three to fifteen members are preferred, and those having about three to twelve members are more preferred), and cycloalkenyl groups having about three to twenty members, such as cyclopropenyl, cyclobutenyl, cyclopentenyl, and cyclohexenyl groups (of which those having about three to fifteen members are preferred, and those having about three to ten members are more preferred); and bridged alicyclic hydrocarbon groups (bridged hydrocarbon groups) typically having a bridged alicyclic ring containing about two to four rings, such as adamantane ring, perhydroindene ring, decalne ring, perhydrofluorene ring, perhydroanthracene ring, perhydrophenanthrene ring, tricyclodecane ring, tricycloundecane ring, tetracyclododecane ring, perhydroacenaphthene ring, perhydrophenalene ring, norbornane ring, and norbornene ring. Examples of the aromatic hydrocarbon group include aromatic hydrocarbon groups having six to twenty carbon atoms, such as phenyl and naphthyl groups, of which those having about six to fourteen carbon atoms are preferred.

Examples of the hydrocarbon group composed of an aliphatic hydrocarbon group and an alicyclic hydrocarbon group bonded to each other include cycloalkyl-alkyl groups such as cyclopentylmethyl, cyclohexylmethyl, and 2-cyclohexylethyl groups, of which $C_{3-20}$ cycloalkyl - $C_{1-4}$ alkyl groups are preferred. Examples of the hydrocarbon group each composed of an aliphatic hydrocarbon group and an aromatic hydrocarbon group bonded to each other include aralkyl groups such as aralkyl groups having seven to eighteen carbon atoms; and alkyl-substituted aryl groups, such as phenyl group or naphthyl group substituted with about one to four alkyl groups each having one to four carbon atoms.

The aliphatic hydrocarbon group, alicyclic hydrocarbon group, aromatic hydrocarbon group, and the group composed of these groups may each have one or more substituents. Such substituents are not particularly limited, as long as they do not adversely affect the reaction and properties of high-molecular-weight crosslinked products after polymerization.

In Formula (1), "m" denotes an integer of 1 to 5, and is preferably 1 or 2, and more preferably 1; "n3" denotes an integer of 2 to 7, and is preferably 3 or 4, and more preferably 4; and "k1" denotes an integer of 0 to 5, wherein the total of "n3" and "k1" equals 2 to 7. Two or more Xs and Ys per molecule, and two or more Rs, if present per molecule, may be the same as or different from each other, respectively.

Compounds of Formula (1) can be synthetically prepared, as starting materials, from known compounds or derivatives derived from known compounds with known reactions. The compounds of Formula (1) are prepared with known reactions such as condensation reactions, substitution reactions, addition reactions, oxidation reactions, and cyclization reactions. Of compounds of Formula (1), a compound having a terminal structure represented by following Formula (5):

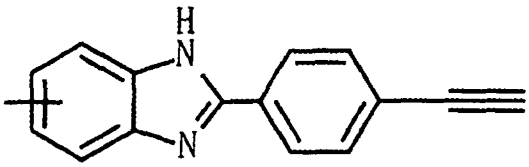

(5)

can be prepared by reacting a corresponding compound having a terminal structure represented by following Formula (6):

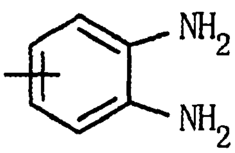

(6)

with a compound represented by following Formula (7-1):

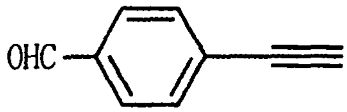

(7 − 1)

The reaction between the compound having a terminal structure of Formula (6) and the compound of Formula (7-1) is generally carried out in a solvent. The solvent can be any one that dissolves materials therein and does not adversely affect the reaction. Examples of such solvents include amides such as N,N-dimethylformamide, N,N-dimethylacetamide (DMAc), and N-methyl-2-pyrrolidone; cyclic aminoacetals such as dimethylimidazolidine and dimethylimidazolidinone (dimethylimidazolidine-dione); sulfoxides such as dimethyl sulfoxide; sulfones; nitriles such as acetonitrile, propionitrile, and benzonitrile; ketones such as acetone, methyl ethyl ketone, diethyl ketone, methyl isobutyl ketone, cyclopentanone, and cyclohexanone; esters such as formic acid esters, acetic acid esters, propionic acid esters, benzoic acid esters, γ-butyrolactone, and propylene glycol monomethyl ether acetate (PGMEA); ethers such as dioxane, tetrahydrofuran, diethyl ether, and ethylene glycol diethyl ether; halogenated hydrocarbons such as dichloromethane, dichloroethane, chloroform, carbon tetrachloride, and chlorobenzene; aromatic hydrocarbons such as benzene, toluene, xylenes, ethylbenzene, and mesitylene; alicyclic hydrocarbons such as cyclohexane and methylcyclohexane; and aliphatic hydrocarbons such as hexane, heptane, and octane. Each of these solvents can be used alone or in combination.

Of these solvents, aprotic polar solvents such as amides, cyclic aminoacetals, and sulfones are preferred, of which more preferred are amides such as N,N-dimethylacetamide (DMAc) and N-methyl-2-pyrrolidone; and cyclic aminoacetals such as dimethylimidazolidine and dimethylimidazolidinone (dimethylimidazolidine-dione).

The reaction is carried out in an oxygen-containing atmosphere within a range not oxidizing the compound having a structure of Formula (6). Typically, the reaction can be carried out in an atmosphere of a mixed gas containing oxygen diluted with an inert gas such as nitrogen or argon gas.

While varying depending typically on types of materials, the reaction temperature is appropriately set within ranges of generally about 0°C to 280°C, and preferably about -30°C to 150°C. The reaction temperature may be constant, or varied continuously or successively. The proportions of the compound having a structure of Formula (6) and the compound of Formula (7-1) can be selected within a broad range; and the two compounds may be used in an equivalent amount [1 mole of the compound of Formula (7-1) is used to 1 mole of the structure of Formula (6)] or one may be used in excess to the other. The amount of the compound having a structure of Formula (6) is, for example, about 0.1 to 1000 equivalents, preferably about 1 to 800 equivalents, more preferably about 10 equivalents or more, and particularly preferably about 10 to 500 equivalents, to the compound of Formula (7-1). The reaction may be carried out according to a common system or procedure such as a batch system, semi-batch system, or continuous system.

The reaction may use any other components according to types of materials. Such components include catalysts such as base catalysts and acid catalysts; reaction agents; trapping agents such as bases and dehydrating agents; and condensation agents such as polyphosphoric acids.

In the preparation of a compound of Formula (1) or an intermediate material thereof, the formation of a heterocyclic ring may be carried out in the following manner. Typically, a benzimidazole ring can be formed by reacting a compound having carboxyl group, a substituted oxycarbonyl group, formyl group, or a haloformyl group with a compound having 3,4-diaminophenyl group, where necessary in the presence of oxygen. A benzoxazole ring can be formed by reacting a compound having carboxyl group, a substituted oxycarbonyl group, formyl group, or a haloformyl group with a compound having 3-amino-4-hydroxyphenyl group or 4-amino-3-hydroxyphenyl group, where necessary in the presence of oxygen. A benzothiazole ring can be formed by reacting a compound having carboxyl group, a substituted oxycarbonyl group, formyl group, or a haloformyl group with a compound having 3-amino-4-mercaptophenyl group or 4-amino-3-mercaptophenyl group, where necessary in the presence of oxygen. Examples of the substituted oxycarbonyl group include alkoxy-carbonyl groups whose alkoxy moiety has about one to six carbon atoms, such as methoxycarbonyl and ethoxycarbonyl groups.

Materials for producing insulating film according to the present invention contain at least any of the ethynyl-containing bridged alicyclic compounds. Preferably, the materials for producing insulating film further contain another ethynyl-containing compound in addition to the ethynyl-containing bridged alicyclic compound. Examples of the other ethynyl-containing compound include compounds each having two or more (e.g., two to four) substituted or unsubstituted ethynyl groups (e.g., the above-mentioned substituted or unsubstituted ethynyl groups) per molecule. Examples of such compounds are compounds each having two or more ethynyl groups (acetylene groups), such as 1,3,5,7-tetrakis(4-phenylacetylene)adamantane, 1,3,5-tris(4-phenylacetylene)adamantane, and 1,3,5-tris(4-phenylacetylene)benzene. The other ethynyl-containing compound for use herein may also be a polymer having a substituted or unsubstituted ethynyl group in its principle chain or side chain. In a material for producing insulating film containing the ethynyl-containing bridged alicyclic compound in combination with such an ethynyl-containing polymer, the ethynyl-containing bridged alicyclic compound acts as a crosslinking agent. Typically, when a material for producing insulating film (hereinafter also referred to as "coating composition") containing the ethynyl-containing bridged alicyclic compound and ethynyl-containing

polymer is applied to a substrate and heated from room temperature to 600°C, preferably to 400°C, a crosslinking reaction proceeds, to yield a film that has a crosslinked structure and exhibits high thermal stability and high mechanical strength. Each of the ethynyl-containing bridged alicyclic compounds and each of the other ethynyl-containing compounds can be used alone or in combination, respectively.

Further, a material for producing insulating film according to the present invention contains a pair of Compounds A and B; and/or a Compound C. The pair of Compounds A and B each contain two or more functional groups or moieties per molecule and are capable of forming a polymer having a pore structure as a result of polymerization through binding of functional groups or moieties of one compound with a functional group or moiety of the other compound. Compound C contains two or more functional groups or moieties per molecule and is capable of forming a polymer having a pore structure as a result of polymerization through binding of one functional group or moiety with another functional group or moiety. Compounds A, B and C satisfy the following condition (i) or (ii):

(i) at least one of Compounds A and B contains a bridged alicyclic skeleton or an aromatic skeleton as a central skeleton; at least one of Compounds A and B has a thermally stable skeleton positioned between the central skeleton and the functional groups or moieties and composed of an aromatic-ring-containing divalent organic group; at least one of Compounds A and B intramolecularly has a flexible unit composed of an organic group containing at least an alkylene group or ether bond and having a total of two to twenty atoms; and the functional groups or moieties of Compound A and the functional groups or moieties of Compound B constitute a pair of functional groups or moieties capable of reacting with each other to form a heterocyclic ring, or the functional groups or moieties of Compound A and the functional groups or moieties of Compound B are both substituted or unsubstituted ethynyl-containing groups, or

(ii) Compound C contains a bridged alicyclic skeleton or an aromatic skeleton as a central skeleton; Compound C has a thermally stable skeleton composed of an aromatic-ring-containing divalent organic group and positioned between the central skeleton and the one functional group or moiety and/or between the central skeleton and the other functional group or moiety; and Compound C has a flexible unit between the central skeleton and the one functional group or moiety and/or between the central skeleton and the other functional group or moiety, the flexible unit composed of an organic group containing at least an alkylene group or ether bond and having a total of two to twenty atoms; and the one functional group or moiety and the other functional group or moiety of Compound C constitute a pair of functional groups or moieties capable of reacting with each other to form a heterocyclic ring, or are both substituted or unsubstituted ethynyl-containing groups.

As used herein, a "compound having two functional groups or moieties that are involved in polymerization" is also referred to as a "bifunctional" compound, one having three functional groups or moieties is also referred to as a "trifunctional" compound, and one having four functional groups or moieties is also referred to as a "tetrafunctional" compound.

In general, when a polymer (high-molecular-weight polymer) having a pore structure is formed from two compounds X and Y, examples of combinations of the compounds X and Y include a combination of a compound X having two or more (e.g., two to four) functional groups or moieties that are bonded to its central skeleton and constitute a two-dimensional structure or three-dimensional structure; with a compound Y having two or more (e.g., two to four and preferably two) functional groups or moieties that are bonded to its central skeleton and constitute a one-dimensional structure (linear structure) or two-dimensional structure (structure constituting two straight lines at a certain angle). In this case, the compound X forms nodes or crosslinks (vertexes) of the polymer, and the compound Y forms junctions (sides) connecting the nodes or crosslinks. Pores are formed in regions surrounded by some nodes or crosslinks and some junctions. The polymer may be a polymer (high-molecular-weight crosslinked product) having a branched structure (preferably a hyper-branched structure) or a polymer composed of non-branched linear polymer molecules. Even a polymer composed of non-branched linear polymer molecules, segments in the polymer molecular chain give excluded volume effect, to inhibit or limit the penetration of one polymer molecule into a region of another polymer molecule, whereby a relatively loose packing structure is formed. This structure is also included in the "pore structure" as used herein.

At least one of Compounds A and B, or of compound C each have a bridged alicyclic skeleton or an aromatic skeleton as its central skeleton.

Representative examples of a bridged alicyclic ring or aromatic ring constituting the bridged alicyclic skeleton or aromatic skeleton as the central skeleton include rings of Formulae (4a) to (4o), and rings each composed of two or more (e.g., two or three) of these rings bonded to each other. In Formula (4j), "r" denotes an integer of 0 to 5 and is preferably an integer of 0 to 2.

Preferred examples of the bridged alicyclic skeleton or aromatic skeleton as the central skeleton include bridged alicyclic skeletons such as adamantane skeleton, biadamantane skeleton, tetraphenyladamantane skeleton, norbornane skeleton, tetramethylnorbornane skeleton, norbornene skeleton, and tetramethylnorbornene skeleton; and aromatic skeletons each containing one or more aromatic rings, such as tetraphenylmethane skeleton, benzene skeleton, naphthalene skeleton, and biphenyl skeleton. The molecular weight of the central skeleton moiety is, for example, about 40 to 1460, and preferably about 60 to 500.

The functional groups can be any ones that have reactivity, and representative examples thereof include carboxyl group, amino group, hydroxyl group, mercapto group, formyl group, silanol group, a halogen atom, carbanion, a substituted or unsubstituted ethynyl group, and a group composed of these groups. Each of these groups may be converted into another reactive derivative group and/or may be protected with a protecting group. Examples of the reactive derivative group include, typically for carboxyl group, a haloformyl group and acid anhydride group (this group is also classified as carboxyl group protected with a protecting group). The protecting group can be any protecting group commonly used in organic syntheses. Examples of the protected carboxyl group include alkoxycarbonyl groups such as methoxycarbonyl and ethoxycarbonyl groups; and substituted oxycarbonyl groups such as benzyloxycarbonyl group. Examples of the protected amino group include acylamino groups such as acetylamino group; amino groups protected with, for example, an alkylidene group, a cycloalkylidene group, or benzylidene group (imine derivatives); and amino groups protected with an alkoxycarbonyl group or aralkylcarboxyl group (carbamic acid ester derivatives). The amino group may be a mono-substituted amino group, in which one of the two hydrogen atoms in the amino group may be substituted with an alkyl group such as methyl group, or phenyl group. Examples of the protected hydroxyl group include hydroxyl groups protected with an acyloxy group such as acetyloxy group, or an aldehyde (acetal, and hemiacetal derivatives).

Examples of a combination of functional groups or moieties that react with each other to form a chemical bond include, but are not limited to, a combination of carboxyl group and amino group (to form an amide bond), a combination of carboxyl group and hydroxyl group (to form an ester bond), a combination of carboxyl group and mercapto group (to form a thioester bond), a combination of hydroxyl group and hydroxyl group (to form an ether bond), a combination of hydroxyl group and mercapto group (to form a thioether bond), a combination of two functional groups capable of forming a carbon-carbon bond, and a combination of two functional groups capable of forming a carbon-nitrogen bond; a combination of one carboxyl group with two amino groups bonded to carbon atoms at the 1- and 2-positions or 1- and 3-positions (to form a five-membered or six-membered ring having two nitrogen atoms, such as imidazole ring), a combination of one formyl group with two amino groups bonded to carbon atoms at the 1- and 2-positions or 1- and 3-positions (to form a five-membered or six-membered ring having two nitrogen atoms, such as imidazole ring, under an oxidative condition such as an oxygen atmosphere), a combination of one carboxyl group with one amino group and one hydroxyl group bonded to carbon atoms at the 1- and 2-positions or 1- and 3-positions (to form a five-membered or six-membered ring having one nitrogen atom and one oxygen atom, such as oxazole ring), a combination of one carboxyl group with one amino group and one mercapto group bonded to carbon atoms at the 1- and 2-positions or 1- and 3-positions (to form a five-membered or six-membered ring having one nitrogen atom and one sulfur atom, such as thiazole ring), a combination of two carboxyl groups bonded to carbon atoms at the 1- and 2-positions or 1- and 3-positions with one amino group (to form a five-membered or six-membered imide ring), and a combination of substituted or unsubstituted ethynyl groups.

Preferably, the functional group or moiety of Compound A and a functional group or moiety of Compound B constitute a pair of functional groups or moieties capable of reacting with each other to form a heterocyclic ring or are both substituted or unsubstituted ethynyl-containing groups. The one functional group or moiety and the other functional group or moiety of Compound C constitute a pair of functional groups or moieties capable of reacting with each other to form a heterocyclic ring, or are both substituted or unsubstituted ethynyl-containing groups.

Preferably, the heterocyclic rings include benzimidazole ring, benzoxazole ring, and benzothiazole ring. Among them, benzimidazole ring and benzoxazole ring are more preferred. As the pair of functional groups or moieties capable of forming a heterocyclic ring, it is preferred that one is carboxyl group, a substituted oxycarbonyl group, formyl group, or a haloformyl group, and the other is 3,4-diaminophenyl group, 3-amino-4-hydroxyphenyl group, 4-amino-3-hydroxyphenyl group, 3-amino-4-mercaptophenyl group, or 4-amino-3-mercaptophenyl group. Examples of the substituted oxycarbonyl group include alkoxy-carbonyl groups whose alkoxy moiety has about one to six carbon atoms, such as methoxycarbonyl and ethoxycarbonyl groups.

Examples of the substituted or unsubstituted ethynyl-containing group include ethynyl group and ethynylphenyl group. When the functional groups or moieties are both substituted or unsubstituted ethynyl-containing groups, it is preferred that one of them is ethynyl group and the other is ethynylphenyl group.

Preferably, at least one of Compounds A and B has a thermally stable skeleton which has an aromatic-ring-containing divalent organic group and is positioned between the central skeleton and the functional groups or moieties. Preferably, Compound C has a thermally stable skeleton which is composed of an aromatic-ring-containing divalent organic group and is positioned between the central skeleton and the one functional group or moiety and/or between the central skeleton and the other functional group or moiety. Thus, the materials for producing insulating film contain at least one monomer component having a thermally stable skeleton and thereby give insulating films having high thermal stability.

Representative examples of the thermally stable skeleton of at least one of Compounds A and B, and the

thermally stable skeleton of Compound C include groups of Formulae (5a) to (5p), and groups each composed of two or more (e.g., two or three) of these groups bonded to each other. In these formulae, "s" denotes an integer of 0 to 5, and is preferably an integer of 0 to 2. For the groups having a hydroxyl group bonded to benzene ring, groups corresponding to these groups, except with mercapto group replacing the hydroxyl group are also preferred.

Preferably, at least one of Compounds A and B has a flexible unit which is composed of an organic group having a total of two to twenty atoms and containing at least one alkylene group or ether bond per molecule. Preferably, Compound C has a flexible unit which is composed of an organic group containing at least an alkylene group or ether bond and having a total of two to twenty atoms and is positioned between the central skeleton and the one functional group or moiety and/or between the central skeleton and the other functional group or moiety. Thus, the materials for producing insulating film contain at least one monomer component having a flexible unit which is easily movable upon reaction (upon polymerization), whereby the functional groups or moieties reliably react to prevent remaining of unreacted functional groups. The resulting insulating films thereby have a further lower relative dielectric constant and exhibit further less variation in relative dielectric constant.

Representative examples of the flexible units composed of an organic group containing at least an alkylene group or ether bond and having a total of two to twenty atoms, of at least one of Compounds A and B, or of Compound C include flexible units composed of groups of Formulae (6a) to (6j). In these formulae, "t" denotes an integer of 1 to 19, and is preferably an integer of 1 to 10; "u" denotes an integer of 1 to 10, and is preferably an integer of 1 to 5; "v" denotes an integer of 1 to 3, and is preferably an integer of 1 or 2; "w" denotes an integer of 1 to 16, and is preferably an integer of 1 to 8; "x" denotes an integer of 1 to 14, and is preferably an integer of 1 to 7; and each of "y" and "z" independently denotes an integer of 0 to 6, and is preferably an integer of 0 to 4, wherein both of "y" and "z" are not simultaneously zero (0).

Preferably, Compounds A, B and C satisfy the following condition (iii) or (iv) in which Compound A is a compound of Formula (1a) or a compound of Formula (1b), Compound B is a compound of Formula (2), and Compound C is a compound of Formula (3).

In Formula (1a), $X^1$ represents a di-, tri-, or tetra-valent bridged alicyclic group or aromatic group; $Y^{1a}$ and $Y^{1b}$ are the same as or different from each other and each represent a single bond, a divalent aromatic hydrocarbon group, a divalent heteroaromatic group, a divalent group corresponding to a precursor of the divalent heteroaromatic group, or a divalent group composed of two or more of these groups bonded to each other; $W^1$ represents a flexible unit composed of a divalent group containing at least an alkylene group or ether bond and having a total of two to twenty atoms; $Z^1$ represents a functional group or moiety capable of reacting with $Z^2$ in following Formula (2) to form a heterocyclic ring, or, only when $Z^2$ in Formula (2) is a substituted or unsubstituted ethynyl-containing group, $Z^1$ may represent a substituted or unsubstituted ethynyl-containing group; $R^1$ represents a hydrogen atom or a hydrocarbon group; "n1" denotes an integer of 2 to 4; and "n2" denotes an integer of 0 to 2, wherein the total of "n1" and "n2" equals 2 to 4, and wherein two or more $Y^{1a}$s, $Y^{1b}$s, $W^1$s, and $Z^1$s per molecule and two or more $R^1$s, if present per molecule, may be the same as or different from each other, respectively.

Examples of a bridged alicyclic ring or aromatic ring constituting the di-, tri-, or tetra-valent bridged alicyclic group or aromatic group as $X^1$ are the rings of Formulae (4a) to (4p) exemplified as the bridged alicyclic ring or aromatic ring constituting the bridged alicyclic group or aromatic group as the central skeletons of at least one of Compounds A and B, or of Compound C. Among them, bridged alicyclic rings are preferred, of which adamantane ring [ring of Formula (4a)] and biadamantane ring are more preferred. The group $X^1$ is preferably trivalent or tetravalent, and is more preferably tetravalent.

Examples of the divalent aromatic hydrocarbon groups as $Y^{1a}$ and $Y^{1b}$ include phenylene group and naphthylene group. Examples of an heteroaromatic ring constituting the divalent heteroaromatic group include benzimidazole ring, benzoxazole ring, and benzothiazole ring. Examples of the divalent group corresponding to a precursor of the divalent heteroaromatic group include groups capable of forming a heteroaromatic group as a result of ring closure (cyclization). Typically, a ring corresponding to a precursor of benzimidazole ring includes a ring having amino group and an acylamino group bonded to the ortho-positions of benzene ring. A ring corresponding to a precursor of benzoxazole ring includes a ring having hydroxyl group and an acylamino group bonded to the ortho-positions of benzene ring. A ring corresponding to a precursor of benzothiazole ring includes a ring having mercapto group and an acylamino group bonded to the ortho-positions of benzene ring.

Preferably, $Y^{1a}$ and $Y^{1b}$ are each independently a single bond or any of the groups of Formulae (5a) to (5p). These groups have been listed as representative examples of the thermally stable skeletons of at least one of Compounds A and B, or of Compound C. In Formulae (5a) to (5p), "s" denotes an integer of 0 to 5, and is preferably an integer of 0 to 2. For the groups having a hydroxyl group bonded to benzene ring, groups corresponding to these groups, except with mercapto group replacing the hydroxyl group are also preferred.

Examples of the flexible unit composed of a divalent group containing at least an alkylene group or ether bond and having a total of two to twenty atoms as $W^1$ are the flexible units composed of the groups of Formulae (6a) to (6j). These flexible units have been listed as representative examples of the flexible units of at least one of Compounds A

and B, or of compound C. In Formulae (6a) to (6j), "t" denotes an integer of 1 to 19, and is preferably an integer of 1 to 10; "u" denotes an integer of 1 to 10, and is preferably an integer of 1 to 5; "v" denotes an integer of 1 to 3, and is preferably an integer of 1 or 2; "w" denotes an integer of 1 to 16, and is preferably an integer of 1 to 8; "x" denotes an integer of 1 to 14, and is preferably an integer of 1 to 7; and each of "y" and "z" independently denotes an integer of 0 to 6, and is preferably an integer of 0 to 4, wherein both of "y" and "z" are not simultaneously zero (0).

The functional group or moiety as $Z^1$ capable of reacting with $Z^2$ to form a heterocyclic ring may be any of functional groups or moieties capable of reacting with $Z^2$ to form, for example, benzimidazole ring, benzoxazole ring, or benzothiazole ring. Examples thereof include 3,4-diaminophenyl group, 3-amino-4-hydroxyphenyl group, 4-amino-3-hydroxyphenyl group, 3-amino-4-mercaptophenyl group, and 4-amino-3-mercaptophenyl groups when $Z^2$ is carboxyl group, a substituted oxycarbonyl group, formyl group, or a haloformyl group; and include carboxyl group, a substituted oxycarbonyl group, formyl group, and a haloformyl group when $Z^2$ is 3,4-diaminophenyl group, 3-amino-4-hydroxyphenyl group, 4-amino-3-hydroxyphenyl group, 3-amino-4-mercaptophenyl group, or 4-amino-3-mercaptophenyl group. Examples of the substituted oxycarbonyl group include alkoxy-carbonyl groups whose alkoxy moiety has about one to six carbon atoms, such as methoxycarbonyl and ethoxycarbonyl groups.

Examples of the substituted or unsubstituted ethynyl-containing group as $Z^1$ include ethynyl group and ethynylphenyl group. When $Z^2$ is ethynyl group, $Z^1$ is preferably ethynylphenyl group; and when $Z^2$ is ethynylphenyl group, $Z^1$ is preferably ethynyl group.

Examples of the hydrocarbon group as $R^1$ include an aliphatic hydrocarbon group, an alicyclic hydrocarbon group, an aromatic hydrocarbon group, and a group composed of these groups bonded to each other. Examples of the aliphatic hydrocarbon group include linear or branched alkyl groups having about one to twenty carbon atoms, such as methyl, ethyl, propyl, isopropyl, butyl, isobutyl, s-butyl, t-butyl, pentyl, hexyl, decyl, and dodecyl groups, of which those having about one to ten carbon atoms are preferred, and those having about one to six carbon atoms are more preferred; linear or branched alkenyl groups having about two to twenty carbon atoms, such as vinyl, allyl, 1-butenyl, and 3-methyl-4-pentenyl groups, of which those having about two to ten carbon atoms are preferred, and those having about two to five carbon atoms are more preferred; and linear or branched alkynyl groups having about two to twenty carbon atoms, such as ethynyl, propynyl, 1-butynyl, and 2-butynyl group, of which those having about two to ten carbon atoms are preferred, and those having about two to five carbon atoms are more preferred.

Examples of the alicyclic hydrocarbon group include: monocyclic alicyclic hydrocarbon groups including cycloalkyl groups having about three to twenty members, such as cyclopropyl, cyclobutyl, cyclopentyl, cyclohexyl, and cyclooctyl groups (of which those having about three to fifteen members are preferred, and those having about three to twelve members are more preferred), and cycloalkenyl groups having about three to twenty members, such as cyclopropenyl, cyclobutenyl, cyclopentenyl, and cyclohexenyl groups (of which those having about three to fifteen members are preferred, and those having about three to ten members are more preferred); and bridged alicyclic hydrocarbon groups (bridged hydrocarbon groups) typically having a bridged alicyclic ring containing about two to four rings, such as adamantane ring, perhydroindene ring, decalne ring, perhydrofluorene ring, perhydroanthracene ring, perhydrophenanthrene ring, tricyclodecane ring, tricycloundecane ring, tetracyclododecane ring, perhydroacenaphthene ring, perhydrophenalene ring, norbornane ring, and norbornene ring. Examples of the aromatic hydrocarbon group include aromatic hydrocarbon groups having about six to twenty carbon atoms, such as phenyl and naphthyl groups, of which those having about six to fourteen carbon atoms are preferred.

Examples of a hydrocarbon group composed of an aliphatic hydrocarbon group and an alicyclic hydrocarbon group bonded to each other include cycloalkyl-alkyl groups such as cyclopentylmethyl, cyclohexylmethyl, and 2-cyclohexylethyl groups, of which $C_{3-20}$ cycloalkyl - $C_{1-4}$ alkyl groups are preferred. Examples of a hydrocarbon group composed of an aliphatic hydrocarbon group and an aromatic hydrocarbon group bonded to each other include aralkyl groups such as aralkyl groups having about seven to eighteen carbon atoms; and alkyl-substituted aryl groups, such as phenyl group or naphthyl group, substituted with about one to four alkyl groups each having one to four carbon atoms.

The aliphatic hydrocarbon group, alicyclic hydrocarbon group, aromatic hydrocarbon group, and group composed of these groups may each have one or more substituents. Such substituents are not particularly limited, as long as they do not adversely affect the reaction and properties of high-molecular-weight crosslinked products (polymers).

Of compounds of Formula (1a), compounds of Formula (7) are important. In Formula (7), $X^1$ represents a di-, tri-, or tetra-valent aromatic or non-aromatic cyclic group; $Y^{1a}$ and $Y^{1b}$ are the same as or different from each other and each represent a single bond, a divalent aromatic hydrocarbon group, a divalent heteroaromatic group, a divalent group corresponding to a precursor of the divalent heteroaromatic group, or a divalent group composed of two or more of these groups bonded to each other, wherein at least one of $Y^{1a}$ and $Y^{1b}$ is a divalent heteroaromatic group or a group containing a divalent group corresponding to a precursor of the divalent heteroaromatic group; $W^1$ represents a flexible unit composed of a divalent group containing at least an alkylene group or ether bond and having a total of two to twenty atoms; $Z^1$ represents carboxyl group, a substituted oxycarbonyl group, formyl group, a haloformyl group, a substituted or unsubstituted ethynyl-containing group, 3,4-diaminophenyl group, 3-amino-4-hydroxyphenyl group, 4-amino-3-hydroxyphenyl group, 3-amino-4-mercaptophenyl group, or 4-amino-3-mercaptophenyl group; $R^1$ represents a hydrogen atom

or a hydrocarbon group; "n1" denotes an integer of 2 to 4; and "n2" denotes an integer of 0 to 2, wherein the total of "n1" and "n2" equals 2 to 4, and wherein two or more $Y^{1a}$s, $Y^{1b}$s, $W^1$s, and $Z^1$s per molecule and two or more $R^1$s, if present per molecule, may be the same as or different from each other, respectively. Preferably, at least one of $Y^{1a}$ and $Y^{1b}$ is a divalent heteroaromatic group containing at least one of benzimidazole ring, benzoxazole ring, and benzothiazole ring, or a divalent group corresponding to a precursor of the divalent heteroaromatic group.

Compounds of Formula (7) can be synthetically prepared from known compounds as starting materials through known reactions such as condensation reactions, substitution reactions, addition reactions, oxidation reactions, and cyclization reactions.

Representative examples of the compounds of Formula (1a) include a compound represented by the following formula:

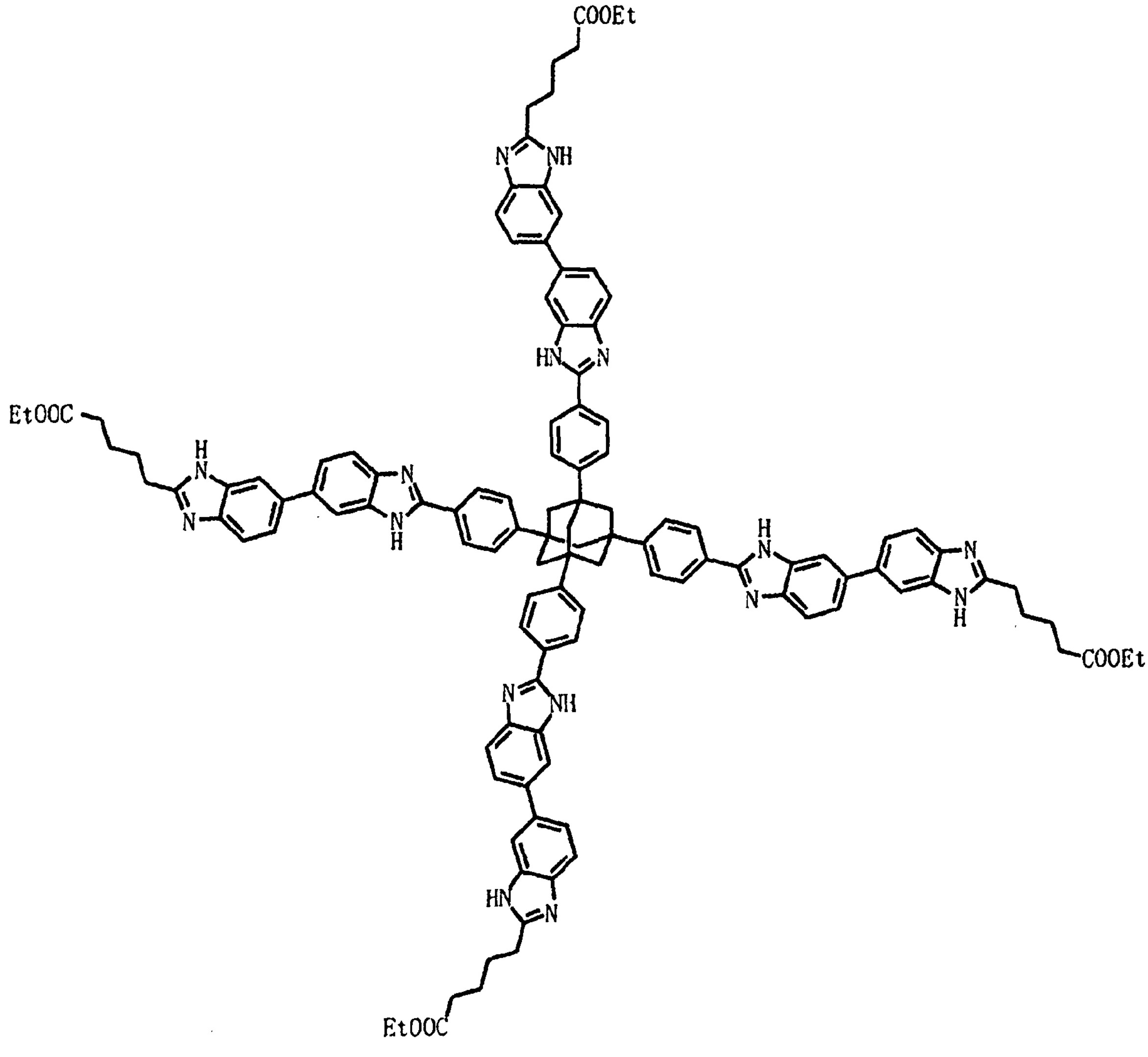

(1a-1)

In Formula (1b), $Y^1$ represents a single bond, a divalent aromatic hydrocarbon group, a divalent heteroaromatic group, a divalent group corresponding to a precursor of the divalent heteroaromatic group, or a divalent group composed of two or more of these groups bonded to each other; W represents a flexible unit composed of a di-, tri-, or tetra-valent group containing at least an alkylene group or ether bond and having a total of two to twenty atoms; $Z^1$ represents a functional group or moiety capable of reacting with $Z^2$ in following Formula (2) to form a heterocyclic ring, or, only when $Z^2$ in Formula (2) is a substituted or unsubstituted ethynyl-containing group, $Z^1$ may represent a substituted or unsubstituted ethynyl-containing group; and "n" denotes an integer of 2 to 4, wherein two or more $Y^1$ s and $Z^1$ s per molecule

may be the same as or different from each other, respectively.

The divalent aromatic hydrocarbon group, divalent heteroaromatic group, and divalent group corresponding to a precursor of the divalent heteroaromatic group as $Y^1$ are as with the divalent aromatic hydrocarbon groups, divalent heteroaromatic groups, and divalent groups corresponding to a precursor of the divalent heteroaromatic groups as $Y^{1a}$ and $Y^{1b}$.

The flexible unit W composed of a di-, tri-, or tetra-valent group containing at least an alkylene group or ether bond and having a total of two to twenty atoms is as with the flexible unit $W^1$ composed of a divalent group containing at least an alkylene group or ether bond and having a total of two to twenty atoms. $Z^1$ is as defined above.

Of compounds of Formula (1b), compounds of Formula (8) are important. In Formula (8), $Y^1$ represents a divalent heteroaromatic group or a divalent group corresponding to a precursor of the divalent heteroaromatic group; W represents a flexible unit composed of a di-, tri-, or tetra-valent group containing at least an alkylene group or ether bond and having a total of two to twenty atoms; $Z^1$ represents carboxyl group, a substituted oxycarbonyl group, formyl group, a haloformyl group, a substituted or unsubstituted ethynyl-containing group, 3,4-diaminophenyl group, 3-amino-4-hydroxyphenyl group, 4-amino-3-hydroxyphenyl group, 3-amino-4-mercaptophenyl group, or 4-amino-3-mercaptophenyl group; and "n" denotes an integer of 2 to 4, wherein two or more $Y^1$s and $Z^1$s per molecule may be the same as or different from each other, respectively. Preferably, $Y^1$s are each a divalent heteroaromatic group containing at least one of benzimidazole ring, benzoxazole ring, and benzothiazole ring, or a divalent group corresponding to a precursor of the divalent heteroaromatic group.

Compounds of Formula (8) can be synthetically prepared from known compounds as starting materials through known reactions such as condensation reactions, substitution reactions, addition reactions, oxidation reactions, and cyclization reactions.

Representative examples of the compounds of Formula (1b) are compounds represented by the following formulae:

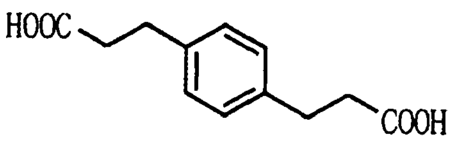

(1b-1)

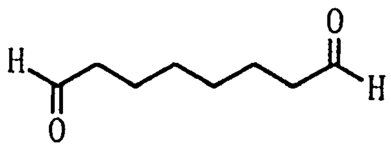

(1b-2)

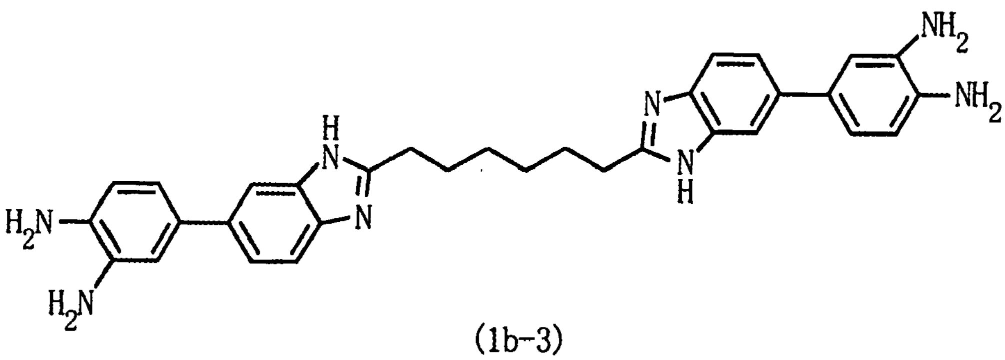

(1b-3)

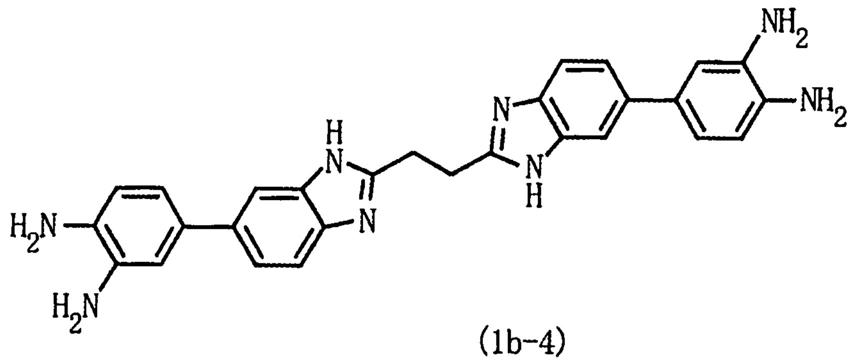

(1b-4)

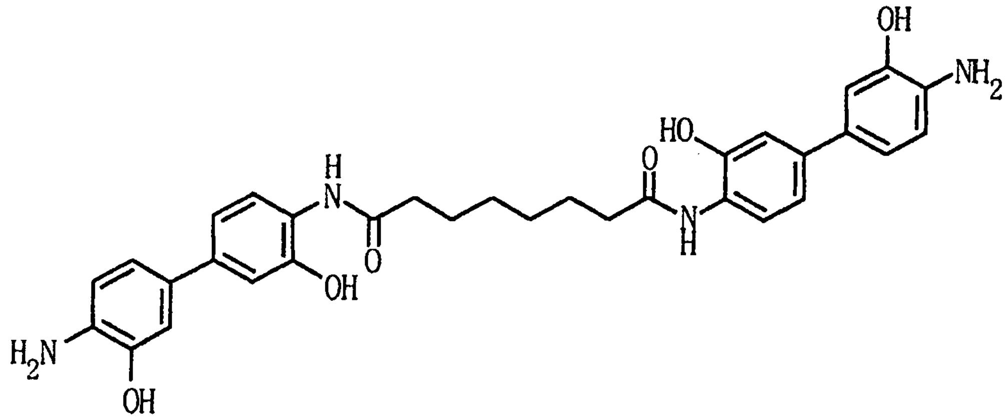

(1b-5)

In Formula (2), $X^2$ represents a di-, tri-, or tetra-valent bridged alicyclic group or aromatic group; $Y^{2a}$ and $Y^{2b}$ are the same as or different from each other and each represent a single bond, a divalent aromatic hydrocarbon group, a divalent heteroaromatic group, a divalent group corresponding to a precursor of the divalent heteroaromatic group, or a divalent group composed of two or more of these groups bonded to each other; $W^2$ represents a flexible unit composed of a divalent group containing at least an alkylene group or ether bond and having a total of two to twenty atoms; $Z^2$ represents a functional group or moiety capable of reacting with $Z^1$ in Formula (1a) or (1b) to form a heterocyclic ring, or, only when $Z^1$ in Formula (1a) or (1b) is a substituted or unsubstituted ethynyl-containing group, $Z^2$ may represent a substituted or unsubstituted ethynyl-containing group; $R^2$ represents a hydrogen atom or a hydrocarbon group; "m1" denotes an integer of 2 to 4; and "m2" denotes an integer of 0 to 2, wherein the total of "m1" and "m2" equals 2 to 4; "i" denotes 0 or 1; and "k" denotes 0 or 1, wherein two or more $Y^{2a}$s, $Y^{2b}$s, $W^2$s, and $Z^2$s per molecule and two or more $R^2$s, if present per molecule, may be the same as or different from each other, respectively.

The di-, tri-, or tetra-valent bridged alicyclic group and aromatic group as $X^2$ are as with the di-, tri-, or tetra-valent bridged alicyclic group and aromatic group as $X^1$. The divalent aromatic hydrocarbon groups, divalent heteroaromatic groups, and divalent groups corresponding to a precursor of the divalent heteroaromatic group as $Y^{2a}$ and $Y^{2b}$ are as with the divalent aromatic hydrocarbon groups, divalent heteroaromatic groups, and divalent groups corresponding to a precursor of the divalent heteroaromatic group as $Y^{1a}$ and $Y^{1b}$. The flexible unit $W^2$ composed of a divalent group containing at least an alkylene group or ether bond and having a total of two to twenty atoms is as with the flexible unit $W^1$ composed of a divalent group containing at least an alkylene group or ether bond and having a total of two to twenty atoms.

The functional group or moiety as $Z^2$ capable of reacting with $Z^1$ to form a heterocyclic ring may be a functional group or moiety capable of reacting with $Z^1$ to form, for example, benzimidazole ring, benzoxazole ring, or benzothiazole ring. Examples thereof include 3,4-diaminophenyl group, 3-amino-4-hydroxyphenyl group, 4-amino-3-hydroxyphenyl group, 3-amino-4-mercaptophenyl group, and 4-amino-3-mercaptophenyl group when $Z^1$ is carboxyl group, a substituted oxycarbonyl group, formyl group, or a haloformyl group; and include carboxyl group, a substituted oxycarbonyl group, formyl group, and a haloformyl group when $Z^1$ is 3,4-diaminophenyl group, 3-amino-4-hydroxyphenyl group, 4-amino-3-hydroxyphenyl group, 3-amino-4-mercaptophenyl group, or 4-amino-3-mercaptophenyl group. Examples of the substituted oxycarbonyl group include alkoxy-carbonyl groups whose alkoxy moiety has about one to six carbon atoms, such as methoxycarbonyl and ethoxycarbonyl groups.

Examples of the substituted or unsubstituted ethynyl-containing group as $Z^2$ include ethynyl group and ethynylphenyl group. When $Z^1$ is ethynyl group, $Z^2$ is preferably ethynylphenyl group; and when $Z^1$ is ethynylphenyl group, $Z^2$ is preferably ethynyl group.

The hydrocarbon group as $R^2$ is as with the hydrocarbon group as $R^1$.

Of compounds of Formula (2), compounds of Formula (9) are important. In Formula (9), $X^1$ represents a di-, tri-, or tetra-valent organic group; $Y^{1a}$, $Y^{1b}$, $Y^{2a}$, and $Y^{2b}$ are the same as or different from one another and each represent a single bond, a divalent aromatic hydrocarbon group, a divalent heteroaromatic group, a divalent group corresponding to a precursor of the divalent heteroaromatic group, or a divalent group composed of two or more of these groups bonded to each other, wherein at least one of $Y^{1a}$ and $Y^{1b}$ is a divalent heteroaromatic group or a group containing a divalent group corresponding to a precursor of the divalent heteroaromatic group; $W^1$ and $W^2$ are the same as or different from each other and each represent a flexible unit composed of a divalent group containing at least an alkylene group or ether bond and having a total of two to twenty atoms; each of $Z^1$ and $Z^2$ independently represents carboxyl group, a substituted oxycarbonyl group, formyl group, a haloformyl group, a substituted or unsubstituted ethynyl-containing group, 3,4-diaminophenyl group, 3-amino-4-hydroxyphenyl group, 4-amino-3-hydroxyphenyl group, 3-amino-4-mercaptophenyl group, or 4-amino-3-mercaptophenyl group; $R^1$ represents a hydrogen atom or a hydrocarbon group; "k" denotes 0 or 1; each of "p1" and "p2" independently denotes an integer of 1 to 3; and "p3" denotes an integer of 0 to 2, wherein the total of "p1", "p2", and "p3" equals 2 to 4, and wherein two or more $Y^{1a}$s, $Y^{1b}$s, $Y^{2a}$s, $Y^{2b}$s, $W^1$s, $W^2$s, $Z^1$s, $Z^2$s, and $R^1$s, if present per molecule, may be the same as or different from each other, respectively. Preferably, at least one of $Y^{1a}$ and $Y^{1b}$ is a divalent heteroaromatic group containing at least one of benzimidazole ring, benzoxazole ring, and benzothiazole ring, or a divalent group corresponding to a precursor of the divalent heteroaromatic group. In another preferred embodiment, at least one of $Y^{2a}$ and $Y^{2b}$ is a divalent heteroaromatic group containing at least one of benzimidazole ring, benzoxazole ring, and benzothiazole ring, or a divalent group corresponding to a precursor of the divalent heteroaromatic group.

Compounds of Formula (9) can be synthetically prepared from known compounds as starting materials through known reactions such as condensation reactions, substitution reactions, addition reactions, oxidation reactions, and cyclization reactions.

Representative examples of the compounds of Formula (2) are compounds represented by the following formula:

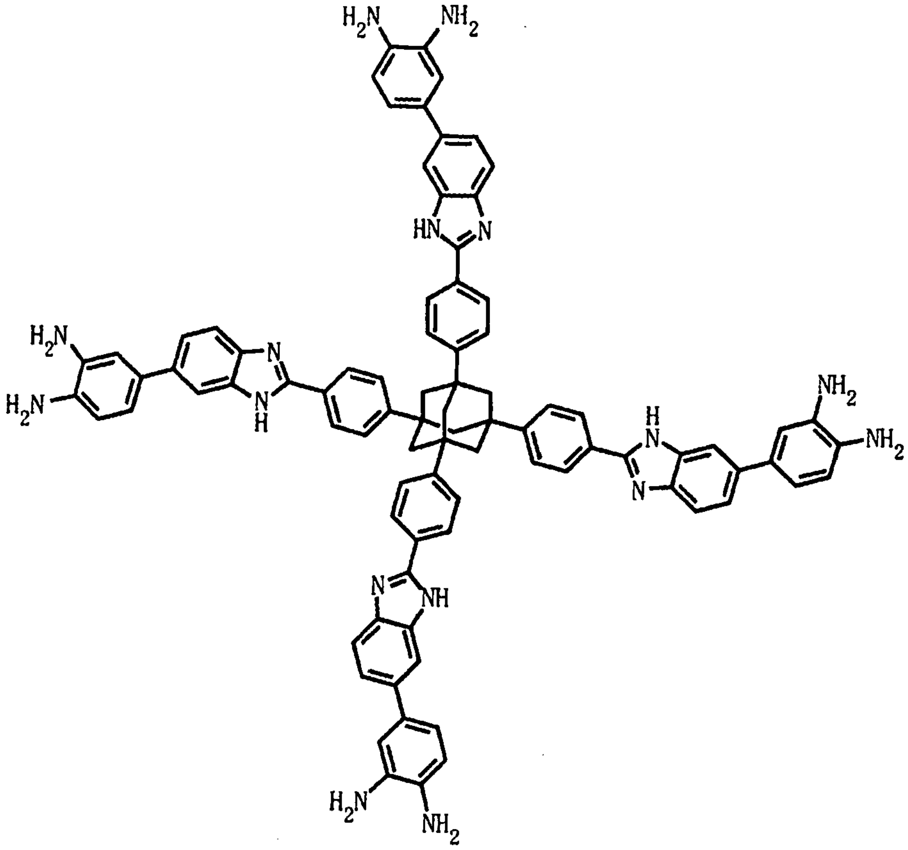

(2-1)

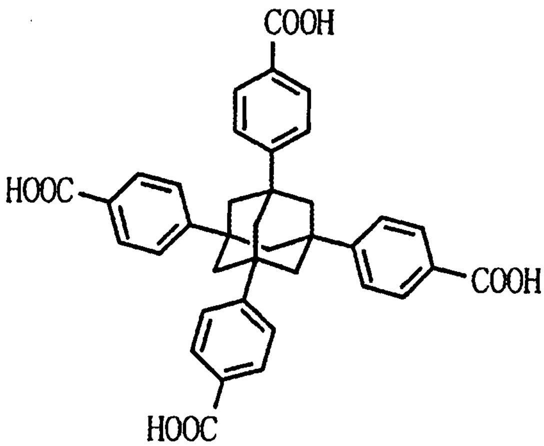

(2-2)

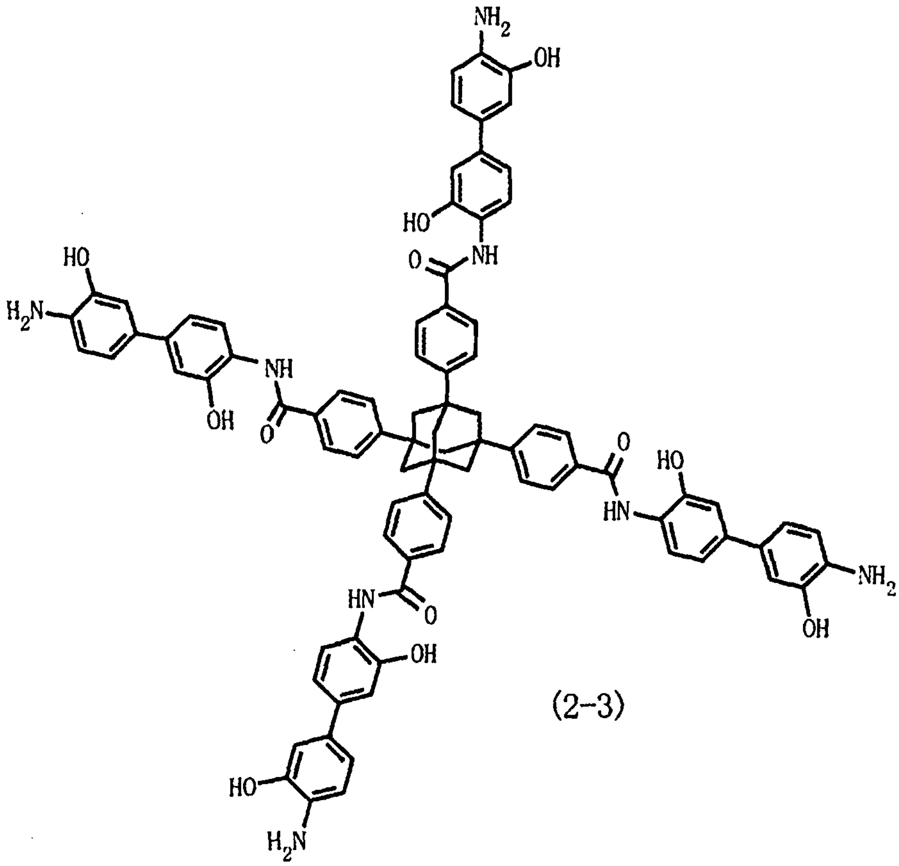

(2-3)

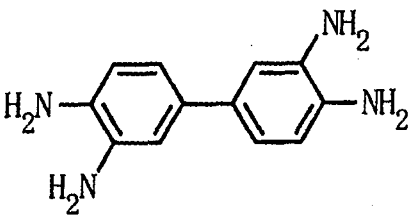

(2-4)

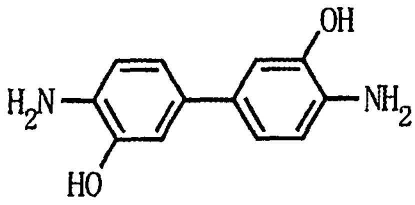

(2-5)

In Formula (3), $X^1$ represents a di-, tri-, or tetra-valent bridged alicyclic group or aromatic group; $Y^{1a}$, $Y^{1b}$, $Y^{2a}$, and $Y^{2b}$ are the same as or different from one another and each represent a single bond, a divalent aromatic hydrocarbon group, a divalent heteroaromatic group, a divalent group corresponding to a precursor of the divalent heteroaromatic group, or a divalent group composed of two or more of these groups bonded to each other; $W^1$ and $W^2$ are the same as or different from each other and each represent a flexible unit composed of a divalent group containing at least an alkylene group or ether bond and having a total of two to twenty atoms; $Z^1$ and $Z^2$ are a pair of functional groups or moieties capable of reacting with each other to form a heterocyclic ring, or $Z^1$ and $Z^2$ are both substituted or unsubstituted ethynyl-containing groups; $R^1$ represents a hydrogen atom or a hydrocarbon group; "k" denotes 0 or 1; each of "p1" and "p2" independently denotes an integer of 1 to 3; and "p3" denotes an integer of 0 to 2, wherein the total of "p1", "p2", and "p3" equals 2 to 4, and wherein two or more $Y^{1a}$s, $Y^{1b}$s, $Y^{2a}$s, $Y^{2b}$s, $W^1$s, $W^2$s, $Z^1$s, $Z^2$s, and $R^1$s, if present per molecule, may be the same as or different from each other, respectively.

The di-, tri-, or tetra-valent bridged alicyclic group and aromatic group as $X^1$, the divalent aromatic hydrocarbon group, divalent heteroaromatic group, and divalent group corresponding to a precursor of the divalent heteroaromatic group as $Y^{1a}$, $Y^{1b}$, $Y^{2a}$, and $Y^{2b}$, the flexible units composed of a divalent group containing at least an alkylene group or ether bond and having a total of two to twenty atoms as $W^1$ and $W^2$, and $Z^1$, $Z^2$, and $R^1$ are as defined above.

Compounds of Formula (3) can be synthetically prepared from known compounds as starting materials through known reactions such as condensation reactions, substitution reactions, addition reactions, oxidation reactions, and cyclization reactions.

A representative example of the compounds of Formula (3) is a compound represented by the following formula:

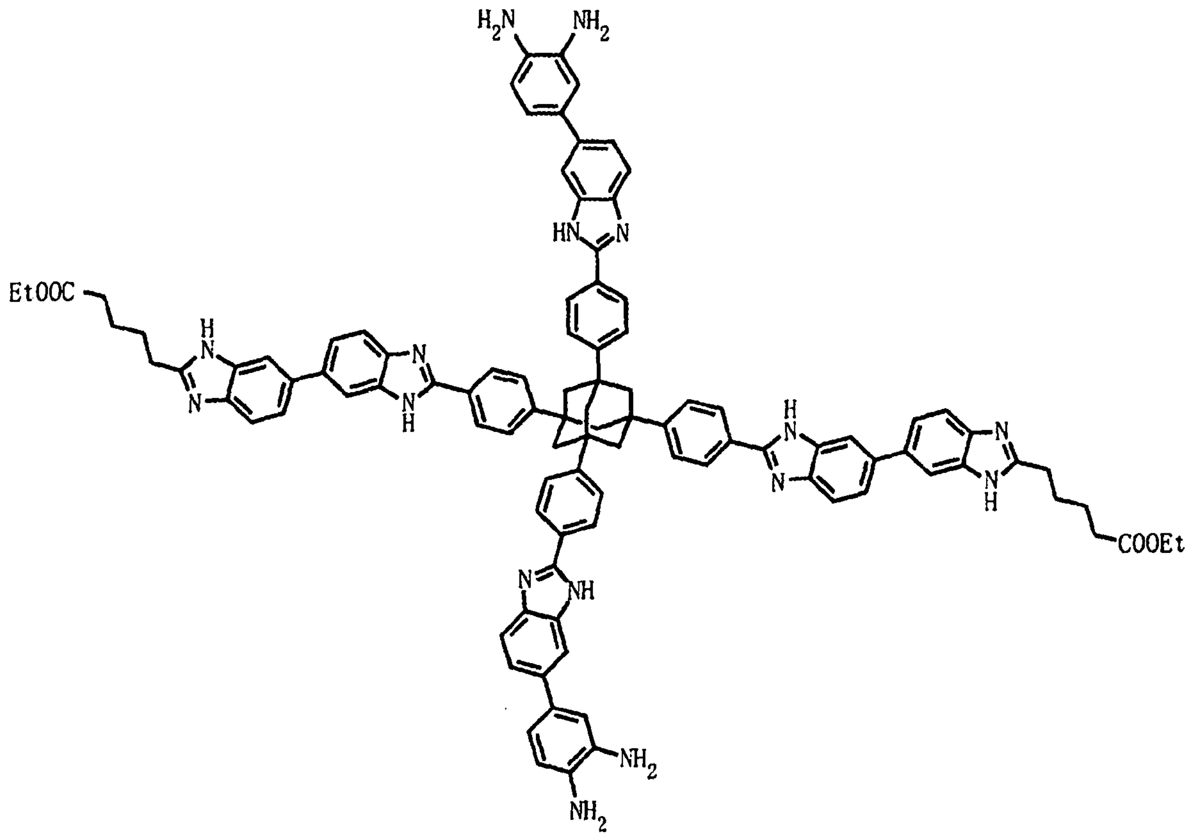

(3-1)

When a material for producing insulating film according to the present invention contains Compound A and Compound B, the ratio (molar ratio) of Compound A to Compound B is, for example, about 1:99 to 99:1, preferably about 10:90 to 90:10, and more preferably about 20:80 to 80:20. Compound A and Compound B may be used in equivalent amounts. Each of Compounds A, Compounds B, and Compounds C can be used alone or in combination, respectively.

Materials for producing a film according to the present invention include materials for producing a film comprising polymers (i) to (iii) dissolved in a solvent. The polymer

(i) is any of the N-substituted benzimidazole-containing bridged alicyclic compounds of Formula (1-1), the polymer (ii) is any of the N-substituted benzimidazole-containing bridged alicyclic compounds wherein "k2" in Formula (1-1) is 1 or 2 (Compound A') and a polyfunctional compound (Compound B') having two or more functional groups or moieties capable of reacting with the reactive functional group $X^3$ of the N-substituted benzimidazole-containing bridged alicyclic Compound A', and the polymer (iii) is any of the N-substituted benzimidazole-containing polymers. Each of these polymers to be dissolved in a solvent may be used alone or in combination.

In the polymer (ii), when the reactive functional group $X^3$ of Compound A' is a substituted or unsubstituted ethynyl group, Compound B' can be a compound having two or more (e.g., two to four) substituted or unsubstituted ethynyl groups per molecule. The substituted or unsubstituted ethynyl groups are as mentioned above. Examples of the compound herein include compounds each having two or more ethynyl groups (acetylene groups), such as 1,3,5,7-tetrakis(4-phenylacetylene)adamantane, 1,3,5-tris(4-phenylacetylene)adamantane, and 1,3,5-tris(4-phenylacetylene) benzene. Compound B' can also be a polymer containing a substituted or unsubstituted ethynyl group in its principle

chain or side chain. In a material for producing insulating film containing the ethynyl-containing Compound A' and the ethynyl-containing polymer, the ethynyl-containing Compound A' acts as a crosslinking agent. Typically, when a material for producing insulating film (coating composition) containing the ethynyl-containing Compound A' and the ethynyl-containing polymer is applied to a substrate and heated from room temperature to 600°C, preferably to 400°C, a crosslinking reaction proceeds to give a film having a crosslinked structure and exhibiting high thermal stability and high mechanical strength. Each of the ethynyl-containing Compounds A' and each of other ethynyl-containing compounds can be used alone or in combination, respectively.

Preferably, a material for producing insulating film according to the present invention may be used as a solution of any of the above-mentioned ethynyl-containing bridged alicyclic compounds, in combination with or without another above-mentioned ethynyl-containing compound, dissolved in an organic solvent.

Further, a material for producing insulating film according to the present invention may be used as a solution of above-mentioned Compounds A and B, and/or Compound C dissolved in an organic solvent.

Examples of the solvents are organic solvents including, for example, amides such as N,N-dimethylformamide, N,N-dimethylacetamide, and N-methyl-2-pyrrolidone; cyclic aminoacetals such as dimethylimidazolidine and dimethylimidazolidinone (dimethylimidazolidine-dione); sulfoxides such as dimethyl sulfoxide; sulfones; nitriles such as acetonitrile, propionitrile, and benzonitrile; ketones such as acetone, methyl ethyl ketone, diethyl ketone, methyl isobutyl ketone, cyclopentanone, and cyclohexanone; esters such as formic acid esters, acetic acid esters, propionic acid esters, benzoic acid esters, ethyl lactate, γ-butyrolactone, and propylene glycol monomethyl ether acetate (PGMEA); ethers such as dioxane, tetrahydrofuran, diethyl ether, ethylene glycol monoethyl ether, ethylene glycol diethyl ether, and propylene glycol monomethyl ether (PGME); alcohols such as methanol, ethanol, propanol, butanol, ethylene glycol, and propylene glycol; halogenated hydrocarbons such as dichloromethane, dichloroethane, chloroform, carbon tetrachloride, and chlorobenzene; nitro compounds such as nitromethane; aromatic hydrocarbons such as benzene, toluene, xylenes, ethylbenzene, and mesitylene; alicyclic hydrocarbons such as cyclohexane and methylcyclohexane; aliphatic hydrocarbons such as hexane, heptane, and octane; and mixtures of these solvents.

Preferably, the materials for producing a film of the present invention further contain one or more another components according to necessity. Examples of another components are the material components for use in the preparation of the respective components to be dissolved, the ethynyl-containing bridged alicyclic compounds, and Compounds A, Compounds B, and Compounds C.

Preferably, materials for producing a film further contain a catalyst typically for promoting polymerization and/or cyclization, as another component. Representative examples of the catalyst include acid catalysts such as sulfuric acid, methanesulfonic acid, and p-toluenesulfonic acid; and base catalysts.

The amount of catalysts is, for example, about 0 to 10 percent by mole, and preferably about 0 to 5 percent by mole, to the N-substituted benzimidazole-containing bridged alicyclic compound of Formula (1-1) [for the polymer (i)], to Compound A' and Compound B' [for the polymer (ii)], or to the N-substituted benzimidazole-containing polymer [for the polymer (iii)]. To the total amount of the ethynyl-containing bridged alicyclic compound and other ethynyl-containing compounds, the amount of catalysts is, for example, about 0 to 10 percent by mole and preferably about 0 to 5 percent by mole. To the total amount of Compounds A, B, and C and is, the amount of catalysts is, for example, about 0 to 10 percent by mole and preferably about 0 to 5 percent by mole.

Preferably, materials for producing a film further contain one or more thickeners for increasing the viscosity of the solution so as to improve coating ability. Representative examples of the thickeners include alkylene glycols and polyalkylene glycols, such as ethylene glycol, diethylene glycol, triethylene glycol, and polyethylene glycol. The amount of the thickener is, for example, about 0 to 20 percent by weight, and preferably about 0 to 10 percent by weight, based on the total amount of the material for producing a film.

Further, the materials for producing a film of the present invention, such as materials for producing insulating film, preferably contain one or more monocarboxylic acids for adjusting molecular weights after polymerization and/or one or more dicarboxylic acids for adjusting the degrees of crosslinking after polymerization. Representative examples of the monocarboxylic acids include monocarboxylic acids such as adamantanecarboxylic acid and benzoic acid; and monocarboxylic acid derivatives such as methyl adamantanecarboxylate and methyl benzoate. Representative examples of the dicarboxylic acids include dicarboxylic acids such as terephthalic acid; and dicarboxylic acid derivatives such as dimethyl terephthalate.

The amount of the monocarboxylic acids is, for example, about 0 to 10 percent by mole, and preferably about 0 to 5 percent by mole, to the total amount of monomer components (e.g., Compounds A and B and/or Compound C) constituting the material for producing a film. The amount of the dicarboxylic acids is, for example, about 0 to 100 percent by mole, and preferably about 0 to 50 percent by mole, to the total amount of monomer components (e.g., Compounds A and B and/or Compound C) constituting the material for producing a film.

Preferably, materials for producing a film, such as materials for producing insulating film, further contain one or more adhesion promoters for increasing adhesion of the film (e.g., insulating film) with a substrate. Representative examples of the adhesion promoters include trimethoxyvinylsilane, hexamethyldisilazane, γ-aminopropyltriethoxysilane,

and aluminum mono(ethyl acetoacetate) diisopropylate.

The amount of the adhesion promoters is, for example, about 0 to 10 percent by weight, and preferably about 0 to 5 percent by weight, to the N-substituted benzimidazole-containing bridged alicyclic compound of Formula (1-1) [for the polymer (i)], to Compound A' and Compound B' [for the polymer (ii)], or to the N-substituted benzimidazole-containing polymer [for the polymer (iii)] each constituting a material for producing a film. To the total amount of monomer components (the ethynyl-containing bridged alicyclic compound and other ethynyl-containing compounds) constituting a material for producing insulating film, the amount of the adhesion promoters is, for example, about 0 to 10 percent by weight, and preferably about 0 to 5 percent by weight. To the total amount of monomer components (Compounds A and B and/or Compound C) constituting a material for producing insulating film, the amount of the adhesion promoters is, for example, about 0 to 10 percent by weight, and preferably about 0 to 5 percent by weight.

The dissolving of monomer components (e.g., Compounds A and B and/or Compound C) and other components may be carried out typically in an air atmosphere, as long as the monomer components and other components are not oxidized, but is preferably carried out in an atmosphere of inert gas such as nitrogen or argon gas. A dissolving temperature of the monomer components and other components is not particularly limited and may be set depending typically on the solubility and stability of the monomer components and other components and the boiling point of the solvent. Typically, the dissolving may be carried out with heating and/or carried out at temperatures of, for example, about 0 to 200°C, and preferably about 10 to 150°C.

The concentration (total concentration) of a main component or components in a material for producing a film can be set according typically to the solubility of the main component or components, coatability, and workability, and is, for example, about 5 to 70 percent by weight, and preferably about 10 to 60 percent by weight. The main component or components herein are the N-substituted benzimidazole-containing bridged alicyclic compound of Formula (1-1) [for the polymer (i)]; Compound A' and Compound B' [for the polymer (ii)]; the N-substituted benzimidazole-containing polymer [for the polymer (iii)]; the ethynyl-containing bridged alicyclic compound and other ethynyl-containing compounds; and Compounds A and B and/or Compound C as monomer components.

Thin films according to the present invention as polymers having a pore structure, insulating films, and thin films can be prepared, for example, by applying any of the materials for producing a film (e.g., a material for producing insulating film) as a coating composition to a substrate; and carrying out a reaction, or drying, or heating. More specifically, they can be prepared typically by evaporating the solvent through heating or baking, and/or carrying out polymerization or cyclization.

Examples of the substrate include silicon wafers, metal substrates, and ceramic substrates. The application (coating) procedure is not particularly limited and can be a common procedure such as spin coating, dip coating, or spraying.

The heating temperature can be any temperature at which the solvent evaporates or the monomer components are converted into a polymer, and generally is about 25°C to 500°C (e.g., about 100°C to 500°C), preferably about 25°C to 450°C, and more preferably about 150°C to 450°C. The heating may be carried out at a constant temperature or at temperatures with stepwise or continuous gradient. The heating procedure may be carried out typically in an air atmosphere, as long as adversely affecting properties of the formed thin film, but is preferably carried out in an atmosphere of inert gas such as nitrogen or argon gas, or in a vacuum atmosphere.

When the monomer component or components contains an N-substituted benzimidazole-containing bridged alicyclic compound of Formula (1-1) wherein $X^3$ is a self-reactive functional group [for the polymer (i)]; or is Compound A' and Compound B' [for the polymer (ii)]), the heating causes evaporation of the solvent and further causes polymerization of the monomer component or components to give a polymer (polymerization product).

Typically, use of a tetrafunctional compound as a monomer component [including a compound of Formula (7-2) or Formula (9-1)] gives a network or reticulated polymer film having a structure of crosslinking in four directions with the central skeleton (e.g., adamantane skeleton) as a vertex (crosslink) and having a multiplicity of pores. This structure is composed of a unit in which three hexagons each commonly possess two sides. Examples of the tetrafunctional compound is a compound of Formula (1-1) in which "n4" is 4 and "m3" is 0; and a compound of Formula (7-2) or (9-1) in which "q" is 4 and "p" is 0. Use of a trifunctional compound as the monomer component gives a highly crosslinked polymer film having a structure of crosslinking in three directions with the central skeleton (e.g., adamantane skeleton) as a vertex (crosslink) and having a multiplicity of pores. This structure is composed of a unit in which three hexagons each commonly possess two vertexes or two sides. Examples of the trifunctional compound include a compound of Formula (1-1) in which "n4" is 3 and "m3" is 1; and a compound of Formula (7-2) or (9-1) in which "q" is 3 and "p" is 1. Use of a bifunctional compound as the monomer component gives a highly porous polymer film having a looser packing structure than that of a high-molecular-weight polymer prepared directly from a monomer mixture. This is because the excluded volume effect between segments of polymer molecular chain inhibits or limits the penetration of one polymer molecule into a region of another polymer molecule. Examples of the bifunctional compound include a compound of Formula (1-1) in which "n4" is 2 and "m3" is 2; and a compound of Formula (7-2) or (9-1) in which "q" is 2 and "p" is 2.

When a monomer contains an ethynyl-containing bridged alicyclic compound and other ethynyl-containing

compounds, the heating causes a monomer component or components to have a higher molecular weight typically through polycondensation in which, for example, an intermolecular reaction of terminal ethynyl groups of the ethynyl-containing bridged alicyclic compound and other ethynyl-containing compounds occurs. This gives a corresponding polymer (high-molecular-weight polymer). When the monomer component is a compound having a precursor structure of a final structure such as a heterocyclic ring, cyclization or another reaction generally proceeds with an increasing molecular weight of the monomer component, to give a polymer (high-molecular-weight polymer) having a desired structure. When the monomer component has a protecting group, molecular weight is increased, and/or cyclization proceeds accompanied by deprotection (leaving of the protecting group). The cyclization gives, for example, imidazole ring, benzimidazole ring, oxazole ring, benzoxazole ring, thiazole ring, or benzothiazole ring, from a precursor structure thereof.

Typically, use of a tetrafunctional compound as the monomer component gives a network or reticulated polymer film having a structure of crosslinking in four directions with the central skeleton (e.g., adamantane skeleton) as a vertex (crosslink) and having a multiplicity of pores. This structure is composed of a unit in which three hexagons each commonly possess two sides. Use of a trifunctional compound as the monomer component gives a highly crosslinked polymer film having a structure of crosslinking in three directions with the central skeleton (e.g., adamantane skeleton) as a vertex (crosslink) and having a multiplicity of pores. This structure is composed of a unit in which three hexagons each commonly possess two vertexes or two sides. Use of a bifunctional compound as the monomer component gives a highly porous polymer film having a looser packing structure than that of a high-molecular-weight polymer prepared directly from a monomer mixture. This is because the excluded volume effect between segments of polymer molecular chain inhibits or limits the penetration of one polymer molecule into a region of another polymer molecule.

When a tetrafunctional compound as the monomer component, such as the tetrafunctional compound of Formula (1) in which "n3" is 4 and "m" is 1, is used, a network or reticulated polymer film has a structure of crosslinking in four directions with the central skeleton (e.g., adamantane skeleton) as a vertex (crosslink) and has a multiplicity of pores. This structure is composed of a unit in which three hexagons each commonly possess two sides. Use of a trifunctional compound as the monomer component gives a highly crosslinked polymer film having a structure of crosslinking in three directions with the central skeleton (e.g., adamantane skeleton) as a vertex (crosslink) and having a multiplicity of pores. This structure is composed of a unit in which three hexagons each commonly possess two vertexes or two sides. An example of the trifunctional compound herein is a compound of Formula (1) in which "n3" is 3 and "m" is 1. Use of a bifunctional compound as the monomer component gives a highly porous polymer film having a looser packing structure than that of a high-molecular-weight polymer prepared directly from a monomer mixture. This is because the excluded volume effect between segments of polymer molecular chain inhibits or limits the penetration of one polymer molecule into a region of another polymer molecule. An example of the bifunctional compound herein is a compound of Formula (1) in which "n3" is 2 and "m" is 1.

When a tetrafunctional compound and/or trifunctional compound in combination with a bifunctional compound is used as monomer components, large pores due to long distances (sides) are formed between adjacent crosslinks (or nodes) to thereby yield a very low dielectric constant. More specifically, the tetrafunctional compound forms a crosslink having a three-dimensional structure branched in four directions, and the trifunctional compound forms a crosslink having a three-dimensional structure branched in three directions; whereby the tetrafunctional compound and/or trifunctional compound is bonded to the bifunctional compound to yield a polymer having a loose pore structure. Single use of a tetrafunctional compound (or trifunctional compound) may cause a high density due to formation of many crosslinks upon polymerization and may cause a higher relative dielectric constant due to reduced degree of freedom of molecule and formation of un-crosslinks. To avoid this, both a tetrafunctional compound and a trifunctional compound are advantageously used in combination to give steric hindrance. The two compounds give large pores formed as a result of polymerization with a bifunctional compound to thereby give a polymer having a low-density loose structure.

When two different tri- or tetra-functional compounds having functional groups capable of reacting with each other are used as monomer components, the two monomer components give steric hindrance with each other and thereby prevent reduction in density upon polymerization. This gives a polymer having a pore structure at the macromolecular level. Specifically, a tri- or tetra-functional compound used as a monomer component is a large molecule which is a tetrahedron (substantially regular tetrahedron) with the central skeleton (e.g., adamantane skeleton) as the center and has a sterically bulky structure (structure with a large volume).

Consequently, a bulky moiety, if introduced by N-alkylation of benzimidazole ring in between a central part of the regular tetrahedron (for example, central moiety of adamantane skeleton) and a terminal functional group, prevents penetration of another monomer into a space inside the tetrahedron structures and also limits entry typically of elongating oligomer and polymer molecules thereinto. This retains the inherent tetrahedron structures of the two monomer components to give a polymer. This polymer has a structure including regularly arrayed pores of sizes corresponding to the volumes of these tetrahedrons and has a low density. In addition, the moiety introduced into monomer molecules as a result of N-alkylation of benzimidazole ring has a low polarity, thereby has a low dielectric constant (about 2.0) and contributes to reduction in dielectric constant of the film.

In a polymerization reaction using these monomer components, the very large steric hindrance of the two tetrahedrons inhibits penetration of one tetrahedron structure into a space inside the other tetrahedron structure and also limits entry typically of elongating oligomer and polymer molecules thereinto. This retains the inherent tetrahedron structures of the two monomer components to give a polymer having a structure that includes regularly arrayed pores of sizes corresponding to the volumes of these tetrahedron and has a low density.

A thin film composed of a polymer thus formed contains a multiplicity of uniformly dispersed pores at the molecular level, thereby has a high porosity and has a low relative dielectric constant. In addition, the thin film also has a sufficient thermal stability and mechanical strength due to crosslinking and is significantly resistant to diffusion of copper from interconnections.

In particular, an N-substituted benzimidazole-containing polymer includes a bridged alicyclic skeleton as a central skeleton and has a structural unit containing an N-substituted benzimidazole ring as a repeating unit. In this embodiment, the substituent bonded to nitrogen atom acts to reduce the polarity and to prevent access of a low-molecular compound to the central skeleton as mentioned above. This gives a thin film having a very low moisture absorptivity and relative dielectric constant.

Additionally, a bridged alicyclic compound has a terminal ethynyl group that does not adversely affect the dielectric constant even if remains unreacted. This compound gives a thin film that shows less variation in relative dielectric constant (K value) and exhibits higher insulation.

Further, at least one monomer component has a flexible unit. The flexible unit acts to make functional groups more movable and ensure a reaction between the functional groups. This reduces unreacted moieties, lowers water-absorptivity due to unreacted amino groups and/or carboxyl groups, reduces variation in relative dielectric constant (K value), and increases insulation. It is believed that dielectric relaxation occurs when an alternating electric field is applied to a film to measure a dielectric constant. The dielectric relaxation is a phenomenon in which a movable unit in the film moves corresponding to the vibration of electric field and thereby absorbs electric field energy. A known thin film composed of a rigid skeleton alone contains large amounts of unreacted polar groups, thereby exhibits large dielectric relaxation, and shows considerable intra-sample variation in relative dielectric constant measurement. In contrast, a thin film according to the present invention containing a flexible unit is uniform and shows less variation in relative dielectric constant measurement, because it contains less amounts of unreacted polar moieties and exhibits less dielectric relaxation.

The thickness of thin films formed typically by heating can be set as appropriate according to the purpose and is generally about 50 nm or more (e.g., about 50 to 2000 nm), preferably about 100 nm or more (e.g., about 100 to 2000 nm), and more preferably about 300 nm or more (e.g., about 300 to 2000 nm). A film having a thickness of less than 50 nm may have insufficient electrical properties such as occurrence of leak current, may be difficult to smoothen by chemical-mechanical polishing (CMP) in semiconductor production processes, and may be unsuitable as an interlayer dielectric film.

Thin films according to the present invention show a low dielectric constant and high thermal stability, are usable typically as insulating films in electronic materials and components for semiconductor devices, and are particularly useful as interlayer dielectric films.

Examples

The present invention will be illustrated in further detail with reference to several examples below. It should be noted, however, these are never construed to limit the scope of the present invention. Thicknesses of polymer films were measured with an ellipsometer. Densities of the polymer films were determined by analysis of X-ray reflectance. Relative dielectric constants of the polymer films were measured in which an aluminum electrode was deposited on the surface of the films. Infrared absorption spectra were measured according to a thin-film transmission method. The symbols "s", "m", and "w" in the infrared absorption spectral data refer to "strong" absorption, "medium" absorption, and "weak" absorption. Weight-average molecular weights are in terms of polystyrene. Densities were measured at 25°C.

Preparation Example A1

Synthesis of Amino-Containing Adamantane Derivative of Formula (2-1):

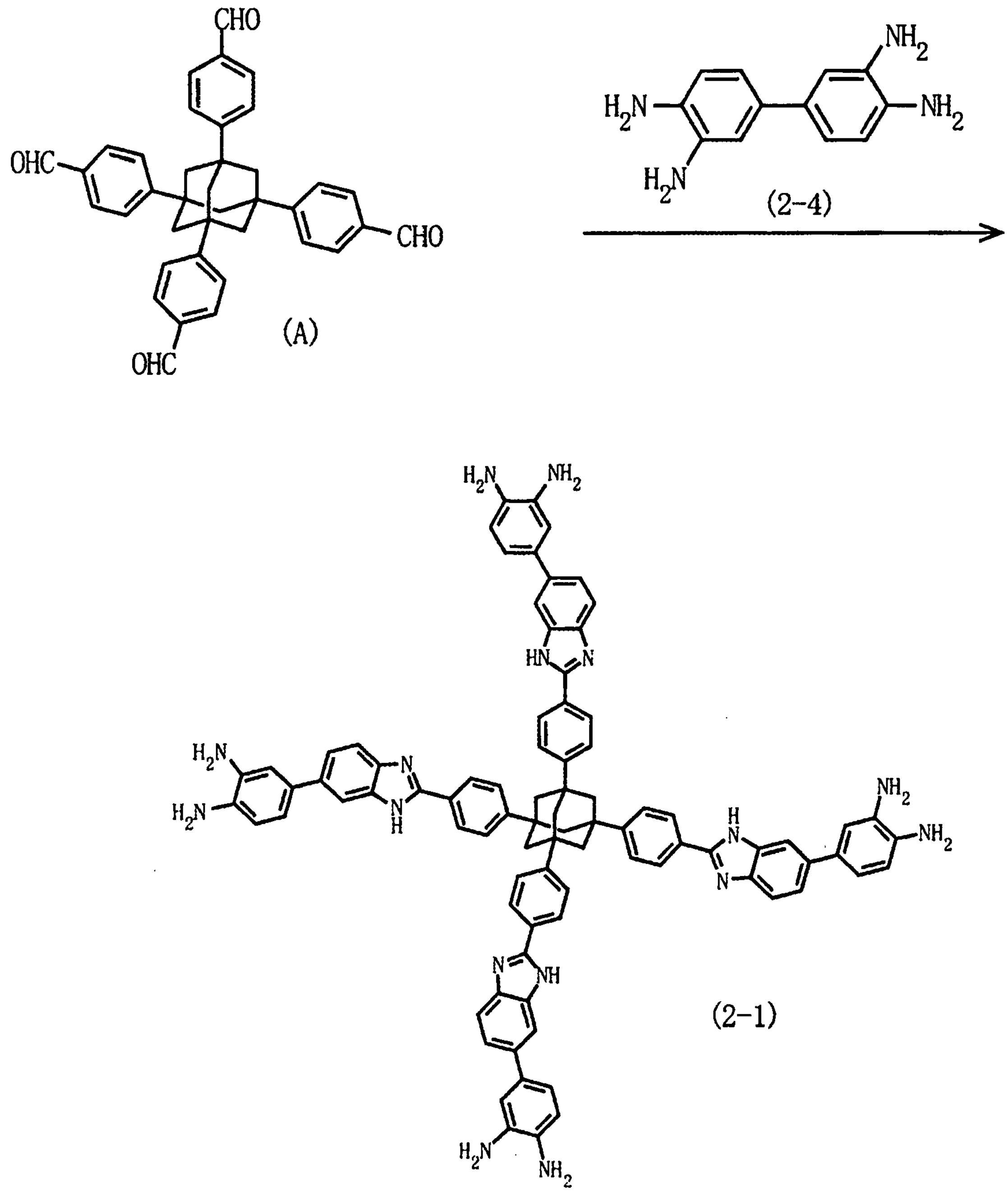

(A)

(2-4)

(2-1)

In a reactor (three-necked flask) was placed 77.68 g (0.362 mol) of 3,3'-diaminobenzidine of Formula (2-4), and this was combined with 307 g of N,N-dimethylacetamide (DMAc), and dissolved to give a solution, and the solution was held at 0°C or lower on an ice bath. To this solution was added dropwise, at a rate of 6 ml/min using a dropping funnel, another solution of 10.1 g (0.018 mol) of adamantane tetrakisbenzaldehyde of Formula (A) in 501 g of DMAc. The dropwise addition was conducted so that the temperature of the reaction mixture did not exceed 0°C. After the completion of dropwise addition, the dropping funnel was washed with 105 g of DMAc, and this washing was also added dropwise to the reaction mixture. While introducing a gaseous mixture of oxygen and nitrogen with an oxygen concentration of 5 percent by mole into the reaction mixture through a Teflon (registered trademark) tube, a reaction was conducted for 9 hours by heating the reactor on an oil bath to keep the liquid temperature at 90°C. After the completion of reaction, the reaction mixture was added dropwise to 9.13 kg of water in another reactor to give a slurry composed of precipitates and a supernatant. The slurry was stirred for about 1 hour after the completion of dropwise addition. Nitrogen gas was bubbled into the reaction mixture during stirring, so as to prevent oxidation of amine. The formed precipitates were collected by filtration, transferred again to the reactor, and combined with 1.83 kg of water, followed by washing with hot water by heating under reflux of water in a nitrogen atmosphere for 30 minutes. The precipitate

were collected by filtration before the temperature dropped, and were dried in a vacuum drier.

After the completion of drying, the precipitates were transferred to a reactor equipped with a tubular reflex condenser, and combined with 1.83 kg of tetrahydrofuran (THF), followed by washing with THF by heating under reflux of THF in a nitrogen atmosphere. The solids were collected by filtration again, and dried in a vacuum drier to give a product. The NMR spectrum and infrared absorption spectrum of the product were measured to give NMR spectral data in FIG. 1 and infrared absorption spectral data in FIG. 2. These data demonstrate that an amino-containing adamantane derivative of Formula (2-4) was formed. The amino-containing adamantane derivative was obtained in an amount of 24.5 g and a yield of 90%.

[NMR Spectral Data]

$^1$H-NMR (DMSO-d6) $\delta$ (ppm): 2.32 (12H <-$CH_2$->), 4.60 (16H <-$NH_2$>), 6.62-6.97 (12H <aromatic protons>), 7.53-7.78 (12H <aromatic protons>), 7.87 (8H), 8.24 (8H), 12.85 (4H)

[Infrared Absorption Spectral Data ($cm^{-1}$)]

3419 (N-H <stretching vibration>), 2933 (C-H of -$CH_2$-<stretching vibration>), 1623 (-C=N- <stretching vibration>), 1420-1520 (aromatic ring <in-plane vibration>), 1280

(aromatic -$NH_2$ <stretching vibration>)

Preparation Example A2

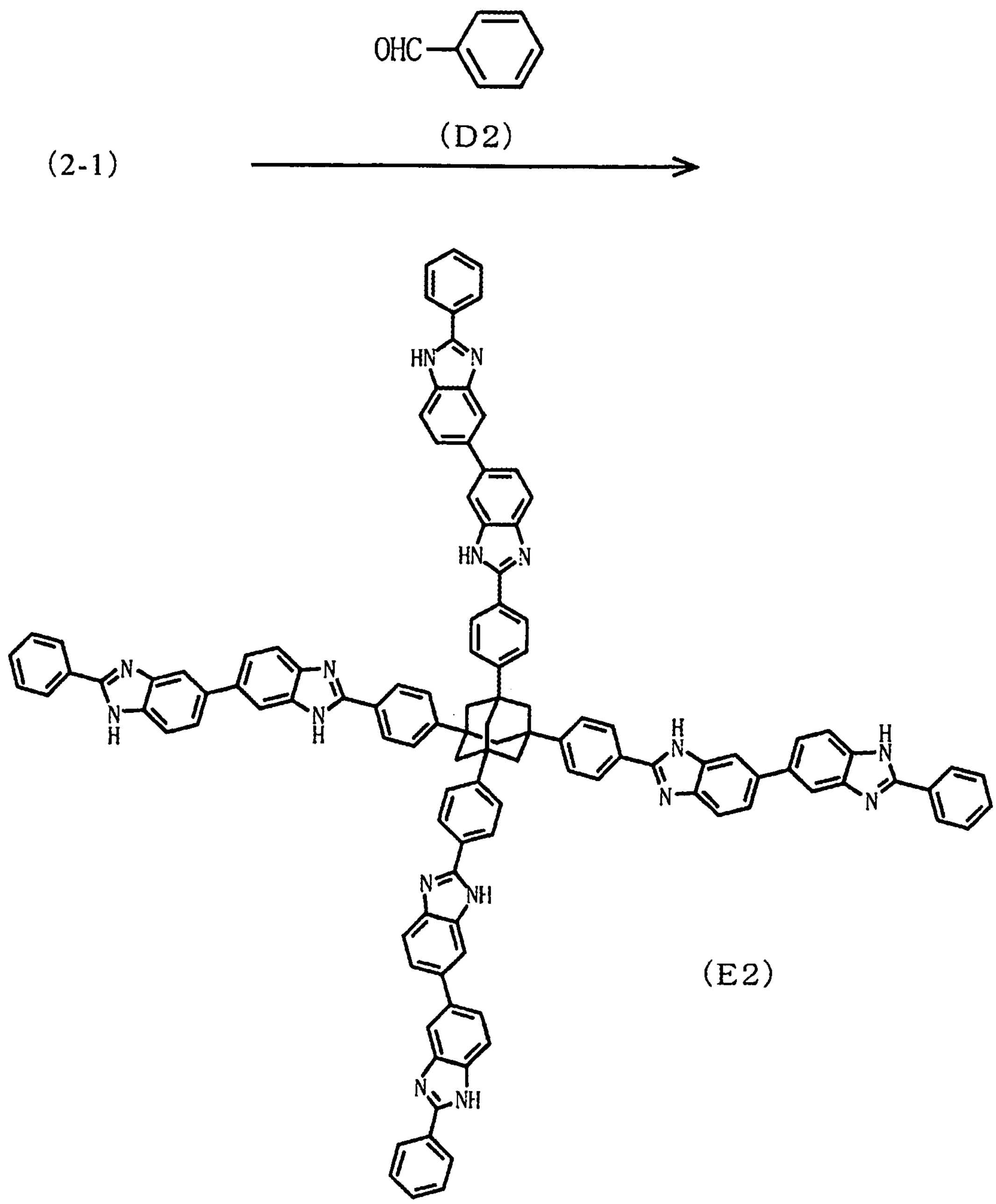

(2-1)

(D2)

(E2)

In a 100-mL two-necked eggplant flask was placed 6 g (4.5 mmol) of the amino-containing adamantane derivative of Formula (2-1), and this was combined with 100 g of N,N-dimethylacetamide (DMAc), followed by stirring at room temperature in a nitrogen atmosphere for 10 minutes to give a solution. The solution was combined with 2.0 g (19 mmol) of benzaldehyde of Formula (D2) added dropwise, raised in temperature to 100°C, and stirred for 13 hours with air bubbling. The reaction mixture was added dropwise to 500 mL of water, filtrated, and thereby yielded 6.5 g of an adamantane derivative of Formula (E2) (terminally capped compound) as a solid in a yield of 86%.

[NMR Spectral Data]

$^{1}$H-NMR (DMSO-d6) δ (ppm): 2.1-2.4 (12H), 7.4-8.4 (60H), 13.0 (8H)

Example A1

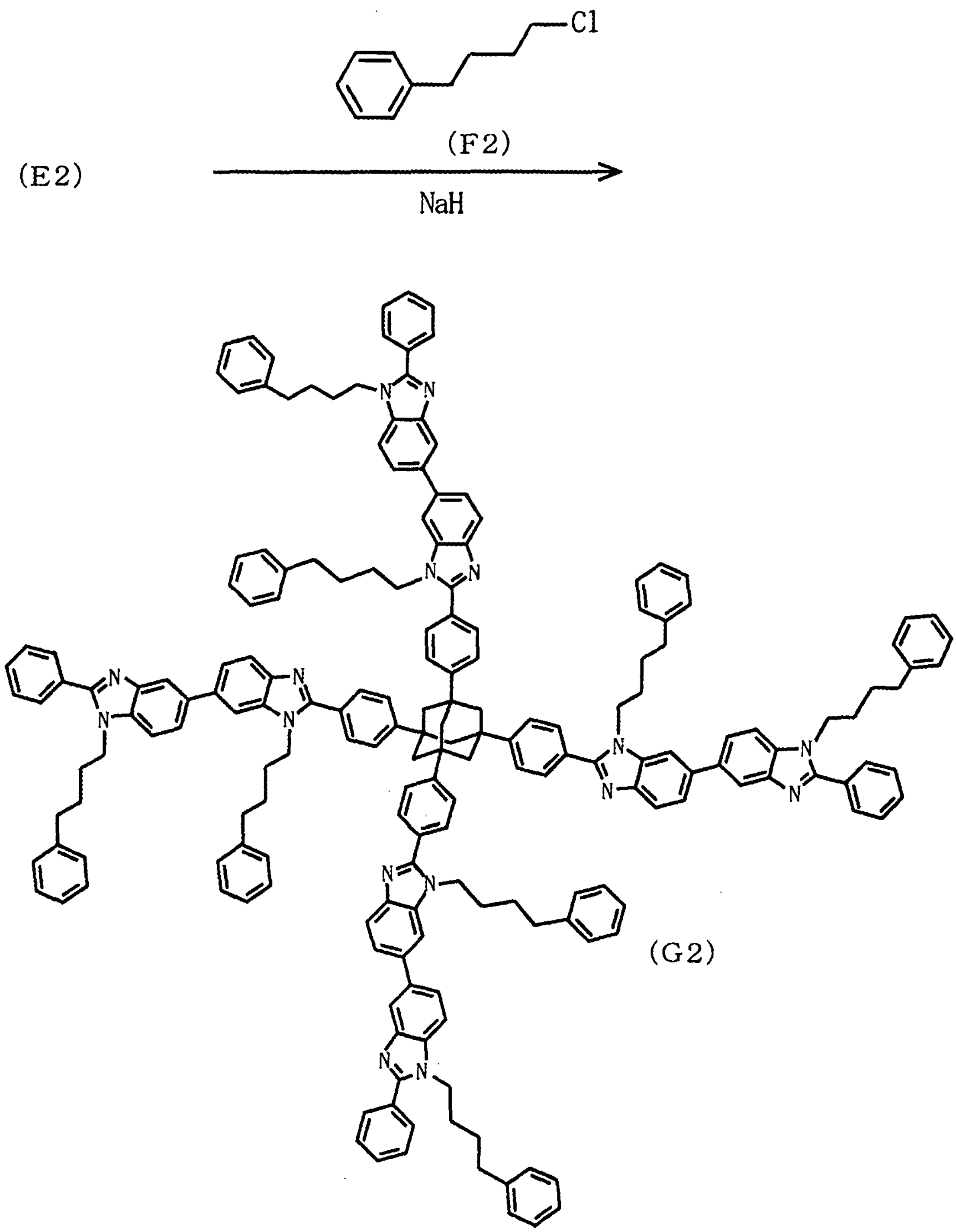


In a 50-mL two-necked eggplant flask was placed 0.48 g (20.0 mmol) of sodium hydride, and this was combined with 10 g of N,N-dimethylacetamide (DMAc) added at 0°C in a nitrogen atmosphere. In addition, a solution of 1.5 g (0.9 mmol) of the solid of Formula (E2) in 15 g of DMAc was prepared and added dropwise through a dropping funnel to the mixture in the eggplant flask at 0°C, followed by stirring at the same temperature for 30 minutes. To this was added dropwise 3.3 g (20.0 mmol) of 4-phenylbutyl chloride of Formula (F2), followed by stirring at 60°C for 10 hours. The reaction mixture was added dropwise to 200 mL of methanol, the mixture was filtrated and thereby yielded 2.2 g of an N-substituted benzimidazole-containing bridged alicyclic compound of Formula (G2) as a solid in a yield of 90%.

[NMR Spectral Data]

$^1$H-NMR (DMSO-d6) δ (ppm): 1.2 (16H), 1.5 (16H), 1.7 (16H), 2.2-2.5 (12H), 4.4 (16H), 6.8-8.0 (100H)

Preparation Example A3

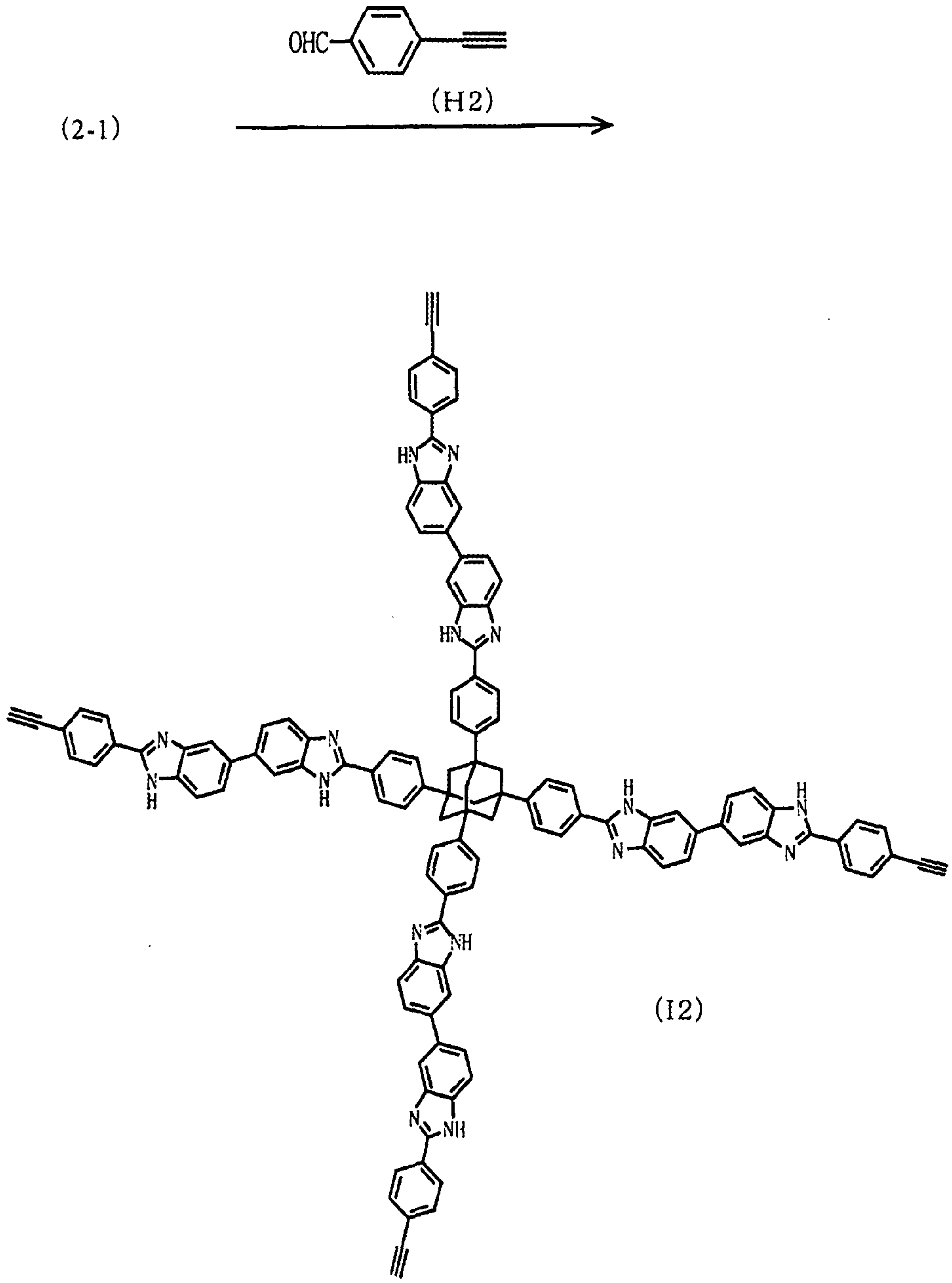
(2-1)

(H2)

(I2)

A solution of 2.08 g of 4-ethynylbenzaldehyde of Formula (H2) in 20 g of N,N-dimethylacetamide (DMAc) was placed in a reactor (three-necked flask). Another solution of 2.65 g of the amino-containing adamantane derivative of Formula (2-1) in 25 g of DMAc was added dropwise thereto at room temperature through a dropping funnel. After the completion of dropwise addition, the dropping funnel was washed with 10 g of DMAc, and this washing was also added dropwise to the mixture in the reactor. While introducing a gaseous mixture of oxygen and nitrogen with an oxygen concentration of 5 percent by mole into the reaction mixture through a Teflon (registered trademark) tube, a reaction was conducted for 7 hours by heating the reactor on an oil bath to maintain the liquid temperature at 80°C. After the completion of reaction, the reaction mixture was added dropwise to 800 g of water in another reactor to give a slurry

composed of precipitates and a supernatant. The slurry was stirred for about 1 hour after the completion of dropwise addition to give precipitates. The precipitates were collected by filtration, transferred again to the reactor, and combined with 400 g of methanol, followed by stirring for 1 hour. The precipitate were collected by filtration and dried in a vacuum drier. After the completion of drying, the precipitates were dissolved in 50 g of DMAc, and the solution was added dropwise to 400 g of methanol to give precipitates. The precipitate were collected by filtration and dried in a vacuum drier to give a product. The NMR spectrum and infrared absorption spectrum of the product were measured to find that an ethynyl-containing adamantane derivative of Formula (I2) was formed. The ethynyl-containing adamantane derivative was obtained in an amount of 3.09 g and a yield of 87%.

[NMR Spectral Data]

$^1$H-NMR (DMSO-d6) $\delta$ (ppm): 2.32 (12H <adamantane -$CH_2$->), 4.38 (4H <ethynyl C-H>), 7.54-8.26 (6H <aromatic ring C-H>), 13.05 (4H <imidazole N-H)

[Infrared Absorption Spectral Data ($cm^{-1}$)]

3422 (N-H <stretching vibration>), 2930 (C-H of -$CH_2$-<stretching vibration>), 2220 (ethynyl group <stretching vibration>, 1620 (-C=N- <stretching vibration>), 1420-1520 (aromatic ring <in-plane vibration>), 1280 (aromatic -N-H <stretching vibration>), 809 (C-H <out-of-plane deformation vibration>

Example A2

(I2)

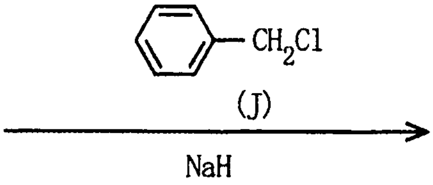


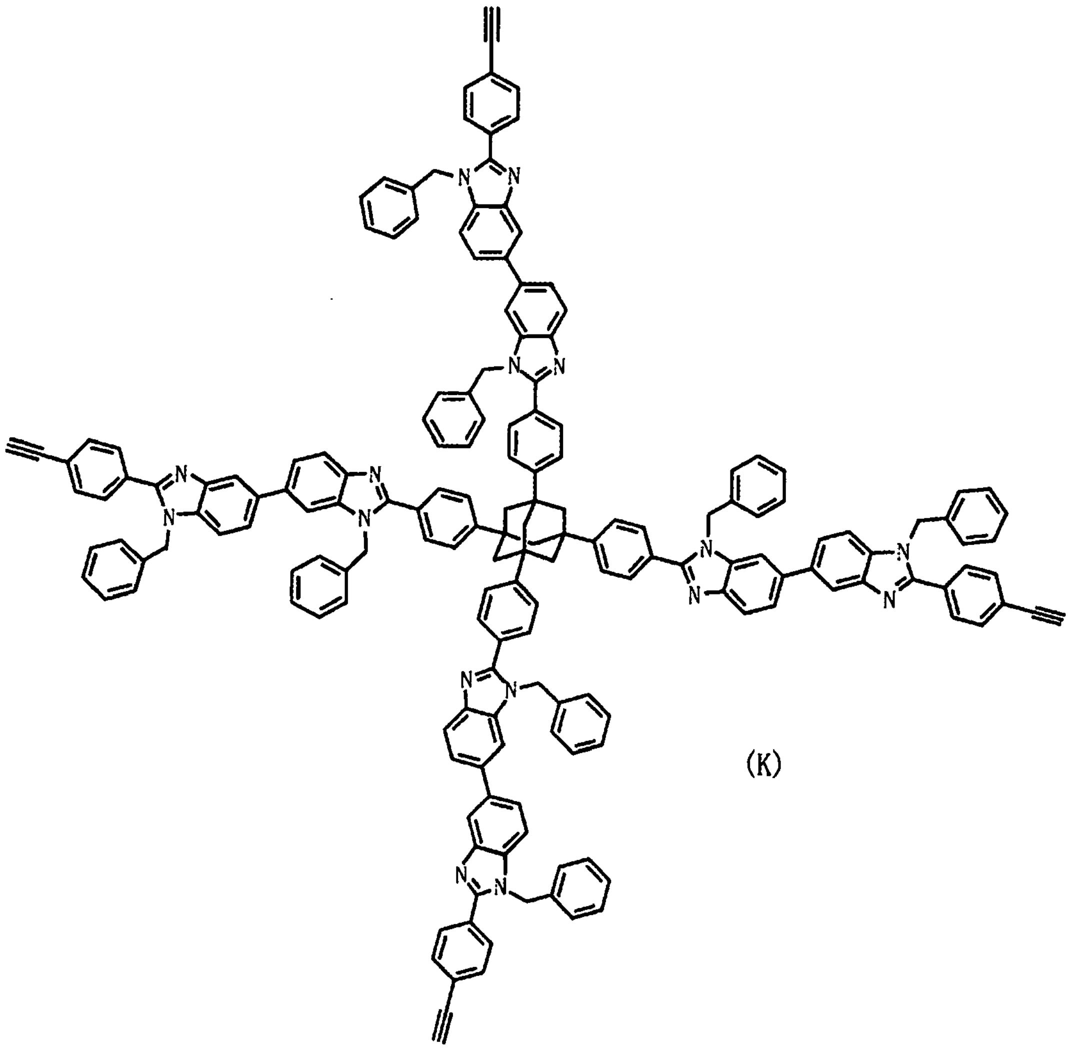

(K)

In a 50-mL two-necked eggplant flask was placed 0.48 g (20.0 mmol) of sodium hydride, and this was combined with 10 g of N,N-dimethylacetamide (DMAc) added at 0°C in a nitrogen atmosphere. A solution of 1.6 g (0.9 mmol) of the ethynyl-containing adamantane derivative of Formula (12) in 15 g of DMAc was prepared and added dropwise through a dropping funnel to the mixture in the eggplant flask at 0°C, followed by stirring at the same temperature for 30 minutes. To this was added dropwise 2.6 g (20.2 mmol) of benzyl chloride of Formula (J), followed by stirring at 60°C for 5 hours. The reaction mixture was added dropwise to 200 mL of water, the resulting mixture was filtrated and thereby yielded 2.0 g of an N-substituted benzimidazole-containing bridged alicyclic compound of Formula (K) as a solid in a yield of 90%.

[NMR Spectral Data]

$^1$H-NMR ($CDCl_3$) δ (ppm): 2.1-2.3 (12H), 3.2 (4H), 5.5 (16H), 7.1-8.1 (96H)

Example A3

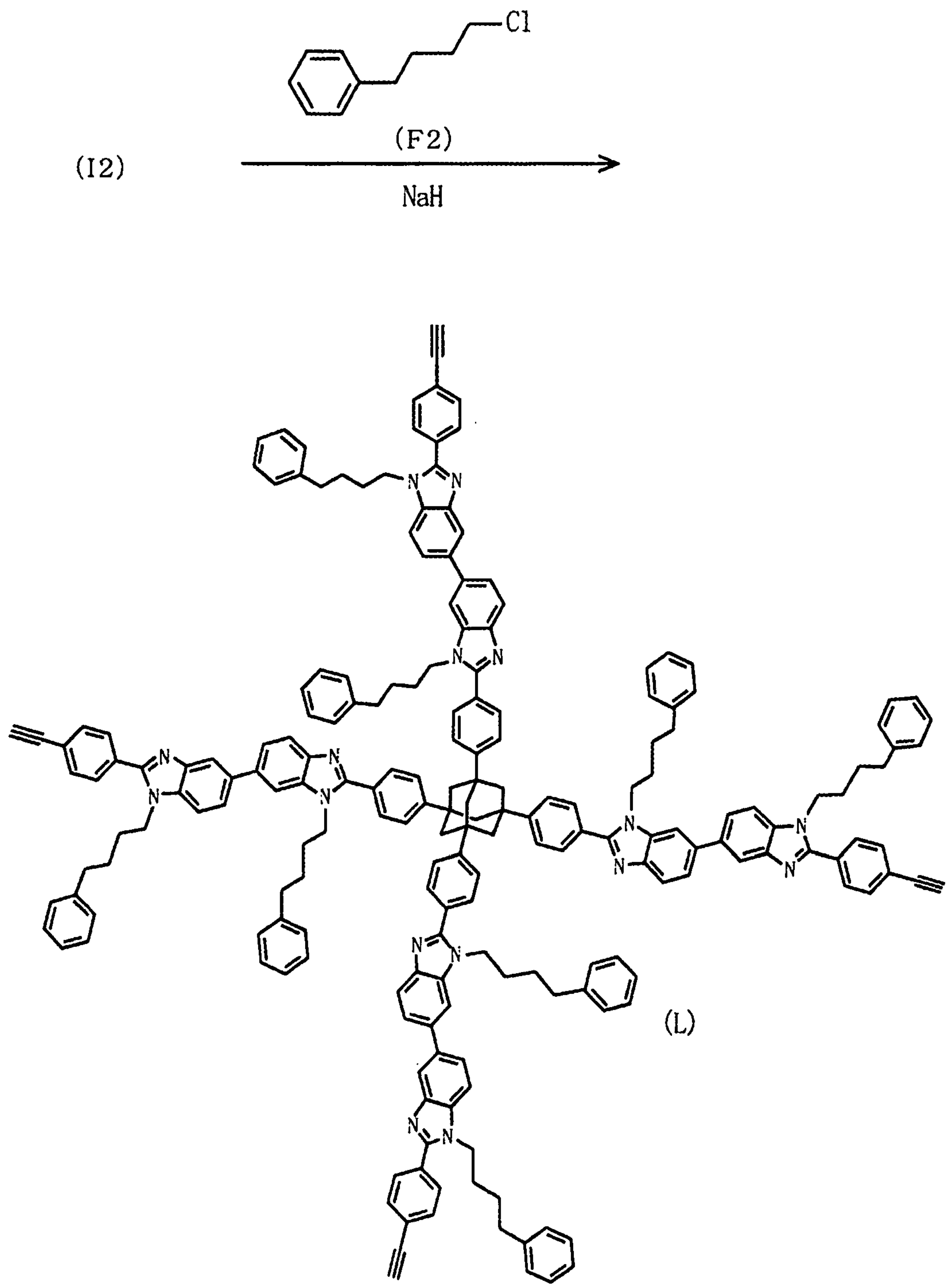


In a 50-mL two-necked eggplant flask was placed 0.48 g (20.0 mmol) of sodium hydride, and this was combined with 10 g of N,N-dimethylacetamide (DMAc) added at 0°C in a nitrogen atmosphere. In addition, a solution of 1.5 g (0.9 mmol) of the ethynyl-containing adamantane derivative of Formula (I2) in 15 g of DMAc was prepared and added dropwise through a dropping funnel to the mixture in the eggplant flask at 0°C, followed by stirring at the same temperature for 30 minutes. To this was added dropwise 3.3 g (20.2 mmol) of 4-phenylbutyl chloride of Formula (F2), followed by stirring

at 100°C for 10 hours. The reaction mixture was added dropwise to 200 mL of water to give precipitates, the precipitates were filtrated and thereby yielded 2.5 g of an N-substituted benzimidazole-containing bridged alicyclic compound of Formula (L) as a solid in a yield of 85%.

[NMR Spectral Data]

$^1$H-NMR ($CDCl_3$) δ (ppm): 1.6 (16H), 1.8 (16H), 2.2-2.5 (12H), 2.6 (16H), 3.2 (4H), 4.3 (16H), 6.8-8.0 (96H)

Preparation Example A4

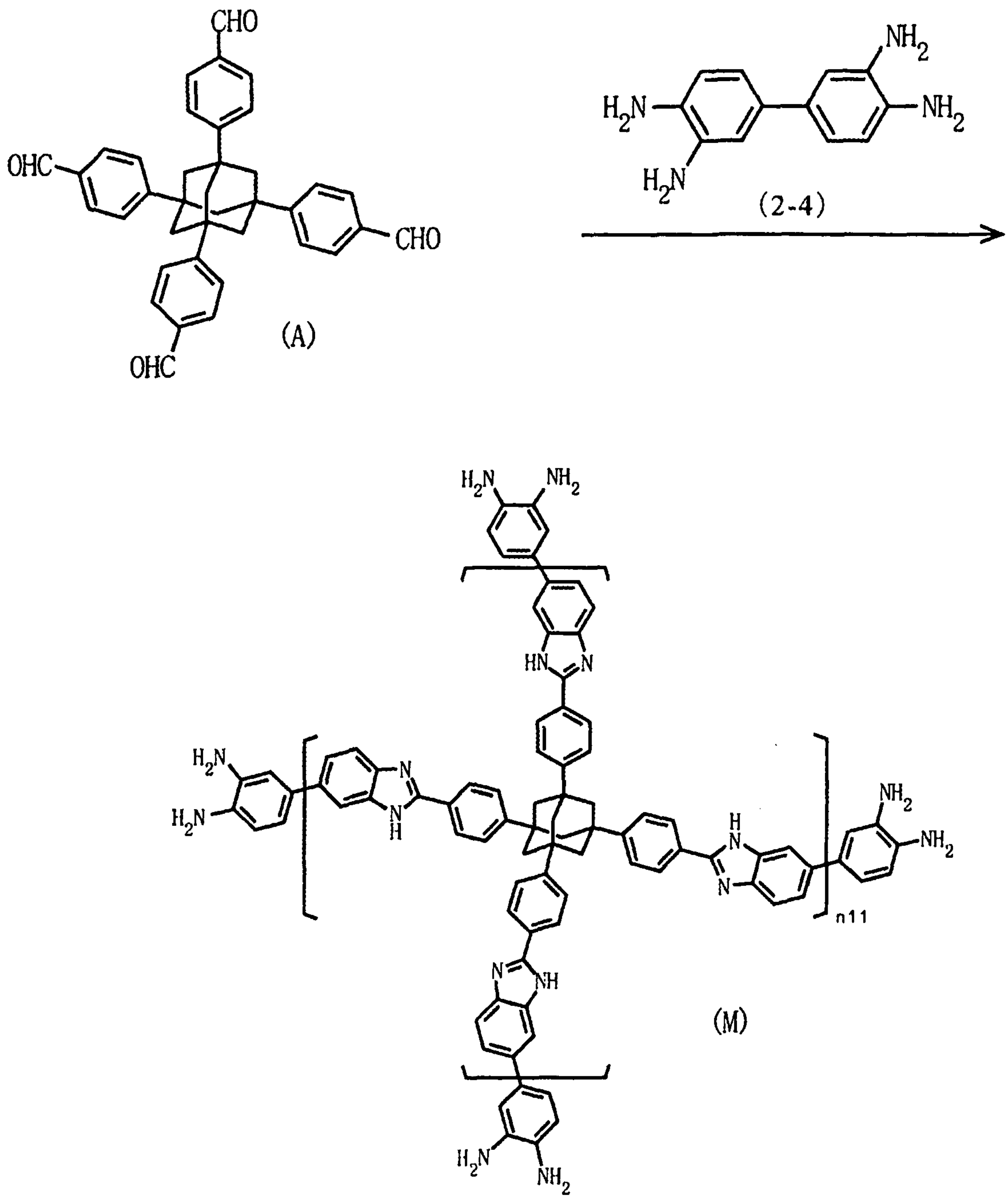
(A)
(2-4)
(M)

In a reactor (three-necked flask) was placed 3.54 g (16.1 mmol) of 3,3'-diaminobenzidine of Formula (2-4), and

this was dissolved in 32 g of N,N-dimethylacetamide (DMAc) to give a solution, and the solution was held at 0°C or lower on an ice bath, while blowing air thereinto through a Teflon (registered trademark) tube. Additionally, a solution of 10.1 g (1.6 mmol) of adamantane tetrakisbenzaldehyde of Formula (A) in 33 g of DMAc was added dropwise through a dropping funnel to the solution in the reactor over 1 hour so that the liquid temperature in the reactor did not exceed 0°C. After the completion of dropwise addition, the reactor was placed on an oil bath at 90°C, and a reaction was conducted with stirring for 6 hours. After the completion of reaction, the reaction mixture was added dropwise to 500 g of water to give a slurry composed of precipitates and a supernatant, and the slurry was stirred for about 1 hour after the completion of dropwise addition. Nitrogen gas was bubbled into the reaction mixture during stirring, so as to prevent oxidation of amine. The precipitates were collected by filtration, transferred again to the reactor, and combined with 1.83 kg of water, followed by washing with hot water by heating in a nitrogen atmosphere under reflux for 30 minutes. The precipitate were collected by filtration before the temperature dropped, dried in a vacuum drier, and thereby yielded 2.1 g of a polymer (precursor polymer) of Formula (M) having a weight-average molecular weight of 22000. In Formula (M), "n11" represents a positive integer (number of repeating units) (hereinafter the same).

Preparation Example A5

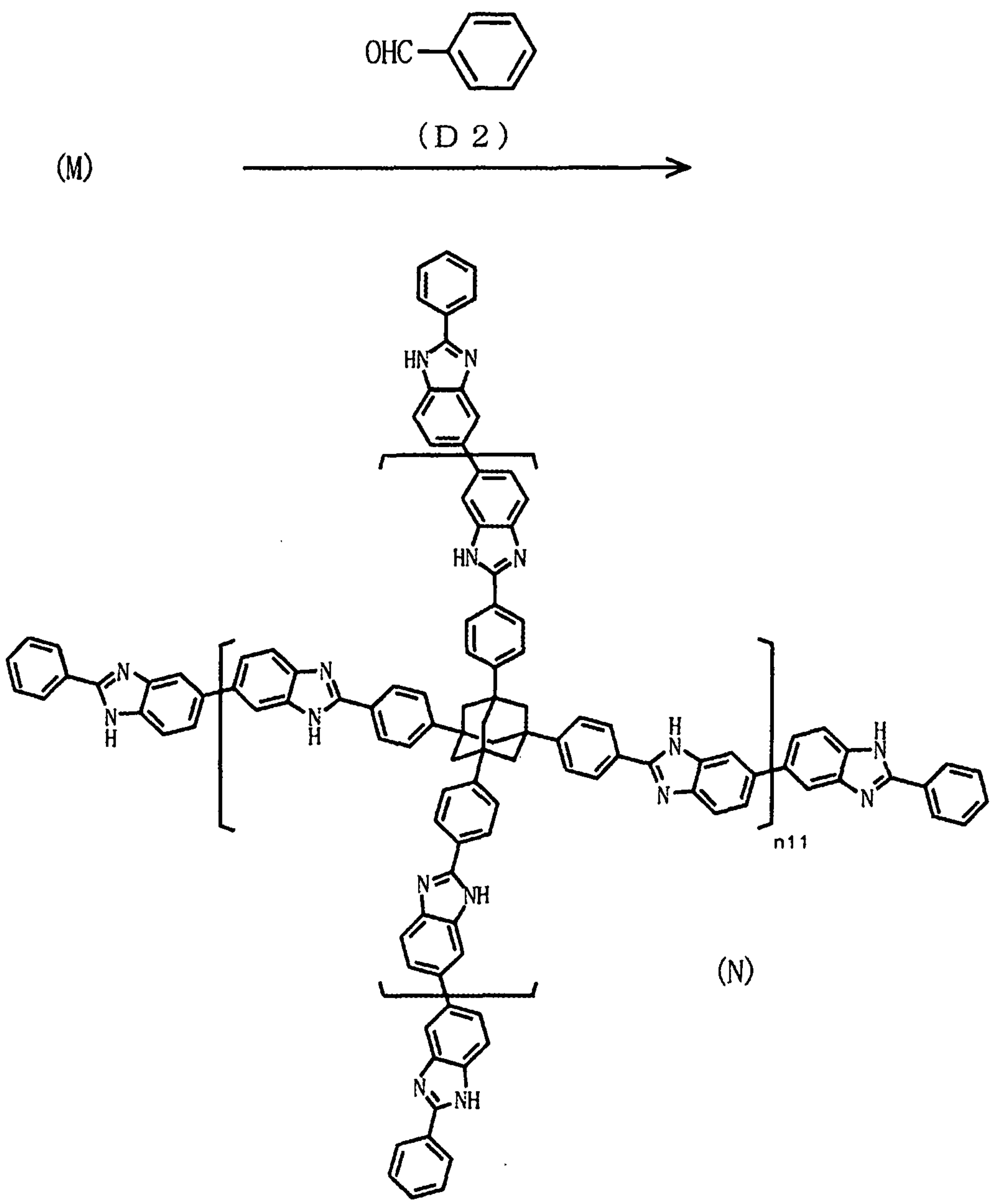
(M)
(D 2)
(N)

In a 100-mL two-necked eggplant flask was placed 2 g of the polymer (precursor polymer) of Formula (M), and 100 g of N,N-dimethylacetamide (DMAc) was added thereto, followed by stirring at room temperature in a nitrogen atmosphere for 10 minutes. To this solution was added dropwise 2.0 g (19 mmol) of benzaldehyde of Formula (D2). The mixture was raised in temperature to 100°C and stirred for 13 hours while bubbling air into the solution. The reaction mixture was added dropwise to 500 mL of water to give precipitates, the precipitates were collected by filtration, dried in a vacuum, and thereby yielded 1.9 g of a terminally capped polymer of Formula (N) as a solid in a yield of 90%.

[NMR Spectral Data]

$^1$H-NMR (DMSO-d6) δ (ppm): 2.1-2.4 (12H), 7.4-8.4 (60H), 13.0 (8H)

Example A4

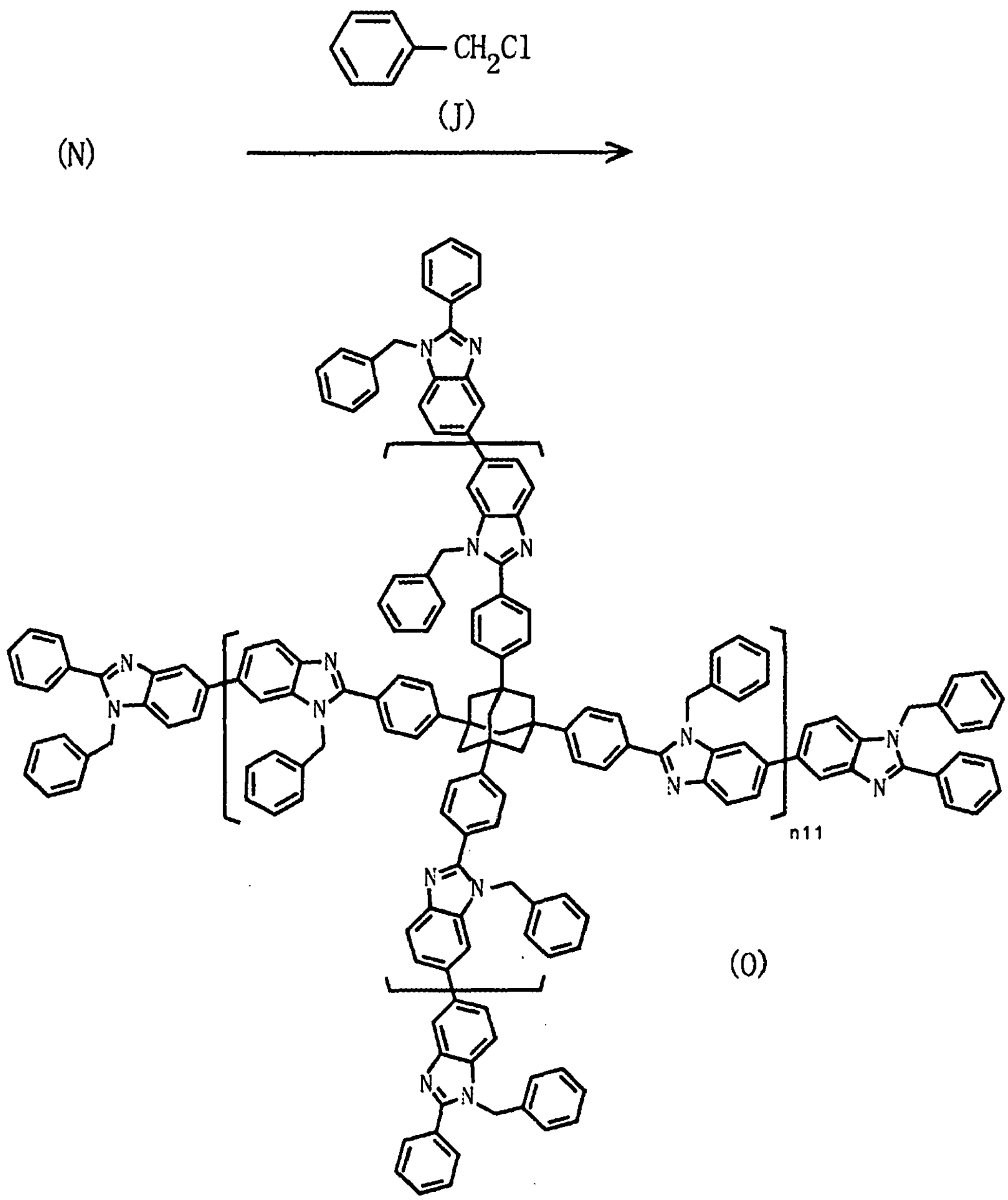
(N)
(J)
(O)

In a 50-mL two-necked eggplant flask was placed 0.48 g (20.0 mmol) of sodium hydride, and 10 g of N,N-dimethylacetamide (DMAc) was added thereto at 0°C in a nitrogen atmosphere. A solution of 1.5 g (0.9 mmol) of the terminally capped polymer of Formula (N) in 15 g of DMAc was prepared and added dropwise through a dropping funnel to the mixture in the eggplant flask at 0°C, followed by stirring at the same temperature for 30 minutes. To this was added dropwise 2.6 g (20.2 mmol) of benzyl chloride of Formula (J), followed by stirring at 60°C for 10 hours. The reaction mixture was added dropwise to 200 mL of methanol, the resulting mixture was filtrated and thereby yielded 2.0 g of an N-substituted benzimidazole-containing bridged alicyclic compound of Formula (O) as a solid in a yield of 90%.

[NMR Spectral Data]

$^1$H-NMR (DMSO-d6) δ (ppm): 1.2 (16H), 1.5 (16H), 1.7 (16H), 2.2-2.5 (12H), 4.4 (16H), 6.8-8.0 (100H)

Preparation Example A6

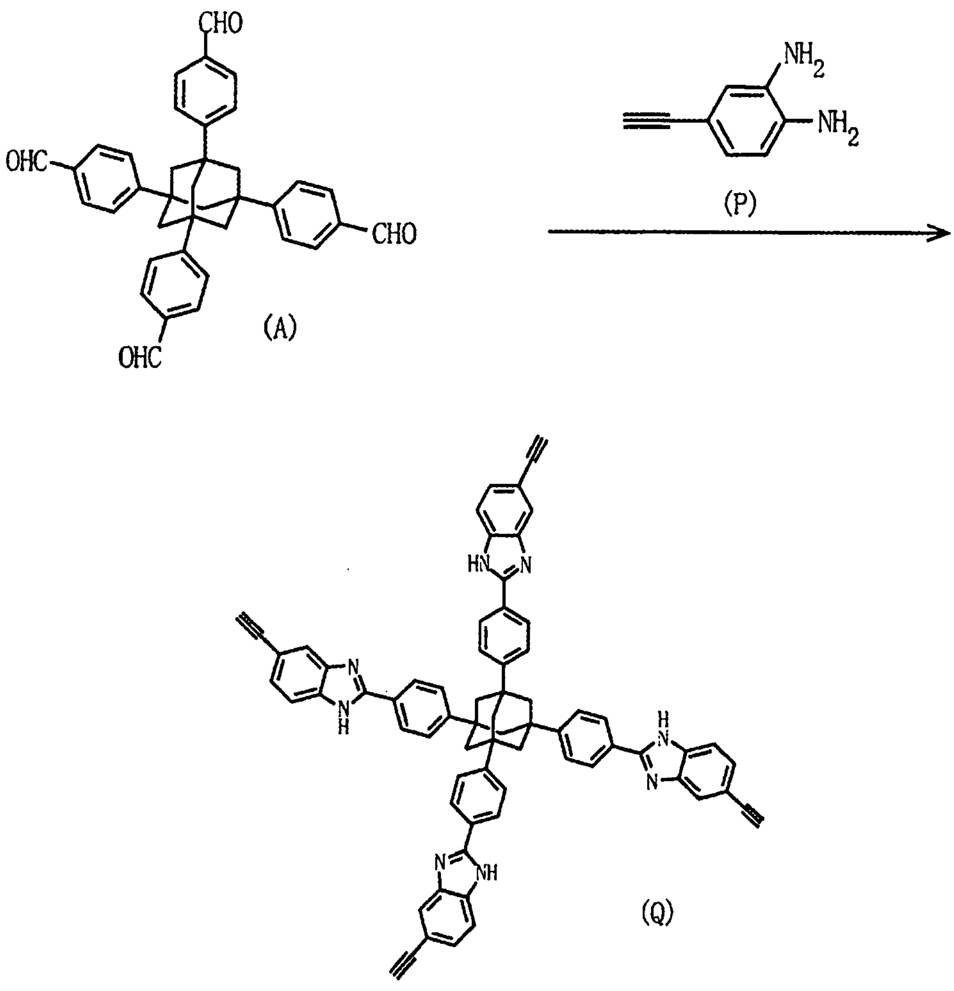

In a 1000-mL four-necked flask was placed 9.6 g (72.0 mmol) of 4-ethynyl-1,2-diaminobenzene of Formula (P), and 50 g of N,N-dimethylacetamide (DMAc) was added thereto to give a solution, and the solution was held at 25°C while blowing air into the solution through a Teflon (registered trademark) tube. The solution was combined with another solution of 5.0 g (9 mmol) of adamantane tetrakisbenzaldehyde of Formula (A) in 100 g of DMAc added dropwise over 1.5 hours through a dropping funnel. After the completion of dropwise addition, the mixture was stirred at 25°C for 1 hour, and raised in temperature to 80°C, followed by carrying out a reaction with stirring for 24 hours. After the completion of reaction, the reaction mixture was cooled to 25°C, and 600 g of pure water was added dropwise, to give a slurry composed of precipitates and a supernatant. The slurry was stirred for about 1 hour after the completion of dropwise addition to give precipitates. The precipitates were collected by filtration, transferred again to the reactor, and combined

with 600 g of methanol, followed by stirring for 1 hour. The precipitate were collected by filtration and dried in a vacuum drier. After the completion of drying, the precipitates were dissolved in 150 g of DMAc, and 600 g of methanol was added dropwise thereto to give precipitates. The precipitate were collected by filtration and dried in a vacuum drier to give a product. The $^1$H-NMR spectrum of the product was measured (see FIG. 3) to find that an ethynyl-containing adamantane derivative of Formula (Q) was formed. The ethynyl-containing adamantane derivative was obtained in an amount of 5.4 g and a yield of 60%.

[NMR Spectral Data]

$^1$H-NMR (DMSO-d6) δ (ppm): 2.1 (12H), 4.0-4.1 (4H), 7.2-8.2 (28H), 13.1 (4H)

Example A5

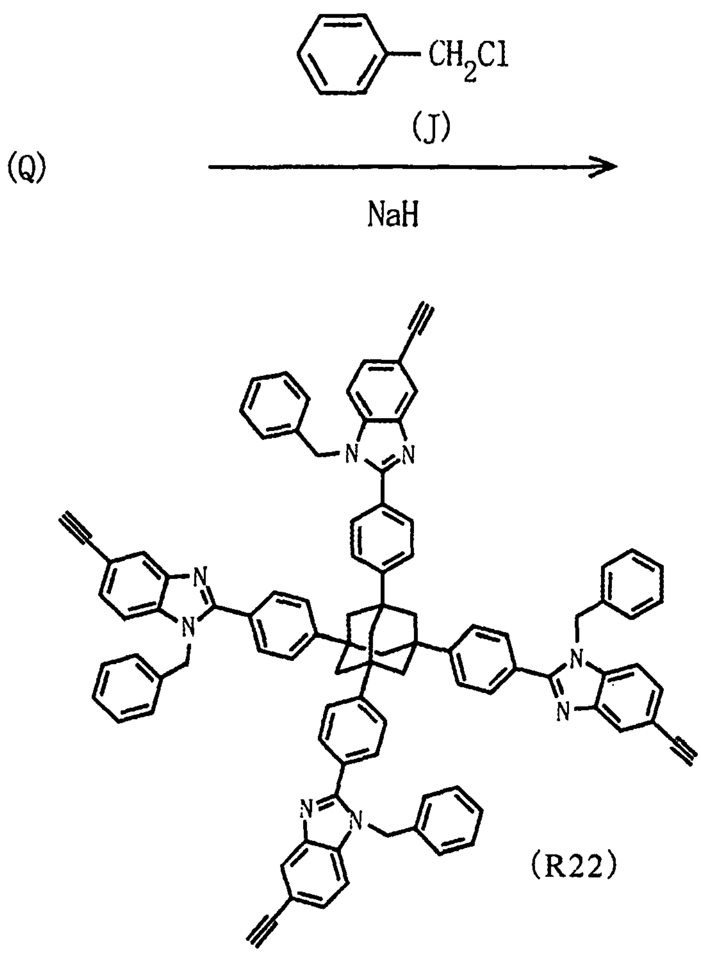


(R22)

In a 100-mL four-necked flask was placed 0.4 g (16 mmol) of sodium hydride, and this was combined with 10 g of N,N-dimethylacetamide (DMAc) added at 25°C in a nitrogen atmosphere. The mixture was further combined with 2.0 g (16 mmol) of benzyl chloride of Formula (J). Additionally, a solution of 2 g (2 mmol) of the ethynyl-containing adamantane derivative of Formula (Q) in 10 g of DMAc was prepared and added dropwise through a dropping funnel to the mixture in the four-necked flask at 25°C. After the completion of dropwise addition, the mixture was stirred at 80°C for 6 hours. After the completion of reaction, the reaction mixture was cooled to 25°C and combined with 10 g of methanol. Next, 50 g of pure water was added to precipitate solids. The solids were collected by filtration and dried in a vacuum drier to give a product. The $^1$H-NMR spectrum of the product was measured (see FIG. 4), to find that an N-substituted ethynylbenzimidazole-containing bridged alicyclic compound of Formula (R22) was formed. The N-substituted ethynylbenzimidazole-containing bridged alicyclic compound was obtained in an amount of 1.5 g and a yield of 55%.

[NMR Spectral Data]

$^1$H-NMR ($CDCl_3$) δ (ppm) : 2.2 (12H), 3.00 (4H), 5.45 (8H), 7.0-8.1 (60H)

Example A6

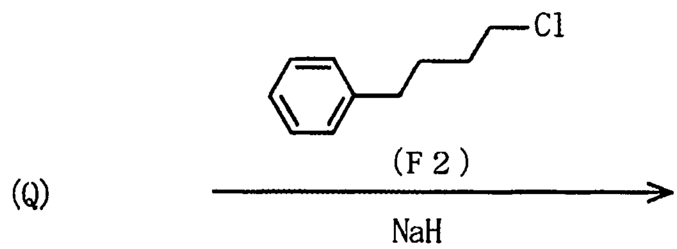


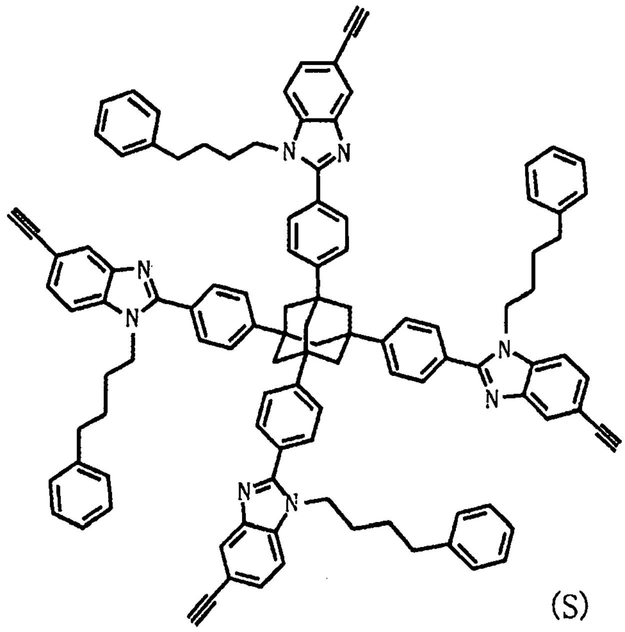

(S)

In a 100-mL four-necked flask was placed 0.4 g (16 mmol) of sodium hydride, and 10 g of N,N-dimethylacetamide (DMAc) was added thereto at 25°C in a nitrogen atmosphere. To the mixture was added 2.7 g (16 mmol) of 4-phenylbutyl chloride of Formula (F2). Additionally, a solution of 2 g (2 mmol) of the ethynyl-containing adamantane derivative of Formula (Q) in 10 g of DMAc was prepared and added dropwise through a dropping funnel to the mixture in the four-necked flask at 25°C. After the completion of dropwise addition, the mixture was stirred at 80°C for 6 hours. After the completion of reaction, the reaction mixture was cooled to 25°C and combined with 10 g of methanol. Next, 50 g of pure water was added, and an oil was separated by decantation. The oil was dissolved again in 10 g of DMAc, and combined with 10 g of methanol and 10 g of pure water, to precipitate solids. The solids were collected by filtration and dried in a vacuum drier to give a product. The $^1$H-NMR spectrum of the product was measured (see FIG. 5), to find that an N-substituted ethynylbenzimidazole-containing bridged alicyclic compound of Formula (S) was formed. The N-substituted ethynylbenzimidazole-containing bridged alicyclic compound was obtained in an amount of 0.75 g and a yield of 25%.

[NMR Spectral Data]

$^1$H-NMR ($CDCl_3$) δ (ppm): 1.17 (8H), 1.60 (8H), 1.88 (8H), 2.1 (12H), 2.94-3.1 (4H), 4.25 (8H), 7.2-8.2 (60H)

Example A7

(Q)

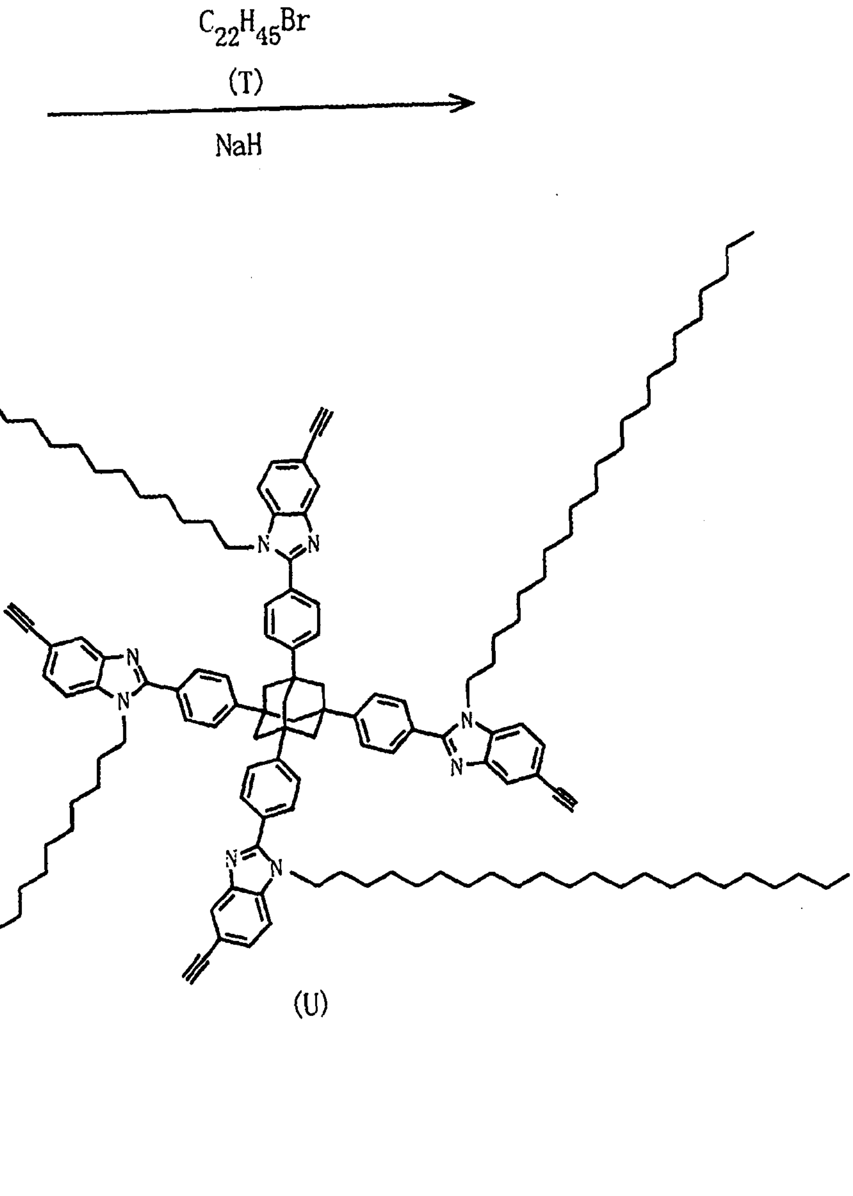


(U)

In a 100-mL four-necked flask was placed 0.25 g (10 mmol) of sodium hydride, and 20 g of N,N-dimethylacetamide (DMAc) was added thereto at 25°C in a nitrogen atmosphere. To this was added 3.2 g (10 mmol) of 1-bromodocosane of Formula (T). Additionally, a solution of 2 g (2 mmol) of the ethynyl-containing adamantane derivative of Formula (Q) in 10 g of DMAc was prepared and added dropwise through a dropping funnel to the mixture in the four-necked flask at 40°C. After the completion of dropwise addition, the mixture was stirred at 80°C for 8 hours. After the completion of reaction, the reaction mixture was cooled to 25°C and combined with 80 g of methanol. Next, 160 g of hexane was added followed by stirring, and the mixture was separated, from which a hexane layer was obtained. The hexane layer was combined with 100 g of pure water, stirred, and separated, from which a hexane layer was obtained again. The hexane layer was concentrated and purified by silica gel column chromatography with hexane as a developing solvent. The resulting hexane solution (eluate) was left stand for 24 hours to precipitate solids. The solids were separated from a liquid by decantation, and dried in a vacuum drier to give a product. The $^1H$-NMR spectrum of the product was measured (see FIG. 6) to find that an N-substituted ethynylbenzimidazole-containing bridged alicyclic compound of Formula (U) was formed. The N-substituted ethynylbenzimidazole-containing bridged alicyclic compound was obtained in an amount of 1.0 g and a yield of 22%.

[NMR Spectral Data]

$^1$H-NMR ($CDCl_3$) δ (ppm): 0.8 (12H), 1.2 (152H), 1.8 (8H), 2.3 (12H), 3.0-3.1 (4H), 4.2 (8H), 7.3-8.0 (28H)

Evaluation Test A1

A 10 percent by weight solution was prepared by dissolving the adamantane derivative (terminally capped compound) of Formula (E2) prepared in Preparation Example A2 in a 1:1 (by weight) mixture of DMAc and 1,3-dimethyl-2-imidazolidinone (DMI). This solution was applied to a wafer using a spin coater. The coated wafer was heated in an electric furnace at 250°C in a nitrogen atmosphere for 1 hour to completely evaporate the solvent to thereby give a thin film having a thickness of 250 nm. The prepared thin film was examined on its electrical properties and found to have a relative dielectric constant of 3.4. In addition, the thin film was left stand in air and showed an increased current due to moisture absorption.

In contrast, a thin film having a thickness of 200 nm was prepared in the same manner from the N-substituted benzimidazole-containing bridged alicyclic compound of Formula (G2) prepared in Example A1. This thin film was examined on its electrical properties and found that it did not show increase in current due to moisture absorption and had a relative dielectric constant of 3.0.

Evaluation Test A2

A 10 percent by weight solution was prepared by dissolving the ethynyl-containing adamantane derivative of Formula (I2) prepared in Preparation Example A3 in a 1:1 (by weight) mixture of DMAc and 1,3-dimethyl-2-imidazolidinone (DMI). This solution was applied to a wafer using a spin coater. The coated wafer was heated in an electric furnace at 400°C in a nitrogen atmosphere for 30 minutes, to proceed a crosslinking reaction to thereby yield a thin film having a thickness of 300 nm. The thin film was examined on its electrical properties and found to have a relative dielectric constant of 3.0. In addition, the thin film was left stand in air and showed an increased current due to moisture absorption.

In contrast, a thin film having a thickness of 220 nm was prepared in the same manner from the N-substituted benzimidazole-containing bridged alicyclic compound of Formula (K) prepared in Example A2. This thin film was examined on its electrical properties and found that it did not show increase in current due to moisture absorption and had a relative dielectric constant of 3.0.

Additionally, another thin film having a thickness of 210 nm was prepared in the same manner from the N-substituted benzimidazole-containing bridged alicyclic compound of Formula (L) prepared in Example A3, and its electrical properties were examined. This thin film did not show increase in current due to moisture absorption and had a relative dielectric constant of 3.0.

Evaluation Test A3

A 10 percent by weight solution was prepared by dissolving the terminally capped polymer of Formula (N) prepared in Preparation Example A5 in a 1:1 (by weight) mixture of DMAc and 1,3-dimethyl-2-imidazolidinone (DMI). This solution was applied to a wafer using a spin coater. The coated wafer was heated in an electric furnace at 250°C in a nitrogen atmosphere for 1 hour to evaporate the solvent completely, to thereby yield a thin film having a thickness of 260 nm. The prepared thin film was examined on its electrical properties and found to have a relative dielectric constant of 3.5. In addition, the thin film was left stand in air and showed an increased current due to moisture absorption.

In contrast, a thin film having a thickness of 280 nm was prepared in the same manner from the N-substituted benzimidazole-containing bridged alicyclic compound of Formula (O) prepared in Example A4. This thin film was examined on its electrical properties and found that it did not show increase in current due to moisture absorption and had a relative dielectric constant of 3.0.

Evaluation Test A4

A 10 percent by weight solution was prepared by dissolving the ethynyl-containing adamantane derivative of Formula (Q) in Preparation Example A6 in a 1:1 (by weight) mixture of DMAc and 1,3-dimethyl-2-imidazolidinone (DMI). This solution was applied to a wafer using a spin coater. The coated wafer was heated in an electric furnace at 350°C in a nitrogen atmosphere for 1 hour to proceed a crosslinking reaction, to thereby yield a thin film having a thickness of 300 nm. The prepared thin film was examined on its electrical properties and found to have a relative dielectric constant of 3.1. Even when left stand in air, the thin film did not show increase in current due to moisture absorption.

Another 10 percent by weight solution was prepared by dissolving the N-substituted benzimidazole-containing bridged alicyclic compound of Formula (R22) prepared in Example A5 in cyclohexanone. This solution was applied to

a wafer using a spin coater. The coated wafer was heated in an electric furnace at 350°C in a nitrogen atmosphere for 1 hour to proceed a crosslinking reaction, to thereby yield a thin film having a thickness of 460 nm. The prepared thin film was examined on its electrical properties and found to have a relative dielectric constant of 3.0. Even when left stand in air, the thin film did not show increase in current due to moisture absorption.

Another 10 percent by weight solution was prepared by dissolving the N-substituted benzimidazole-containing bridged alicyclic compound of Formula (S) prepared in Example A6 in cyclohexanone. This solution was applied to a wafer using a spin coater. The coated wafer was heated in an electric furnace at 350°C in a nitrogen atmosphere for 1 hour to proceed a crosslinking reaction, to thereby yield a thin film having a thickness of 400 nm. The prepared thin film was examined on its electrical properties and found to have a relative dielectric constant of 3.0. Even when left stand in air, the thin film did not show increase in current due to moisture absorption.

A 7 percent by weight solution was prepared by dissolving the N-substituted benzimidazole-containing bridged alicyclic compound of Formula (U) prepared in Example A7 in cyclohexanone. This solution was applied to a wafer using a spin coater. The coated wafer was heated in an electric furnace at 300°C in a nitrogen atmosphere for 1 hour to proceed a crosslinking reaction, to thereby yield a thin film having a thickness of 310 nm. The prepared thin film was examined on its electrical properties and found to have a relative dielectric constant of 2.6. Even when left stand in air, the thin film did not show increase in current due to moisture absorption.

FIG. 7 shows leak current characteristics of the thin film formed from the ethynyl-containing adamantane derivative prepared in Preparation Example A6, and of the thin films formed from the N-substituted ethynylbenzimidazole-containing bridged alicyclic compounds prepared in Examples A5 to A7. FIG. 7 is shown with the abscissa indicating the applied field intensity (V/cm) and the ordinate indicating the leak current density ($A/cm^2$). The leak current characteristics were measured according to a probe method.

Example B1

Synthesis of Ethynyl-Containing Adamantane Derivative

(2-1)

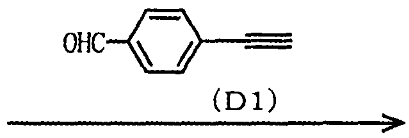

(D1)

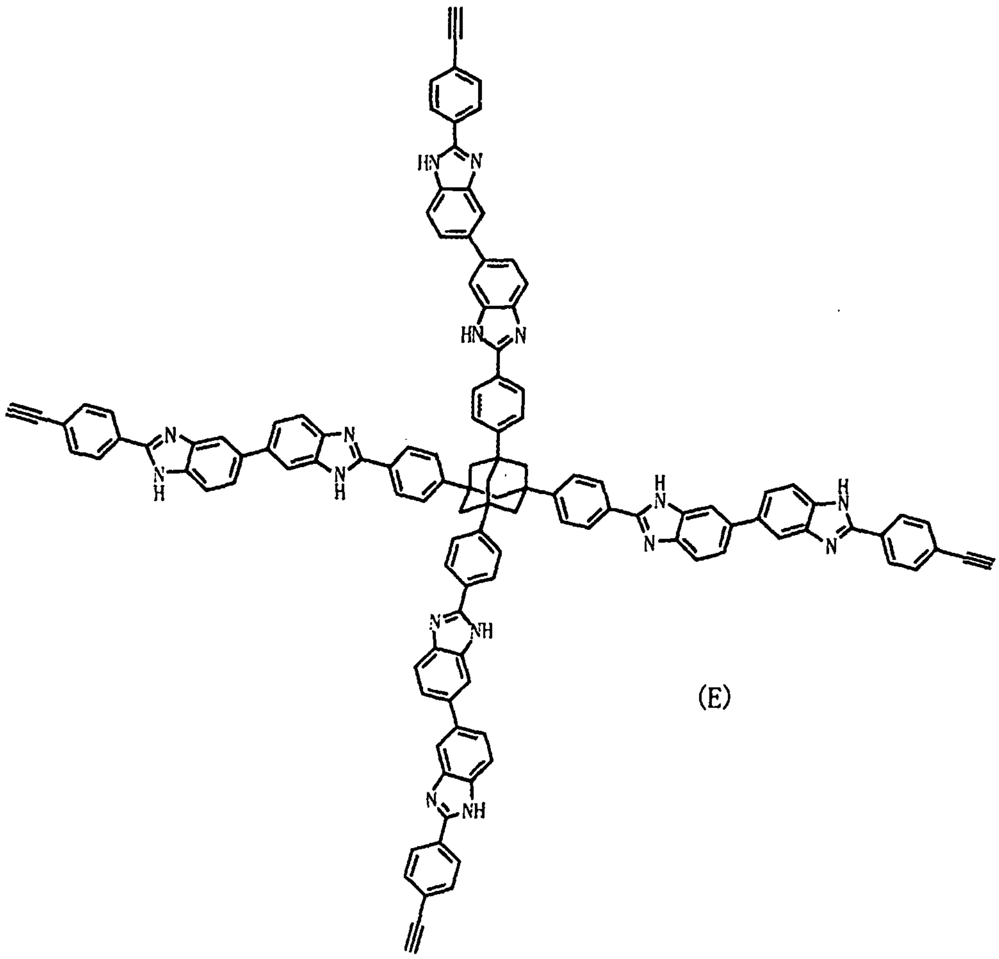

(E)

In a reactor (three-necked flask) was placed a solution of 2.08 g of 4-ethynylbenzaldehyde of Formula (D1) in 20 g of N,N-dimethylacetamide (DMAc). Another solution of 2.65 g of the amino-containing adamantane derivative of Formula (2-1) in 25 g of DMAc was added dropwise thereto at room temperature through a dropping funnel. After the completion of dropwise addition, the dropping funnel was washed with 10 g of DMAc, and this washing was also added dropwise to the mixture in the reactor. While introducing a gaseous mixture of oxygen and nitrogen with an oxygen concentration of 5 percent by mole into the reaction mixture through a Teflon (registered trademark) tube, a reaction was conducted for 7 hours by heating the reactor on an oil bath to keep the liquid temperature at 80°C. After the completion of reaction, the reaction mixture was added dropwise to 800 g of water in another reactor, to give a slurry composed of precipitates and a supernatant. The slurry was stirred for about 1 hour after the completion of dropwise addition to give precipitates. The precipitates were collected by filtration, transferred again to the reactor, and combined with 400 g of methanol, followed by stirring for 1 hour. The precipitate were collected by filtration and dried in a vacuum drier. After the completion of drying, the precipitates were dissolved in 50 g of DMAc, and the solution was added dropwise to 400 g of methanol to give precipitates. The precipitate were collected by filtration and dried in a vacuum drier to give a product. The NMR spectrum and infrared absorption spectrum of the product were measured to find that an ethynyl-containing adamantane derivative of Formula (E) was prepared. The ethynyl-containing adamantane derivative was obtained in an amount of 3.09 g and a yield of 87%.

[NMR Spectral Data]

$^{1}$H-NMR (DMSO-d6) δ (ppm): 2.32 (12H <adamantane -$CH_2$->), 4.38 (4H <ethynyl C-H>), 7.54-8.26 (6H <aromatic ring C-H>), 13.05 (4H <imidazole N-H)

[Infrared Absorption Spectral Data (cm$^{-1}$)]

3422 (N-H <stretching vibration>), 2930 (C-H of -$CH_2$-<stretching vibration>), 2220 (ethynyl group <stretching vibration>, 1620 (-C=N- <stretching vibration>), 1420-1520 (aromatic ring <in-plane vibration>), 1280 (aromatic -N-H <stretching vibration>), 809 (C-H <out-of-plane deformation vibration>

Preparation of Coating Composition

A stirrer was placed in a 30-mL flask equipped with a three-way stopcock; while introducing nitrogen gas into the flask, 800 mg of the above-prepared ethynyl-containing adamantane derivative and a 1:1 (by weight) mixture of DMAc and 1,3-dimethyl-2-imidazolidinone (DMI) were placed, followed by stirring at 30°C for 1 hour, to give a 10 percent by weight solution of the ethynyl-containing adamantane derivative. The prepared solution was brought to room temperature and filtrated sequentially through 0.2-μm and 0.1-μm Teflon (registered trademark) filters, to give a coating composition (material for producing insulating film).

Formation of Insulating film

An aliquot (2 to 3 mL) of the above-prepared coating composition was dropped onto a silicon wafer, and a coat was formed by spin coating at a controlled revolution number of 1000 to 3000 rpm. Next, the coat was baked in a quartz chamber by elevating the temperature from room temperature to 400°C in a nitrogen atmosphere, to give a film. The film had a thickness of 267 nm and a relative dielectric constant of 3.0.

[Infrared Absorption Spectral Data (cm$^{-1}$)]

3422 (N-H <stretching vibration>), 2930 (C-H of -$CH_2$-<stretching vibration>), 1620 (-C=N- <stretching vibration>), 1420-1520 (aromatic ring <in-plane vibration>), 1280 (aromatic -N-H <stretching vibration>), 806 (C-H <out-of-plane deformation vibration>

Example B2

Synthesis of Ethynyl-Containing Adamantane Derivative

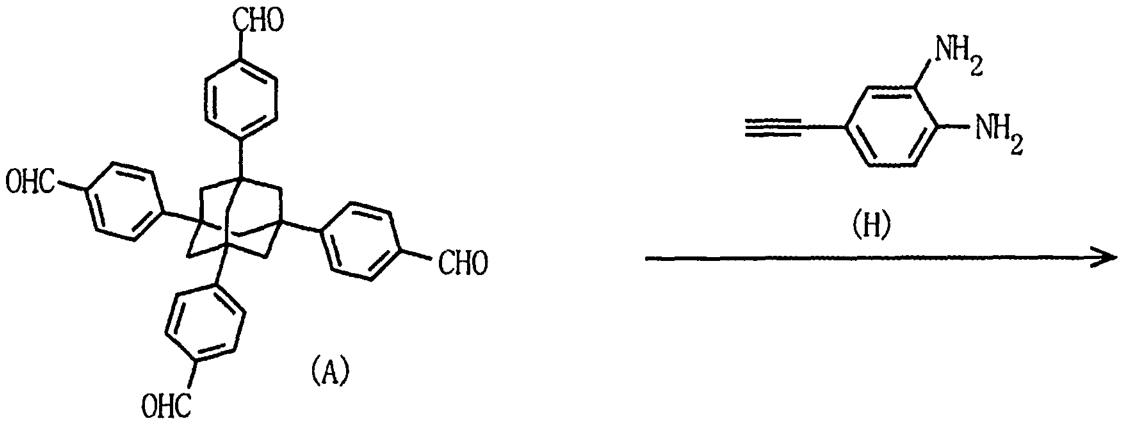

(A)

(H)

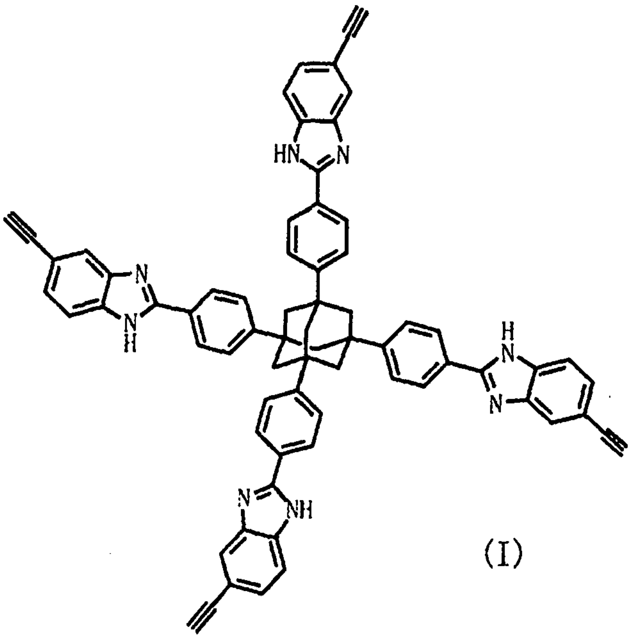

(I)

In a 1000-mL four-necked flask was placed 9.6 g (72.0 mmol) of 4-ethynyl-1,2-diaminobenzene of Formula (H), and 50 g of N,N-dimethylacetamide (DMAc) was added thereto, to give a solution, and the solution was held at 25°C while blowing air into the solution through a Teflon (registered trademark) tube. To this mixture (solution) was added dropwise a solution of 5.0 g (9 mmol) of adamantane tetrakisbenzaldehyde of Formula (A) in 100 g of DMAc through a dropping funnel over 1.5 hours. After the completion of dropwise addition, the mixture was stirred at 25°C for 1 hour, and then raised in temperature to 80°C, followed by carrying out a reaction with stirring for 24 hours. After the completion of reaction, the reaction mixture was cooled to 25°C, and 600 g of pure water was added dropwise to give a slurry composed of precipitates and a supernatant. The slurry was stirred for about 1 hour after the completion of dropwise addition to give precipitates. The precipitates were collected by filtration, transferred again to the reactor, and combined with 600 g of methanol, followed by stirring for 1 hour. The precipitate were collected by filtration and dried in a vacuum drier. After the completion of drying, the precipitates were dissolved in 150 g of DMAc, and 600 g of methanol was added dropwise thereto, to give precipitates. The precipitate were collected by filtration and dried in a vacuum drier to give a product. The $^{1}$H-NMR spectrum of the product was measured (see FIG. 8), to find that an ethynyl-containing adamantane derivative of Formula (I) was formed. The ethynyl-containing adamantane derivative was obtained in an amount of 5.4 g and a yield of 60%.

[NMR Spectral Data]

$^{1}$H-NMR (DMSO-d6) δ (ppm): 2.1 (12H), 4.0-4.1 (4H), 7.2-8.2 (28H), 13.1 (4H)

Preparation of Coating Composition

The above-prepared ethynyl-containing adamantane derivative of Formula (I) was dissolved in a 1:1 (by weight) mixture of DMAc and 1,3-dimethyl-2-imidazolidinone (DMI), to give a 10 percent by weight solution of the ethynyl-containing adamantane derivative.

Formation of Insulating film

The above-prepared coating composition (material for producing insulating film) was applied to a silicon wafer using a spin coater. The coated wafer was heated in an electric furnace at 350°C in a nitrogen atmosphere for 1 hour to proceed a crosslinking reaction, to thereby yield a thin film having a thickness of 300 nm. The prepared thin film was examined on its electrical properties and found to have a relative dielectric constant of 3.1. Even when left stand in air, the thin film did not show increase in current due to moisture absorption.

Comparative Example B1

Preparation of Coating Composition

A stirrer was placed in a 30-mL flask equipped with a three-way stopcock. While introducing nitrogen gas into the flask, 255 mg of a carboxylic acid compound of following Formula (F), 550 mg of an amine compound of following Formula (G), and a 1:1 (by weight) mixture of DMAc and DMI were placed in the flask, and the mixture was stirred at 30°C for 1 hour, to give a solution having a total concentration of the two compounds of 10 percent by weight. The prepared solution was brought to room temperature and filtrated sequentially through 0.2-μm and 0.1-μm Teflon (registered trademark) filters, to give a coating composition (material for producing insulating film).

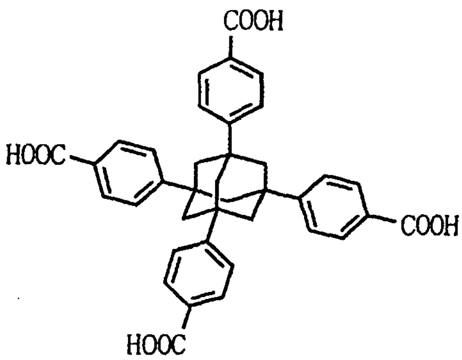

(F)

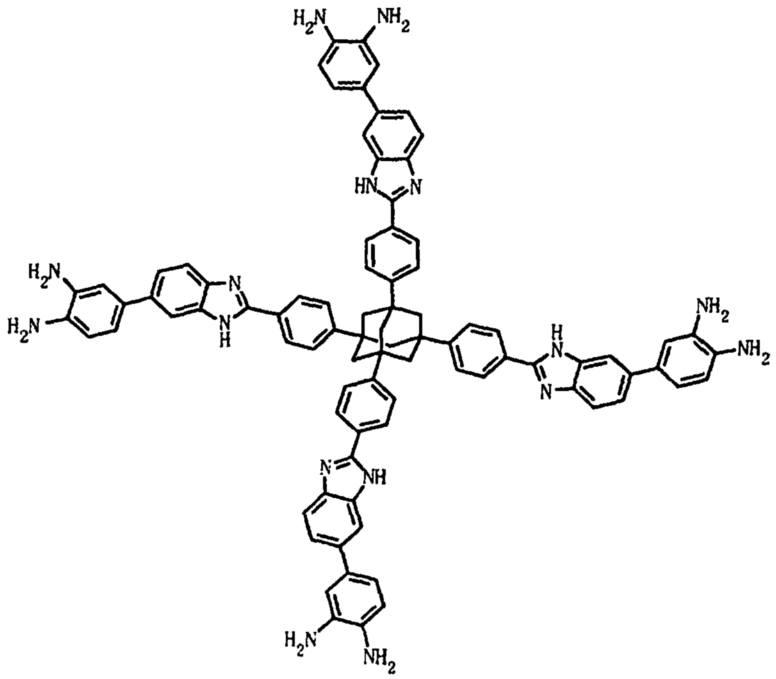

(G)

Formation of Insulating film

2 to 3 mL of the above-prepared coating composition was dropped onto a silicon wafer, and a coat was formed by spin coating at a controlled revolution number of 1000 to 3000 rpm. Next, the coat was baked in a quartz chamber by elevating the temperature from room temperature to 400°C in a nitrogen atmosphere, to give a film. The film had a thickness of 260 nm and a relative dielectric constant of 3.5.

[Infrared Absorption Spectral Data ($cm^{-1}$)]

3419 (N-H <stretching vibration>), 2933 (C-H of -$CH_2$-<stretching vibration>), 1626 (-C=N- <stretching vibration>), 1420-1520 (aromatic ring <in-plane vibration>), 1282 (aromatic -N-H <stretching vibration>), 806 (C-H <out-of-plane deformation vibration>

Evaluation Test B

Relative dielectric constants of the insulating films prepared in Examples B1 and B2 and Comparative Example B1 were measured at frequencies of 1 kHz to 1 MHz according to a probe method. The insulating film prepared in Comparative Example B1 had a relative dielectric constant of 3.5, whereas the insulating films (using ethynyl-containing materials) prepared in Examples B1 and B2 had relative dielectric constants of 3.0 and 3.1, respectively. In Examples B1 and B2, the materials did not contain highly polar unreacted terminals and thereby gave films having low relative dielectric constants. These results demonstrate that ethynyl group having low polarity acts to reduce dielectric constants of the resulting films.

Preparation Example 2

Synthesis of Ethoxycarbonylbutyl-Containing Adamantane Derivative of Formula (1a-1)

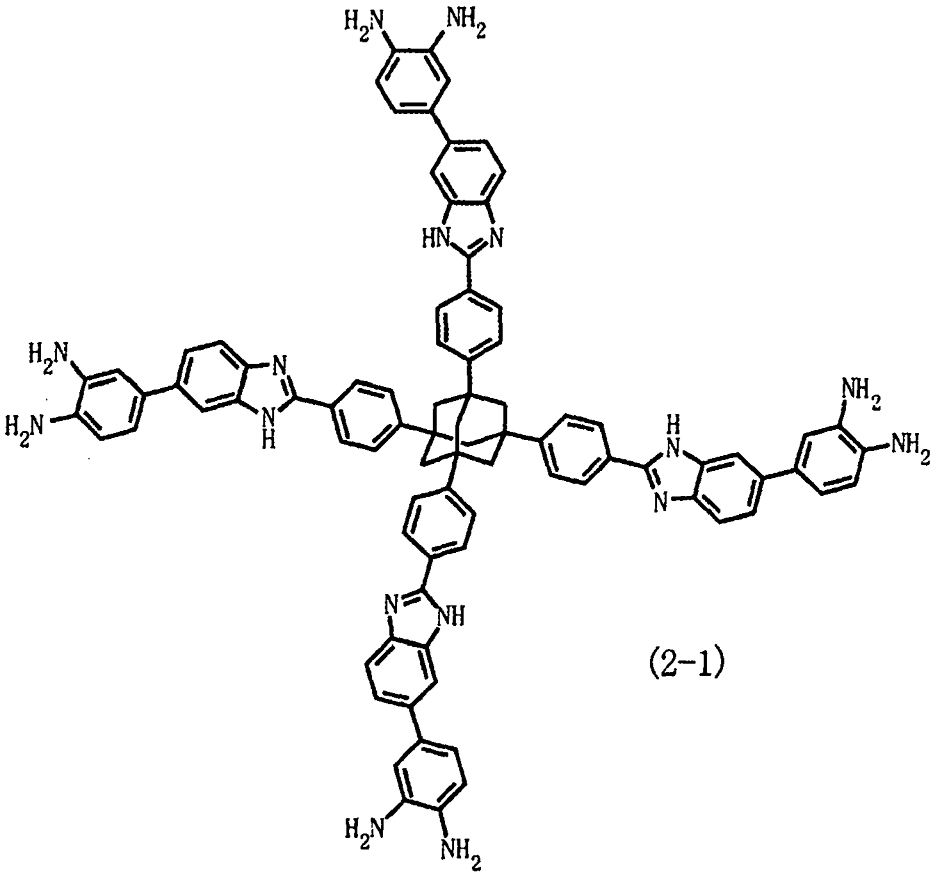

(2-1)

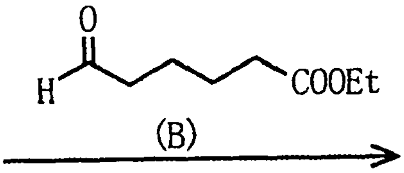

(B)

(1a-1)

In a reactor (three-necked flask) were placed 1.51 g (1.13 mmol) of an amino-containing adamantane derivative of Formula (2-1) and 1.07 g (6.77 mmol) of an aldehyde compound of Formula (B), and the mixture was further mixed with 13.5 g of N,N-dimethylacetamide (DMAc) to give a solution. While introducing air to the solution (reaction mixture) through a Teflon (registered trademark) tube, the solution was reacted for 6 hours by heating the reactor on an oil bath to keep the liquid temperature at 90°C. The reaction mixture was added dropwise to 150 ml of water in another reactor, to give a slurry composed of precipitates and a supernatant. The slurry was stirred for about 1 hour after the completion of dropwise addition, to give precipitates. The precipitates were collected by filtration, transferred again to the reactor, washed with ethyl acetate, the resulting precipitates were collected by filtration, and dried in a vacuum drier to give a product.

The NMR spectrum of the product was measured to find that a compound of Formula (1a-1) was formed.

[NMR Spectral Data]

$^1$H-NMR (DMSO-d6) δ (ppm): 1.17 (12H <-$CH_3$>), 1.63 (8H <-$CH_2$->), 1.83 (8H <-$CH_2$->), 2.35-2.37 (20H <-$CH_2$->), 2.85 (8H <-$CH_2$->), 4.06 (8H <-$NH_2$>), 7.48-8.27 (40H <aromatic protons>), 12.26-12.98 (8H <-NH->)

Preparation Example 3

Synthesis of Ethoxycarbonylbutyl-and-Amino-Containing Adamantane Derivative of Formula (3-1)

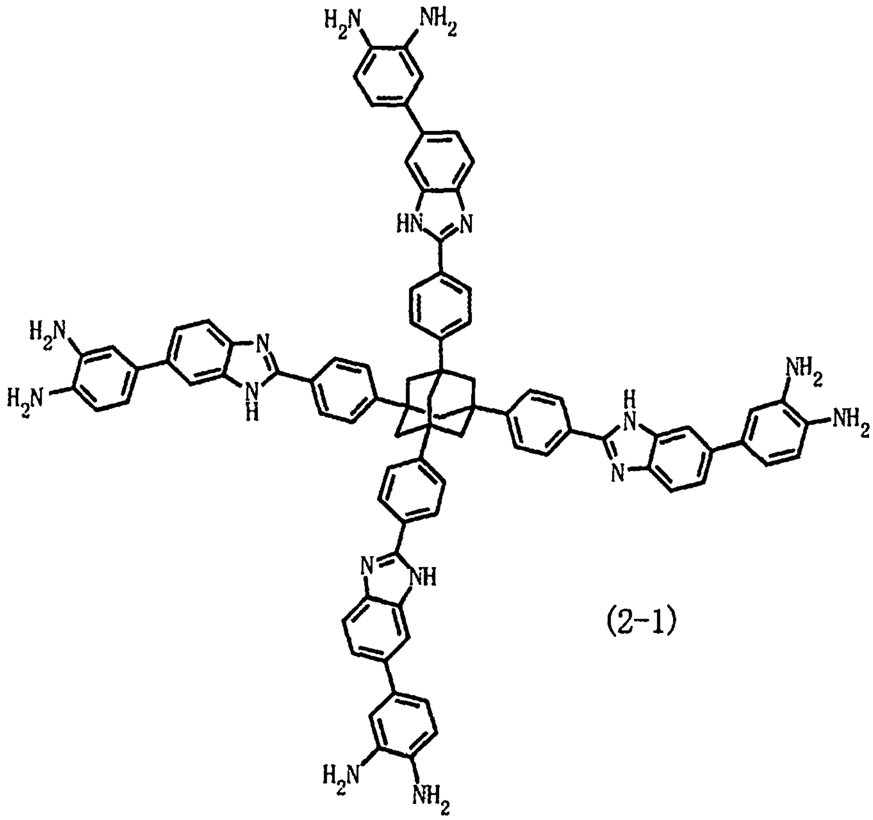

(2-1)

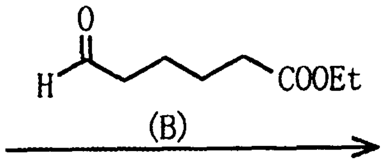


(3-1)

In a reactor (one-necked flask) was placed 1.42 g (1.053 mmol) of a compound of Formula (2-1), and this was mixed with 12.7 g of N,N-dimethylacetamide (DMAc) to give a solution, and the solution was held at 0°C or lower on an ice bath. To this solution in the reactor was added dropwise a solution of 0.37 g (2.11 mmol) of ethyl 5-formylpentanecarboxylate of Formula (B) in 0.799 g of DMAc using a syringe. The dropwise addition was conducted so that the temperature of the reaction mixture did not exceed 0°C. After the completion of dropwise addition, the mixture was reacted at room temperature for 4.5 hours, and was further reacted for 2.5 hours while heating the reactor on an oil bath to keep the liquid temperature at 90°C, while introducing air into the reaction mixture through a Teflon (registered trademark) tube. After the completion of reaction, the reaction mixture was nitrogen replacement was conducted five times, and shaded with aluminum foil, to give a solution of an ethoxycarbonylbutyl-and-amino-containing adamantane derivative of Formula (3-1).

Preparation Example 4

Synthesis of Amino-Containing Compound of Formula (1b-3)

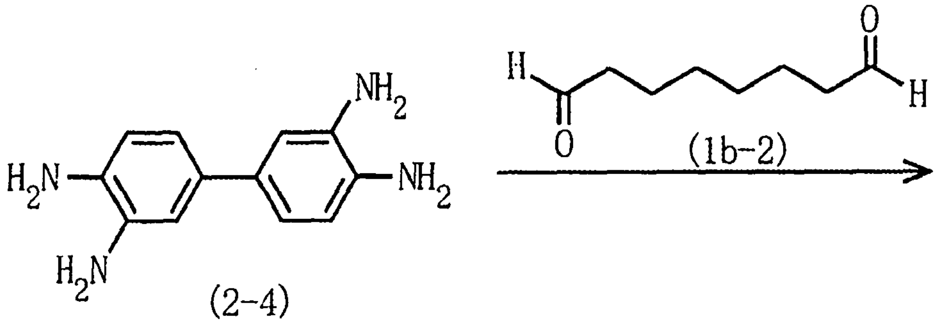

(2-4) (1b-2) (1b-3)

In a reactor (three-necked flask) was placed 35.8 g (167 mmol) of 3,3'-diaminobenzidine of Formula (2-4), and this was mixed with 143.0 g of N,N-dimethylacetamide (DMAc) to give a solution, and the solution was held at 0°C or lower on an ice bath. To this solution in the reactor was added dropwise a solution of 1.0 g (6.68 mmol) of octanedialdehyde in 50.4 g of DMAc through a dropping funnel at a rate of 2.5 ml/min. The dropwise addition was conducted so that the temperature of the reaction mixture did not exceed 0°C. After the completion of dropwise addition, the dropping funnel was washed with 10 ml of DMAc, and this washing was also added dropwise to the mixture in the reactor. After the completion of dropwise addition, the mixture was reacted for 4 hours by heating the reactor on an oil bath to keep the liquid temperature at 60°C, while introducing air into the reaction mixture through a Teflon (registered trademark) tube. After the completion of reaction, the reaction mixture was added dropwise to 2000 ml of water in another reactor, to give a slurry composed of precipitates and a supernatant. The slurry was stirred for about 1 hour after the completion of dropwise addition. During the stirring, the reaction mixture was held in a nitrogen atmosphere so as to avoid amine oxidation. The formed precipitates were collected by filtration, transferred again to the reactor, and combined with 700 ml of water, followed by washing with hot water by heating under reflux in a nitrogen atmosphere for 30 minutes. The precipitates were collected by filtration before the temperature dropped. This procedure was repeated seven times. Next, the resulting precipitates were collected by filtration, transferred again to the reactor, combined with methanol to give a solution, and the solution was added dropwise to water in another reactor, to give a slurry composed of precipitates and a supernatant. The slurry was stirred in a nitrogen atmosphere for about 1 hour. The precipitate were collected by filtration and dried in a vacuum drier to give a product.

The NMR spectrum of the product was measured to find that a compound of Formula (1b-3) was formed. The compound of Formula (1b-3) was obtained in an amount of 2.0 g and a yield of 54%.

[NMR Spectral Data]

$^1$H-NMR (DMSO-d6) δ (ppm): 1.22 (4H <-$CH_2$->), 1.39 (4H <-$CH_2$->), 1.77 (4H <-$CH_2$->), 2.49 (8H <-$CH_2$->), 2.79 (2H <-$CH_2$->), 4.50 (4H <-$NH_2$>), 6.56 (2H <aromatic protons>), 6.69 (2H <aromatic protons>), 6.84 (2H <aromatic protons>), 7.24 (2H <aromatic protons>), 7.35-7.54 (4H <aromatic protons>), 12.09 (4H <-NH->)

Preparation Example 5

Synthesis of Amino-Containing Compound of Formula (1b-4)

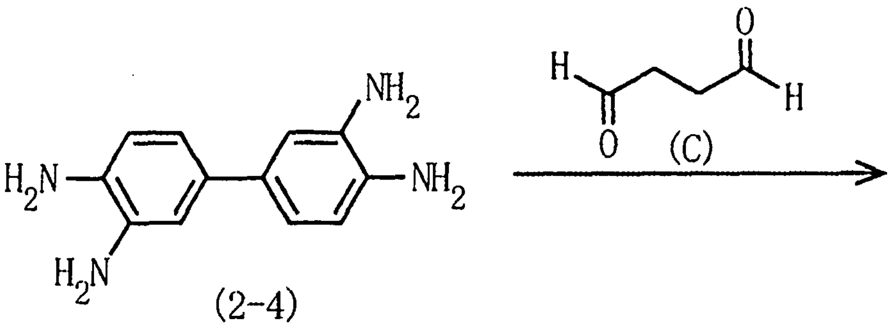

(2-4) (C) (1b-4)

In a reactor (three-necked flask) was placed 62.3 g (290 mmol) of 3,3'-diaminobenzidine of Formula (2-4), and this was combined with 100 ml of N,N-dimethylacetamide (DMAc) to give a solution, and the solution was held at 0°C or lower on an ice bath. To the solution in the reactor was added dropwise a solution of 1.26 g (14.5 mmol) of succinaldehyde in 100 ml of DMAc at a rate of 2 ml/min through a dropping funnel. The dropwise addition was conducted so that the temperature of the reaction mixture did not exceed 0°C. After the completion of dropwise addition, the dropping funnel was washed with 10 ml of DMAc, and this washing was also added dropwise to the mixture in the reactor. After the completion of dropwise addition, the mixture was reacted for 4 hours by heating the reactor on an oil bath to keep the liquid temperature at 60°C, while introducing air into the reaction mixture through a Teflon (registered trademark) tube. After the completion of reaction, 100 ml of DMAc was distilled off from the reaction mixture using an evaporator. The resulting mixture was added dropwise to 1000 ml of water in another reactor, to give a slurry composed of precipitates and a supernatant. The slurry was stirred for about 1 hour after the completion of dropwise addition. During the stirring, the reaction mixture was held in a nitrogen atmosphere so as to avoid amine oxidation. The formed precipitates were collected by filtration, transferred again to the reactor, and combined with 700 ml of water, followed by washing with hot water by heating under reflux in a nitrogen atmosphere for 30 minutes. The precipitates were collected by filtration before the temperature dropped. This procedure was repeated seven times. Next, the resulting precipitates were collected by filtration, transferred again to the reactor, combined with methanol to give a solution, and the solution was added dropwise to water in another reactor, to give a slurry composed of precipitates and a supernatant. The slurry was stirred in a nitrogen atmosphere for about 1 hour. The resulting precipitates were collected by filtration and dried in a vacuum drier to give a product.

The NMR spectrum of the product was measured to find that a compound of Formula (1b-4) was formed. The compound of Formula (1b-4) was obtained in an amount of 801.2 mg and a yield of 46%.

[NMR Spectral Data]

$^1$H-NMR (DMSO-d6) δ (ppm): 2.80 (4H <-$CH_2$->), 4.51 (8H <-$NH_2$>), 6.57 (2H <aromatic protons>), 6.70 (2H <aromatic protons>), 6.85 (2H <aromatic protons>), 7.24 (2H <aromatic protons>), 7.35-7.55 (4H <aromatic protons>), 12.08 (2H <-NH->)

Preparation Example 6

Synthesis of Amino-and-Hydroxyl-Containing Compound of Formula (1b-5)

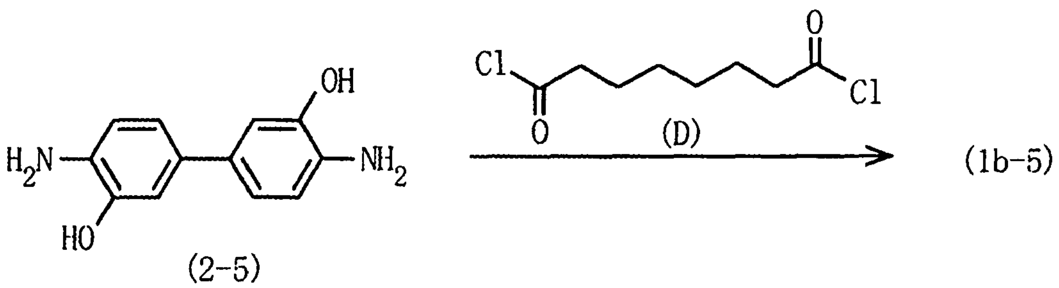

(2-5) (D) (1b-5)

In a reactor (Erlenmeyer flask) was placed 1.5 g (7.11 mmol) of hexane-1,6-dicarbonyl chloride, and this was combined with 307 g of N,N-dimethylacetamide (DMAc) to give a solution, and the solution was cooled to -20°C on a dry ice/methanol bath. Likewise, 30.7 g (142 mmol) of 3,3'-dihydroxybenzidine of Formula (2-4) was placed in another reactor (Erlenmeyer flask), and this was combined with 307 g of N,N-dimethylacetamide (DMAc) to give a solution, and the solution was cooled to -20°C on a dry ice/methanol bath. The two solutions were charged at an equivalent rate through syringes into yet another reactor held at -18°C or lower using a cooling unit, followed by stirring for 1.5 hours. After the completion of reaction, the solvent was distilled off using an evaporator, to a solids concentration of 15 percent by weight. The residual mixture was added dropwise to 1000 ml of methanol in another reactor, to give a slurry composed of precipitates and a supernatant. The slurry was stirred for about 1 hour after the completion of dropwise addition, to give precipitates. The precipitates were collected by filtration, transferred again to a flask, and dissolved in DMAc to a solids concentration of 15 percent by weight. The solution was added dropwise to 1000 ml of methanol in another reactor, to give a slurry composed of precipitates and a supernatant. The slurry was stirred for about 1 hour after the completion

of dropwise addition to give precipitates, and the precipitates were collected by filtration. This procedure was repeated seven times. The resulting solids were dried in a vacuum drier to give a product.

The NMR spectrum of the product was measured to find that a compound of Formula (1b-5) was formed.

[NMR Spectral Data]

$^1$H-NMR (DMSO-d6) δ (ppm) : 1.35 (4H <-$CH_2$->), 2.40 (4H <-$CH_2$->), 2.50 (4H <-$CH_2$->), 4.63 (4H <-$NH_2$>), 6.57-7.05 (10H <aromatic protons>), 7.64 (2H <aromatic protons>), 9.10-9.78 (6H)

*Comparative Example 1 [Preparation of Film from Compounds (2-1) and* (2-2) *(Film 1)]*

In a thoroughly dried reactor (eggplant flask) were placed 464 mg (0.752 mmol) of the carboxylic acid compound of Formula (2-2) and 1330 mg (0.752 mmol) of the amine compound of Formula (2-1), and these were dissolved in 6.73 g of N,N-dimethylacetamide (DMAc) and 6.73 g of 1,3-dimethyl-2-imidazolidinone (DMI) added thereto, to give a solution. While stirring the solution, evacuation of the reactor using a vacuum pump and nitrogen replacement was conducted, and this procedure was repeated a total of three times. The reactor was shaded, and the solution was stirred for 1 hour and then left stand overnight without stirring. The resulting solution was filtrated sequentially through 0.2-μm and 0.1-μm PTFE filter papers, and the filtrate was applied to a silicon substrate by spin coating at 1000 rpm for 20 seconds and then at 3000 rpm for 20 seconds. Immediately thereafter, the coat was baked by heating from 50°C to 250°C, holding at 250°C for 30 minutes, heating from 250°C to 400°C, and holding at 400°C for 30 minutes, to give a Film 1. Film 1 had a thickness of 170.0 nm.

[Infrared Absorption Spectral Data ($cm^{-1}$)]

3419 (N-H <stretching vibration>), 2933 (-$CH_2$-<stretching vibration>), 1623 (-C=N- <stretching vibration>), 1420-1520 (aromatic <in-plane vibration>), 1280 (aromatic - $NH_2$ <stretching vibration>), 807 (C-H <out-of-plane deformation vibration>)

***Example 1 [Preparation of Film from Compounds (2-2) and (1b-3) (Film 2)]***

In a thoroughly dried reactor (eggplant flask) were placed 395 mg (0.641 mmol) of the carboxylic acid compound of Formula (2-2) and 680 mg (1.28 mmol) of the amine compound of Formula (1b-3), and these were dissolved in 3.49 g of N,N-dimethylacetamide (DMAc) and 3.50 g of 1,3-dimethyl-2-imidazolidinone (DMI) added thereto, to give a solution. While stirring the solution, evacuation of the reactor using a vacuum pump and nitrogen replacement was conducted, and this procedure was repeated a total of three times. The reactor was shaded, and the solution was stirred for 1 hour and then left stand overnight without stirring. The resulting solution was filtrated sequentially through 0.2-μm and 0.1-μm PTFE filter papers, and the filtrate was applied to a silicon substrate by spin coating at 1000 rpm for 20 seconds and then at 3000 rpm for 20 seconds. Immediately thereafter, the coat was baked by heating from 50°C to 250°C, holding at 250°C for 30 minutes, heating from 250°C to 400°C, and holding at 400°C for 30 minutes, to give a Film 2. Film 2 had a thickness of 214.7 nm.

[Infrared Absorption Spectral Data ($cm^{-1}$)]

3445 (N-H <stretching vibration>), 2930 (-$CH_2$-<stretching vibration>), 1620 (-C=N- <stretching vibration>), 1420-1520 (aromatic <in-plane vibration>), 1280 (aromatic - $NH_2$ <stretching vibration>), 806 (C-H <out-of-plane deformation vibration>)

***Example 2 [Preparation of Film from Compounds (1b-1) and (2-1) (Film 3)]***

In a thoroughly dried reactor (eggplant flask) were placed 0.401 g (1.81 mmol) of 1,4-phenylenedipropionic acid of Formula (1b-1) and 1.20 mg (0.904 mmol) of the amine compound of Formula (2-1), and these were dissolved in 5.88 g of N,N-dimethylacetamide (DMAc) and 5.88 g of 1,3-dimethyl-2-imidazolidinone (DMI) added thereto, to give a solution. While stirring the solution, evacuation of the reactor using a vacuum pump and nitrogen replacement was conducted, and this procedure was repeated a total of three times. The reactor was shaded, and the solution was stirred for 1 hour and then left stand overnight without stirring. The resulting solution was filtrated sequentially through 0.2-μm and 0.1-μm PTFE filter papers, and the filtrate was applied to a silicon substrate by spin coating at 1000 rpm for 20 seconds and then at 3000 rpm for 20 seconds. Immediately thereafter, the coat was baked by heating from 50°C to 250°C, holding at 250°C for 30 minutes, heating from 250°C to 400°C, and holding at 400°C for 30 minutes, to give a

Film 3. Film 3 had a thickness of 217.2 nm.

[Infrared Absorption Spectral Data ($cm^{-1}$)]

3420 (N-H <stretching vibration>), 2933 ($-CH_2-$<stretching vibration>), 1620 (-C=N- <stretching vibration>), 1420-1520 (aromatic <in-plane vibration>), 1260 (aromatic - $NH_2$ <stretching vibration>), 810 (C-H <out-of-plane deformation vibration>)

***Example 3 [Preparation of Film from Compounds (1a-1) and (2-1) (Film 4)]***

In a thoroughly dried reactor (eggplant flask) were placed 1.00 g (0.531 mmol) of the ester compound of Formula (1a-1) and 0.707 g (0.531 mmol) of the amine compound of Formula (2-1), and these were dissolved in 7.2 g of N,N-dimethylacetamide (DMAc) and 7.1 g of 1,3-dimethyl-2-imidazolidinone (DMI) added thereto, to give a solution. While stirring the solution, evacuation of the reactor using a vacuum pump and nitrogen replacement was conducted, and this procedure was repeated a total of three times. The reactor was shaded, and the solution was stirred for 1 hour and then left stand overnight without stirring. The resulting solution was filtrated sequentially through 0.2-μm and 0.1-μm PTFE filter papers, and the filtrate was applied to a silicon substrate by spin coating at 1000 rpm for 20 seconds and then at 3000 rpm for 20 seconds. Immediately thereafter, the coat was baked by heating from 50°C to 250°C, holding at 250°C for 30 minutes, heating from 250°C to 400°C, and holding at 400°C for 30 minutes, to give a Film 4. Film 4 had a thickness of 211.1 nm.

[Infrared Absorption Spectral Data ($cm^{-1}$)]

3419 (N-H <stretching vibration>), 2931 ($-CH_2-$<stretching vibration>), 1623 (-C=N- <stretching vibration>), 1420-1520 (aromatic <in-plane vibration>), 1282 (aromatic - $NH_2$ <stretching vibration>), 805 (C-H <out-of-plane deformation vibration>)

***Example 4 [Preparation of Film from Compound (3-1) (Film 5)]***

A solution of the ethoxycarbonylbutyl-and-amino-containing adamantane derivative of Formula (3-1) prepared in Preparation Example 3 was filtrated sequentially through 0.2-μm and 0.1-μm PTFE filter papers, and the filtrate was applied to a silicon substrate by spin coating at 1000 rpm for 20 seconds and then at 3000 rpm for 20 seconds. Immediately thereafter, the coat was baked by heating from 50°C to 250°C, holding at 250°C for 30 minutes, heating from 250°C to 400°C, and holding at 400°C for 30 minutes, to give a Film 5. Film 5 had a thickness of 114.7 nm.

[Infrared Absorption Spectral Data ($cm^{-1}$)]

3419 (N-H <stretching vibration>), 2929 ($-CH_2-$<stretching vibration>), 1620 (-C=N- <stretching vibration>), 1420-1520 (aromatic <in-plane vibration>), 1280 (aromatic - $NH_2$ <stretching vibration>), 810 (C-H <out-of-plane deformation vibration>)

Evaluation Test 1

Relative dielectric constants were measured at arbitrary twelve points in each of the films prepared in Examples 1 to 4 and Comparative Example 1. The results are shown in FIGS. 9 and 10, with the abscissa indicating the number of measurement point and the ordinate indicating the relative dielectric constant. FIGS. 9 and 10 demonstrate that the film of Comparative Example 1 shows a large intra-sample variation in relative dielectric constant, but the films of Examples 1 to 4 each show a small intra-sample variation in relative dielectric constant, indicating that they are uniform films.

Evaluation Test 2

The leak currents of the films prepared in Example 1 and Comparative Example 1 were measured. The results are shown in FIG. 11 with the abscissa indicating the applied field E (V/cm) and the ordinate indicating the leak current ($A/cm^2$). FIG. 11 demonstrates that the film of Example 1 is superior in insulation to the film of Comparative Example 1.

While there have been described what are at present considered to be the preferred embodiments of the present invention, it should be understood by those skilled in the art that various modifications, combinations, subcombinations, and alterations may occur depending on design requirements and other factors insofar as they are within the

spirit and scope of the appended claims or the equivalents thereof.

## Claims

1. An N-substituted benzimidazole-containing bridged alicyclic compound represented by following Formula (1-1):

$$\left[ \left( R^a \right)_{m3} Z^3 \left[ Y^{11} - A^3 - Y^2 \left( X^3 \right)_{k2} \right] \right]_{n4} \qquad (1-1)$$

wherein $Z^3$ represents a bridged alicyclic skeleton; $Y^{11}$ represents a single bond or a divalent organic group; $Y^2$ represents a single bond or a di- or tri-valent organic group; $X^3$ represents a hydrogen atom or a reactive functional group; $R^a$ represents a hydrogen atom or a hydrocarbon group; $A^3$ represents a group represented by one of following Formulae (a) and (b):

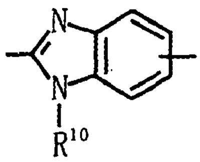
(a)

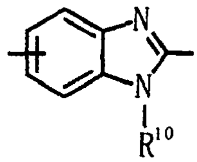
(b)

wherein $R^{10}$ represents a monovalent organic group, wherein, in each of Formulae (a) and (b), the left side is to be bonded to $Y^{11}$, and the right side is to be bonded to $Y^2$; "n4" denotes an integer of 2 to 7; "m3" denotes an integer of 0 to 5; and "k2" denotes an integer of 0 to 2, wherein the total of "n4" and "m3" equals 2 to 7, and wherein two or more $Y^{11}$s, $Y^2$s, $X^3$s, $A^3$s, and $R^{10}$s per molecule, and two or more $X^3$s and $R^a$s, if present per molecule, may be the same as or different from one another, respectively.

2. The N-substituted benzimidazole-containing bridged alicyclic compound of claim 1, wherein the monovalent organic group as $R^{10}$ is an aliphatic hydrocarbon group, an alicyclic hydrocarbon group, an aromatic hydrocarbon group, or a group composed of two or more of these groups bonded to each other with or without the interposition of at least one of oxygen atom and sulfur atom.

3. The N-substituted benzimidazole-containing bridged alicyclic compound of any one of claims 1 to 2, wherein the monovalent organic group as $R^{10}$ is a group represented by any one of the following formulae:

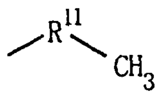

(31a)

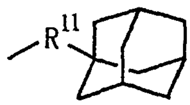

(31b)

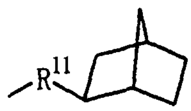

(31c)

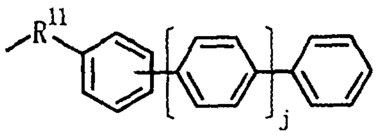

(31d)

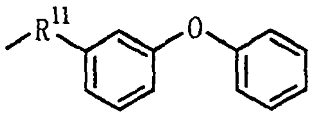

(31e)

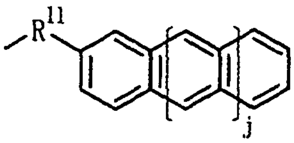

(31f)

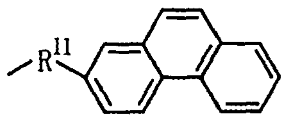

(31g)

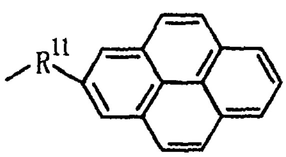

(31h)

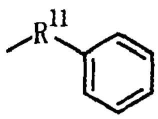

(31i)

wherein $R^{11}$ represents a single bond or a divalent aliphatic hydrocarbon group having one to fifty carbon atoms; and "j" denotes an integer of 0 to 3.

**4.** The N-substituted benzimidazole-containing bridged alicyclic compound of any one of claims 1 to 3, wherein the divalent organic groups as $Y^{11}$ and $Y^2$ are each independently an alkylene group, an alkenylene group, an alkynylene group, a divalent alicyclic hydrocarbon group, an arylene group, a divalent heterocyclic group, or a group composed of two or more of these divalent organic groups bonded to each other, or a group composed of one or more of these divalent organic groups bonded to at least one atom selected from oxygen atom (-O-) and sulfur atom (-S-).

**5.** The N-substituted benzimidazole-containing bridged alicyclic compound of any one of claims 1 to 4, wherein the divalent organic groups as $Y^{11}$ and $Y^2$ are each independently a divalent group represented by any one of the following formulae, or a divalent group composed of two or more of these groups bonded to each other:

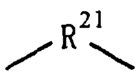 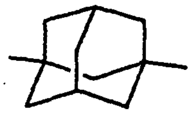 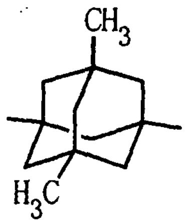 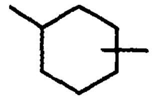 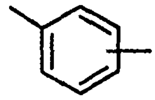

(42a) (42b) (42c) (42d) (42e)

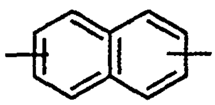 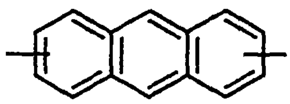 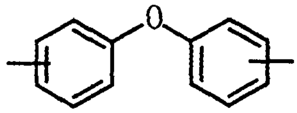

(42f) (42g) (42h)

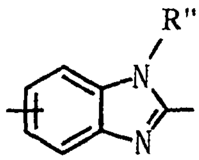 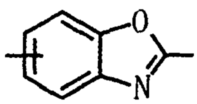 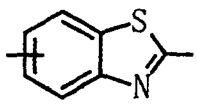

(42i) (42j) (42k)

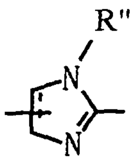 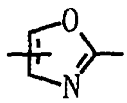 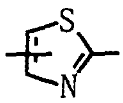

(42l) (42m) (42n)

wherein $R^{21}$ represents a divalent aliphatic hydrocarbon group having one to fifty carbon atoms; and R" represents a hydrogen atom or a monovalent organic group, wherein the left and right bonds in these formulae may direct to the left and right sides or to the right and left sides, respectively, in Formula (1-1).

**6.** The N-substituted benzimidazole-containing bridged alicyclic compound of any one of claims 1 to 5, wherein the reactive functional group as $X^3$ is a group selected from the group consisting of a substituted or unsubstituted ethynyl group, a substituted or unsubstituted vinyl group, a halogen atom, an unsubstituted or mono-substituted amino group, a haloformyl group, acid anhydride group, acid azido group, hydrazido group, cyano group, an acyl group, carboxyl group, a substituted oxycarbonyl group, hydroxyl group, mercapto group, an imino group, and an alkoxysilyl group.

**7.** An N-substituted benzimidazole-containing polymer obtained by a polymerization of a compound A', the compound A' being the N-substituted benzimidazole-containing bridged alicyclic compound of any one of claims 1 to 6 with "k2" in Formula (1-1) being 1 or 2.

**8.** An N-substituted benzimidazole-containing polymer obtained by a reaction between a compound A' and a compound B',
wherein the compound A' is the N-substituted benzimidazole-containing bridged alicyclic compound of any one of claims 1 to 6 with "k2" in Formula (1-1) being 1 or 2, and
wherein the compound B' is a polyfunctional compound comprising two or more functional groups or moieties capable of reacting with the reactive functional group $X^3$ of the compound A'.

**9.** An N-substituted benzimidazole-containing polymer comprising a repeating unit represented by any one of following

Formulae (51a), (51b) and (51c):

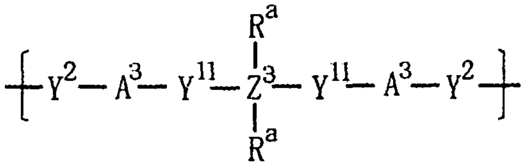

(51a)

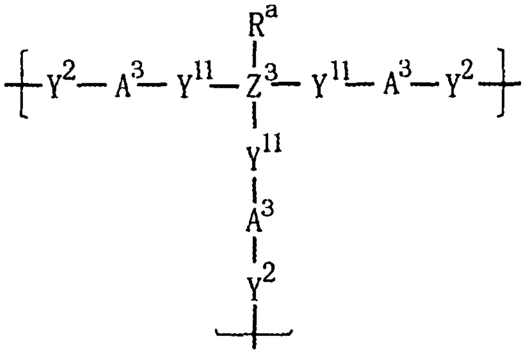

(51b)

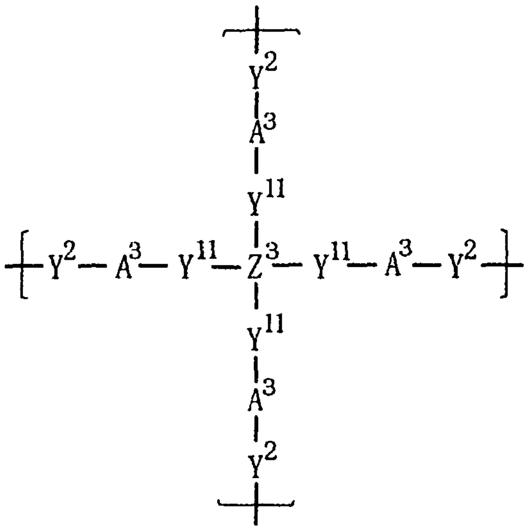

(51c)

wherein $Z^3$ represents a bridged alicyclic skeleton; $Y^{11}$ represents a single bond or a divalent organic group; $Y^2$ represents a single bond or a divalent organic group; $R^a$ represents a hydrogen atom or a hydrocarbon group; and $A^3$ represents a group represented by one of following Formulae (a) and (b):

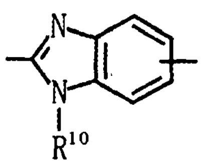

(a)

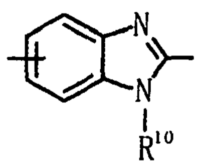

(b)

wherein $R^{10}$ represents a monovalent organic group, the left side is to be bonded to $Y^{11}$, and the right side is to be

bonded to $Y^2$ in each of Formulae (a) and (b).

**10.** A material for producing a film, comprising the N-substituted benzimidazole-containing bridged alicyclic compound of any one of claims 1 to 6, the compound dissolved in a solvent.

**11.** A material for producing a film, comprising a compound A' and a compound B' dissolved in a solvent,
wherein the compound A' is the N-substituted benzimidazole-containing bridged alicyclic compound of any one of claims 1 to 6 wherein "k2" in Formula (1-1) is 1 or 2, and
wherein the compound B' is a polyfunctional compound comprising two or more functional groups or moieties capable of reacting with the reactive functional group $X^3$ of the compound A'.

**12.** A material for producing a film, comprising the N-substituted benzimidazole-containing polymer of any one of claims 7 to 9, wherein said polymer is dissolved in a solvent.

**13.** An ethynyl-containing bridged alicyclic compound represented by following Formula (1):

$$\left[ \left( R - \right)_{k1} Z \left( - X \left( - Y \right)_m \right) \right]_{n3} \qquad (1)$$

wherein Z represents a bridged alicyclic skeleton; X represents a divalent or higher-valent organic group containing a heterocyclic ring or a precursor structure thereof; Y represents a substituted or unsubstituted ethynyl-containing group; R represents a hydrogen atom or a hydrocarbon group; "m" denotes an integer of 1 to 5; "n3" denotes an integer of 2 to 7; and "k1" denotes an integer of 0 to 5, wherein the total of "n3" and "k1" equals 2 to 7, and wherein two or more Xs and Ys per molecule, and two or more Rs, if present per molecule, may be the same as or different from each other, respectively.

**14.** The ethynyl-containing bridged alicyclic compound of claim 13, or the N-substituted benzimidazole-containing bridged alicyclic compound of any of claims 1 to 6, wherein a bridged alicyclic ring constituting the bridged alicyclic skeleton as Z or Z3, respectively, is a ring represented by any one of the following formulae, or a ring composed of two or more of these rings bonded to each other:

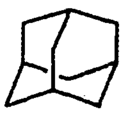
(2a)
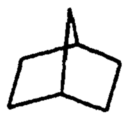
(2b)
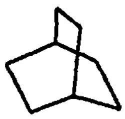
(2c)
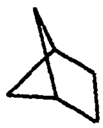
(2d)
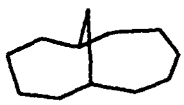
(2e)
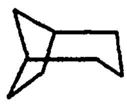
(2f)

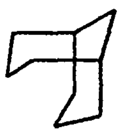
(2g)
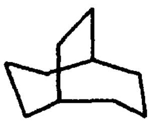
(2h)
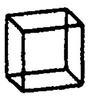
(2i)
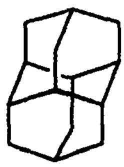
(2j)

**15.** The ethynyl-containing bridged alicyclic compound of claim 13 or 14, wherein the organic group represented by X is a group selected from the group consisting of: an imidazolyl group; a benzimidazolyl group; an oxazolyl group; a benzoxazolyl group; a thiazolyl group; a benzothiazolyl group; a precursor group of any of these heterocyclic groups; a group composed of two or more of these heterocyclic groups or their precursor groups bonded to each other; and a group composed of one or more of these heterocyclic groups or their precursor groups bonded to one or more aromatic hydrocarbon groups.

**16.** The ethynyl-containing bridged alicyclic compound of anyone of claims 13, 14 and 15, wherein the organic group represented by X is a group represented by any one of the following formulae, or a group composed of two or more of these groups bonded to each other:

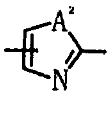
(3a)

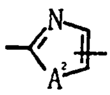
(3b)

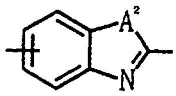
(3c)

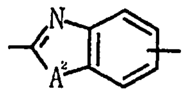
(3d)

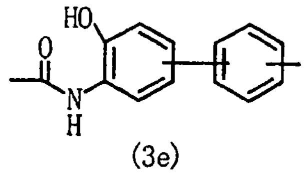
(3e)

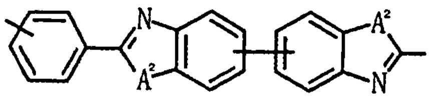
(3f)

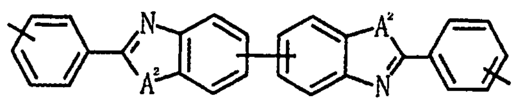
(3g)

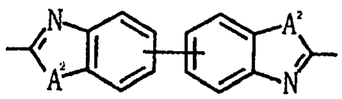
(3h)

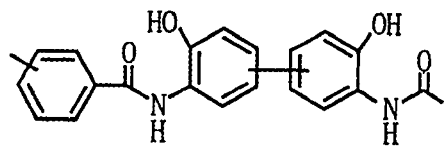
(3i)

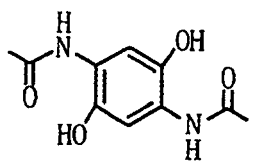
(3j)

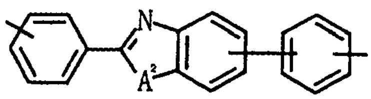
(3k)

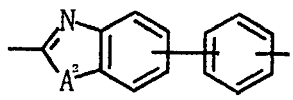
(3l)

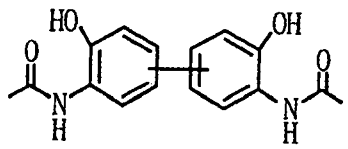
(3m)

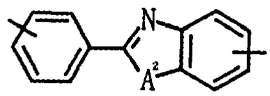
(3n)

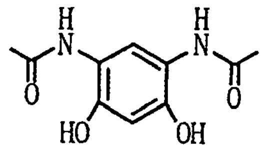
(3o)

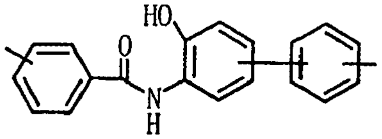
(3p)

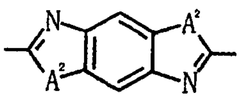
(3q)

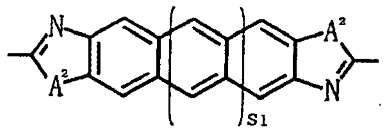
(3r)

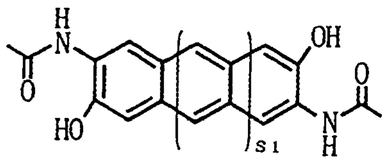

(3s)

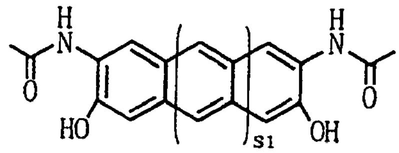

(3t)

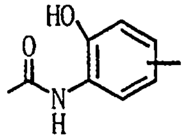

(3u)

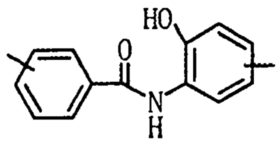

(3v)

wherein $A^2$ represents -NH-, oxygen atom, or sulfur atom; and "s1" denotes an integer of 0 to 5, and wherein each of rings in the formulae may have one or more substituents.

**17.** A material for producing an insulating film, the material comprising the ethynyl-containing bridged alicyclic compound of any one of claims 13 to 16, optionally further comprising one or more other ethynyl-containing compounds.

**18.** A material for producing an insulating film, the material comprising a pair of compounds A and B, and/or a compound C: wherein the pair of the compounds A and B each contain two or more functional groups or moieties per molecule and form a polymer having a pore structure as a result of polymerization through binding of the functional group or moiety of one compound with the functional group or moiety of the other compound;
wherein the compound C contains two or more functional groups or moieties per molecule and forms a polymer having a pore structure as a result of polymerization through binding of one of the functional group or moiety with the other of the functional group or moiety; and
wherein in the pair of the compounds A and B, and/or the compound C satisfy the following condition (i) or (ii):

(i) at least one of the compounds A and B contains a bridged alicyclic skeleton or an aromatic skeleton as a central skeleton,

at least one of the compounds A and B has a thermally stable skeleton positioned between the central skeleton and the functional groups or moieties and the thermally stable skeleton is composed of an aromatic-ring-containing divalent organic group,
at least one of the compounds A and B intramolecularly has a flexible unit composed of an organic group containing at least an alkylene group or ether bond and having a total of two to twenty atoms, and
the functional groups or moieties of the compound A and the functional groups or moieties of the compound B constitute a pair of functional groups or moieties capable of reacting with each other to form a heterocyclic ring, or the functional groups or moieties of the compound A and the functional groups or moieties of the compound B are both substituted or unsubstituted ethynyl-containing groups; or

(ii) the compound C contains a bridged alicyclic skeleton or an aromatic skeleton as a central skeleton,

the compound C has a thermally stable skeleton composed of an aromatic-ring-containing divalent organic group and positioned between the central skeleton and the one of the functional group or moiety and/or between the central skeleton and the other of the functional group or moiety,
the compound C has a flexible unit between the central skeleton and the one of the functional group or moiety and/or between the central skeleton and the other of the functional group or moiety, the flexible unit composed of an organic group containing at least an alkylene group or ether bond and having a total of two to twenty atoms, and
the one the functional group or moiety and the other of the functional group or moiety of the compound C constitute a pair of functional groups or moieties capable of reacting with each other to form a heterocyclic ring, or are both substituted or unsubstituted ethynyl-containing groups.

**19.** The material for producing an insulating film of claim 18, wherein the compounds A, B, and C satisfy the following condition (iii) or (iv):

(iii) the compound A is a compound represented by following Formula (1a) or Formula (1b), and the compound B is a compound represented by following Formula (2):

Formula (1a):

$$\left(R^1 \right)_{n2} X^1 \left( Y^{1a} - W^1 - Y^{1b} - Z^1 \right)_{n1} \qquad (1a)$$

wherein $X^1$ represents a di-, tri-, or tetra-valent bridged alicyclic group or aromatic group; $Y^{1a}$ and $Y^{1b}$ are the same as or different from each other and each represent a single bond, a divalent aromatic hydrocarbon group, a divalent heteroaromatic group, a divalent group corresponding to a precursor of the divalent heteroaromatic group, or a divalent group composed of two or more of these groups bonded to each other; $W^1$ represents a flexible unit composed of a divalent group containing at least an alkylene group or ether bond and having a total of two to twenty atoms; $Z^1$ represents a functional group or moiety capable of reacting with $Z^2$ in following Formula (2) to form a heterocyclic ring, or, only when $Z^2$ in Formula (2) is a substituted or unsubstituted ethynyl-containing group, $Z^1$ represents a substituted or unsubstituted ethynyl-containing group; $R^1$ represents a hydrogen atom or a hydrocarbon group; "n1" denotes an integer of 2 to 4; "n2" denotes an integer of 0 to 2, and wherein the total of "n1" and "n2" equals 2 to 4; two or more $Y^{1a}$s, $Y^{1b}$s, $W^1$s, and $Z^1$s per molecule and two or more $R^1$s, if present per molecule, may be the same as or different from each other, respectively; or

Formula (1b):

$$W \left( Y^1 - Z^1 \right)_n \qquad (1b)$$

wherein $Y^1$ represents a single bond, a divalent aromatic hydrocarbon group, a divalent heteroaromatic group, a divalent group corresponding to a precursor of the divalent heteroaromatic group, or a divalent group composed of two or more of these groups bonded to each other; W represents a flexible unit composed of a di-, tri-, or tetra-valent group containing at least an alkylene group or ether bond and having a total of two to twenty atoms; $Z^1$ represents a functional group or moiety capable of reacting with $Z^2$ in following Formula (2) to form a heterocyclic ring, or, only when $Z^2$ in Formula (2) is a substituted or unsubstituted ethynyl-containing group, $Z^1$ represents a substituted or unsubstituted ethynyl-containing group; and "n" denotes an integer of 2 to 4, wherein two or more $Y^1$s and $Z^1$s per molecule may be the same as or different from each other, respectively; and

Formula (2):

$$\left( \left( R^2 \right)_{m2} X^2 \right)_i \left( Y^{2a} \left( W^2 - Y^{2b} \right)_k Z^2 \right)_{m1} \qquad (2)$$

wherein $X^2$ represents a di-, tri-, or tetra-valent bridged alicyclic group or aromatic group; $Y^{2a}$ and $Y^{2b}$ are the same as or different from each other and each represent a single bond, a divalent aromatic hydrocarbon group, a divalent heteroaromatic group, a divalent group corresponding to a precursor of the divalent heteroaromatic group, or a divalent group composed of two or more of these groups bonded to each other; $W^2$ represents a flexible unit composed of a divalent group containing at least an alkylene group or ether bond and having a total of two to twenty atoms; $Z^2$ represents a functional group or moiety capable of reacting with $Z^1$ in Formula (1a) or (1b) to form a heterocyclic ring, or, only when $Z^1$ in Formula (1a) or (1b) is a substituted or unsubstituted

ethynyl-containing group, $Z^2$ represents a substituted or unsubstituted ethynyl-containing group; $R^2$ represents a hydrogen atom or a hydrocarbon group; "m1" denotes an integer of 2 to 4; "m2" denotes an integer of 0 to 2, wherein the total of "m1" and "m2" equals 2 to 4; "i" denotes 0 or 1; and "k" denotes 0 or 1, wherein two or more $Y^{2a}$S, $Y^{2b}$S, $W^2$S, and $Z^2$S per molecule and two or more $R^2$S, if present per molecule, may be the same as or different from each other, respectively; or

(iv) the compound C is a compound represented by following Formula (3):

$$\left(Z^1 - Y^{1b} - W^1 - Y^{1a}\right)_{p1} \underset{\left(R^1\right)_{p3}}{\underset{|}{X^1}} \left(Y^{2a} \left(W^2 - Y^{2b}\right)_k Z^2\right)_{p2} \qquad (3)$$

wherein $X^1$ represents a di-, tri-, or tetra-valent bridged alicyclic group or aromatic group; $Y^{1a}$, $Y^{1b}$, $Y^{2a}$, and $Y^{2b}$ are the same as or different from one another and each represent a single bond, a divalent aromatic hydrocarbon group, a divalent heteroaromatic group, a divalent group corresponding to a precursor of the divalent heteroaromatic group, or a divalent group composed of two or more of these groups bonded to each other; $W^1$ and $W^2$ are the same as or different from each other and each represent a flexible unit composed of a divalent group containing at least an alkylene group or ether bond and having a total of two to twenty atoms; $Z^1$ and $Z^2$ are a pair of functional groups or moieties capable of reacting with each other to form a heterocyclic ring, or $Z^1$ and $Z^2$ are both substituted or unsubstituted ethynyl-containing groups; $R^1$ represents a hydrogen atom or a hydrocarbon group; "k" denotes 0 or 1; each of "p1" and "p2" independently denotes an integer of 1 to 3; and "p3" denotes an integer of 0 to 2, wherein the total of "p1", "p2", and "p3" equals 2 to 4, and wherein two or more $Y^{1a}$ s, $Y^{1b}$s, $Y^{2a}$s, $Y^{2b}$s, $W^1$s, $W^2$s, $Z^1$s, $Z^2$s, and $R^1$s, if present per molecule, may be the same as or different from each other, respectively.

**20.** The material for producing an insulating film of claim 19, wherein a bridged alicyclic ring or aromatic ring constituting the di-, tri-, or tetra-valent bridged alicyclic group or aromatic group as $X^1$ in Formula (1a) and/or Formula (3) is a ring represented by any one of the following formulae, or a ring composed of two or more of these rings bonded to each other;

or the material for producing an insulating film of claim 18, wherein a bridged alicyclic ring or aromatic ring constituting the bridged alicyclic skeleton or aromatic skeleton as the central skeleton of at least one of the compounds A and B, or that of the compound C is a ring represented by any one of the following formulae, or a ring composed of two or more of these rings bonded to each other:

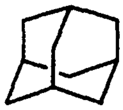
(4a)
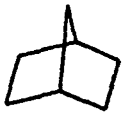
(4b)
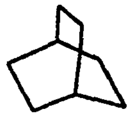
(4c)
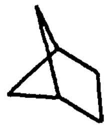
(4d)
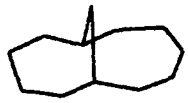
(4e)
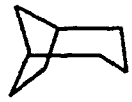
(4f)

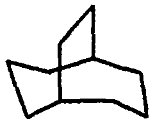
(4g)
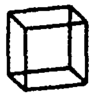
(4h)
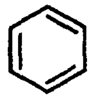
(4i)
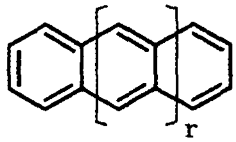
(4j)
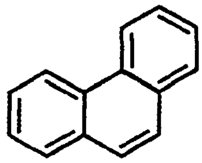
(4k)

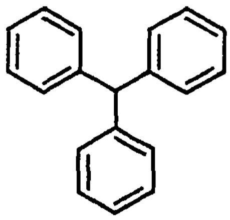
(4l)
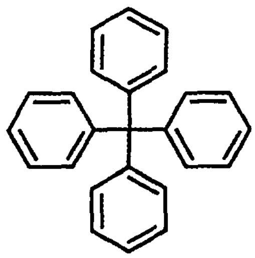
(4m)
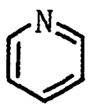
(4n)
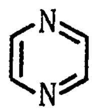
(4o)
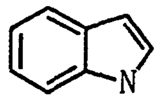
(4p)

wherein "r" denotes an integer of 0 to 5.

**21.** The material for producing an insulating film of claim 19, wherein each of $Y^{1a}$s, $Y^{1b}$s, $Y^{1}$s, $Y^{2a}$s, and $Y^{2b}$s in Formulae (1a), (1b), (2), and (3) is independently a single bond, or a group represented by any one of the following formulae, or a group composed of two or more of these groups bonded to each other; or the

material for producing an insulating film of claim 18, wherein the thermally stable skeleton of at least one of the compounds A and B, or the thermally stable skeleton of the compound C is a group represented by any one any one of the following formulae, or a group composed of two or more of these groups bonded to each other:

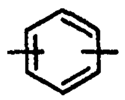 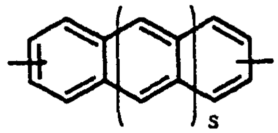 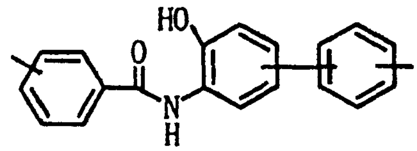

(5a) (5b) (5c)

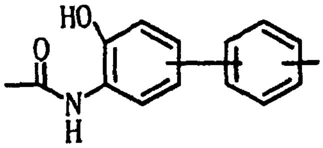 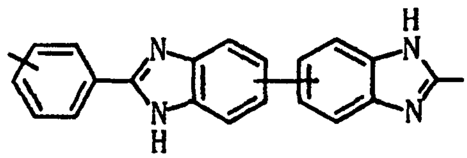

(5d) (5e)

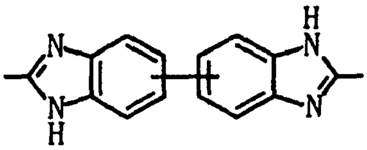 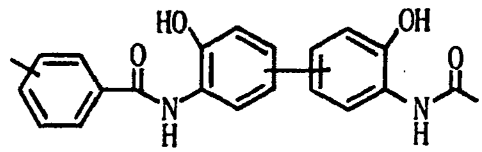

(5f) (5g)

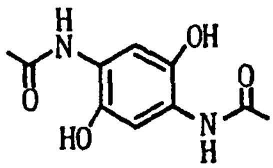 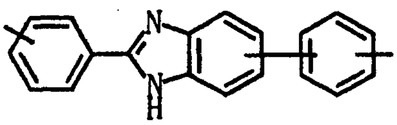 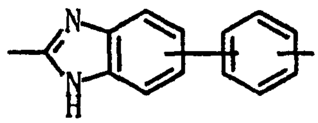

(5h) (5i) (5j)

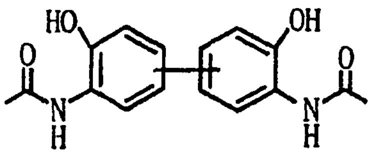 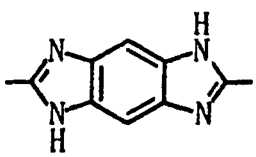 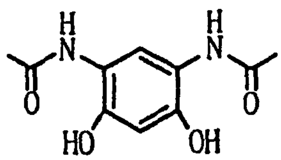

(5k) (5l) (5m)

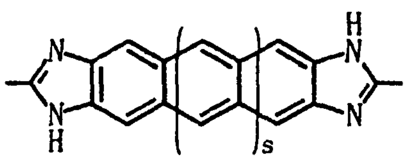 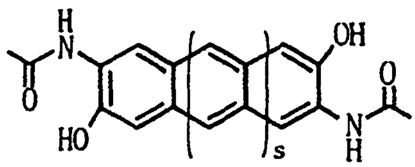

(5n) (5o)

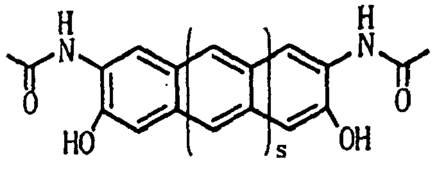

(5p)

wherein "s" denotes an integer of 0 to 5.

**22.** The material for producing an insulating film of claim 19, wherein each of $W^1$s, W, and $W^2$s in Formulae (1a), (1b), (2), and (3) is independently a flexible unit comprising a group represented by any one of the following formulae; or the material for producing an insulating film of claim 18, wherein the flexible unit of at least one of the compounds A and B, or the flexible unit compound C is a flexible unit composed of a group represented by any one of the following formulae:

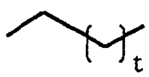
(6a)

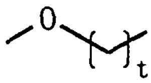
(6b)

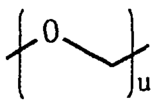
(6c)

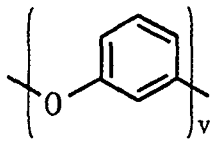
(6d)

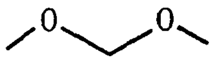
(6e)

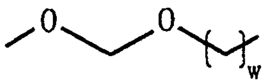
(6f)

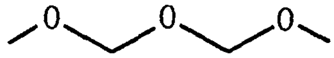
(6g)

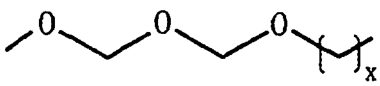
(6h)

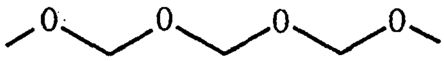
(6i)

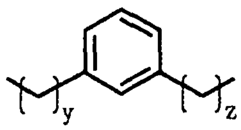
(6j)

wherein "t" denotes an integer of 1 to 19; "u" denotes an integer of 1 to 10; "v" denotes an integer of 1 to 3; "w" denotes an integer of 1 to 16; "x" denotes an integer of 1 to 14; and each of "y" and "z" independently denotes an integer of 0 to 6, wherein both of "y" and "z" are not simultaneously zero (0).

**23.** The material for producing an insulating film of claim 19, wherein the heterocyclic ring formed as a result of a reaction between $Z^1$ in Formula (1a) or (1b) and $Z^2$ in Formula (2), and/or the heterocyclic ring formed as a result of a reaction between $Z^1$ in Formula (3) and $Z^2$ in Formula (3) is a ring selected from a group consisting of benzimidazole ring, benzoxazole ring, and benzothiazole ring; or the material for producing an insulating film of claim 18, wherein the heterocyclic ring formed as a result of a reaction between the functional groups or moieties of the compound A and the functional groups or moieties of the compound B, or the heterocyclic ring formed as a result of a reaction between the one of the functional group or moiety and the other of the functional group or moiety of the compound C is a ring selected from a group consisting of benzimidazole ring, benzoxazole ring, and benzothiazole ring.

**24.** The material for producing an insulating film of claim 19,
wherein one of $Z^1$, in Formula (1a) or (1b), and $Z^2$ in Formula (2), or one of $Z^1$ and $Z^2$ in Formula (3) is a group selected from a group consisting of a carboxyl group, a substituted oxycarbonyl group, formyl group, a haloformyl group, and a substituted or unsubstituted ethynyl-containing group,
wherein the other of $Z^1$, in Formula (1a) or (1b), and $Z^2$ in Formula (2), or the other of $Z^1$ and $Z^2$ in Formula (3) is a group selected from a group consisting of 3,4-diaminophenyl group, 3-amino-4-hydroxyphenyl group, 4-amino-3-hydroxyphenyl group, 3-amino-4-mercaptophenyl group, 4-amino-3-mercaptophenyl group, and a substituted or unsubstituted ethynyl-containing group, and
wherein, when the one is a substituted or unsubstituted ethynyl-containing group, the other is also a substituted or unsubstituted ethynyl-containing group.

**25.** The material for producing an insulating film of any one of claims 18 to 24, wherein the compounds A and B, and/or the compound C are dissolved in an organic solvent and are a solution.

**26.** A polymer having a pore structure, obtained by polymerization of the material for producing an insulating film of any one of claims 18 to 24.

**27.** A producing method of an insulating film, the method comprising the steps of:

applying the material for producing an insulating film of claim 25 to a substrate; and
carrying out polymerization of the applied material to form an insulating film composed of a polymer having a pore structure; or comprising the steps of:

applying the material of any one of claims 10 to 12 to a substrate; and
drying the applied material or carrying out a reaction of the applied material by heating, to give a thin film; or comprising the steps of:

applying the material of claim 17 to a substrate; and carrying out polymerization of the applied material to form the insulating film containing a polymer having a pore structure.

**28.** A film obtained by the method of claim 27 or a film comprising the polymer of claim 26.

**29.** A polymerizable compound represented by the following formula (7):

$$\left( R^1 \right)_{n2} X^1 \left( Y^{1a} - W^1 - Y^{1b} - Z^1 \right)_{n1} \qquad (7)$$

wherein $X^1$ represents a di-, tri-, or tetra-valent aromatic or non-aromatic cyclic group; $Y^{1a}$ and $Y^{1b}$ are the same as or different from each other and each represent a single bond, or a group selected from a group consisting of a divalent aromatic hydrocarbon group, a divalent heteroaromatic group, a divalent group corresponding to a precursor of the divalent heteroaromatic group, and a divalent group composed of two or more of these groups bonded to each other, wherein at least one of $Y^{1a}$ and $Y^{1b}$ is a divalent heteroaromatic group or a group containing a divalent group corresponding to a precursor of the divalent heteroaromatic group; $W^1$ represents a flexible unit composed of a divalent group containing at least an alkylene group or ether bond and having a total of two to twenty atoms; $Z^1$ represents a group selected from a group consisting of a carboxyl group, a substituted oxycarbonyl group, a formyl group, a haloformyl group, a substituted or unsubstituted ethynyl-containing group, 3,4-diaminophenyl group, 3-amino-4-hydroxyphenyl group, 4-amino-3-hydroxyphenyl group, 3-amino-4-mercaptophenyl group, and 4-amino-3-mercaptophenyl group; $R^1$ represents a hydrogen atom or a hydrocarbon group; "n1" denotes an integer of 2 to 4; and "n2" denotes an integer of 0 to 2, wherein the total of "n1" and "n2" equals 2 to 4, and wherein two or more $Y^{1a}$s, $Y^{1b}$s, $W^1$s, and $Z^1$s per molecule and two or more $R^1$s, if present per molecule, may be the same as or different from each other, respectively.

**30.** The polymerizable compound of claim 29, wherein at least one of $Y^{1a}$ and $Y^{1b}$ is a divalent heteroaromatic group containing at least one of benzimidazole ring, benzoxazole ring, and benzothiazole ring, or a divalent group corresponding to a precursor of the divalent heteroaromatic group.

**31.** A polymerizable compound represented by following Formula (8):

$$W \left( Y^1 - Z^1 \right)_n \qquad (8)$$

wherein $Y^1$ represents a divalent heteroaromatic group or a divalent group corresponding to a precursor of the divalent heteroaromatic group; W represents a flexible unit composed of a di-, tri-, or tetra-valent group containing at least an alkylene group or ether bond and having a total of two to twenty atoms; $Z^1$ represents a group selected from a group consisting of a carboxyl group, a substituted oxycarbonyl group, a formyl group, a haloformyl group, a substituted or unsubstituted ethynyl-containing group, 3,4-diaminophenyl group, 3-amino-4-hydroxyphenyl group, 4-amino-3-hydroxyphenyl group, 3-amino-4-mercaptophenyl group, and 4-amino-3-mercaptophenyl group; and "n" denotes an integer of 2 to 4, wherein two or more $Y^1$s and $Z^1$s per molecule may be the same as or different from each other, respectively.

**32.** The polymerizable compound of claim 31, wherein $Y^1$s are each a divalent heteroaromatic group containing at least one of benzimidazole ring, benzoxazole ring, and benzothiazole ring, or a divalent group corresponding to a precursor of the divalent heteroaromatic group.

**33.** A polymerizable compound represented by following Formula (9):

$$\left( Z^1 - Y^{1b} - W^1 - Y^{1a} \right)_{p1} \underset{\left( R^1 \right)_{p3}}{\underset{|}{X^1}} \left( Y^{2a} \left( W^2 - Y^{2b} \right)_k Z^2 \right)_{p2} \quad (9)$$

wherein $X^1$ represents a di-, tri-, or tetra-valent organic group; $Y^{1a}$, $Y^{1b}$, $Y^{2a}$, and $Y^{2b}$ are the same as or different from one another and each represent a single bond or a group selected from a group consisting of a divalent aromatic hydrocarbon group, a divalent heteroaromatic group, a divalent group corresponding to a precursor of the divalent heteroaromatic group, and a divalent group composed of two or more of these groups bonded to each other, wherein at least one of $Y^{1a}$ and $Y^{1b}$ is a divalent heteroaromatic group or a group containing a divalent group corresponding to a precursor of the divalent heteroaromatic group; $W^1$ and $W^2$ are the same as or different from each other and each represent a flexible unit composed of a divalent group containing at least an alkylene group or ether bond and having a total of two to twenty atoms; each of $Z^1$ and $Z^2$ independently represents a group selected from a group consisting of a carboxyl group, a substituted oxycarbonyl group, a formyl group, a haloformyl group, a substituted or unsubstituted ethynyl-containing group, 3,4-diaminophenyl group, 3-amino-4-hydroxyphenyl group, 4-amino-3-hydroxyphenyl group, 3-amino-4-mercaptophenyl group, or 4-amino-3-mercaptophenyl group; $R^1$ represents a hydrogen atom or a hydrocarbon group; "k" denotes 0 or 1; each of "p1" and "p2" independently denotes an integer of 1 to 3; and "p3" denotes an integer of 0 to 2, wherein the total of "p1", "p2", and "p3" equals 2 to 4, and wherein two or more $Y^{1a}$s, $Y^{1b}$s, $Y^{2a}$s, $Y^{2b}$s, $W^1$s, $W^2$s, $Z^1$s, $Z^2$s, and $R^1$s, if present per molecule, may be the same as or different from each other, respectively.

**34.** The polymerizable compound of claim 33, wherein at least one of $Y^{1a}$ and $Y^{1b}$ is a divalent heteroaromatic group containing at least one of benzimidazole ring, benzoxazole ring, and benzothiazole ring, or a divalent group corresponding to a precursor of the divalent heteroaromatic group.

**35.** The polymerizable compound of claim 33 or claim 34, wherein at least one of $Y^{2a}$ and $Y^{2b}$ is a divalent heteroaromatic group containing at least one of benzimidazole ring, benzoxazole ring, and benzothiazole ring, or a divalent group corresponding to a precursor of the divalent heteroaromatic group.

Figure 1

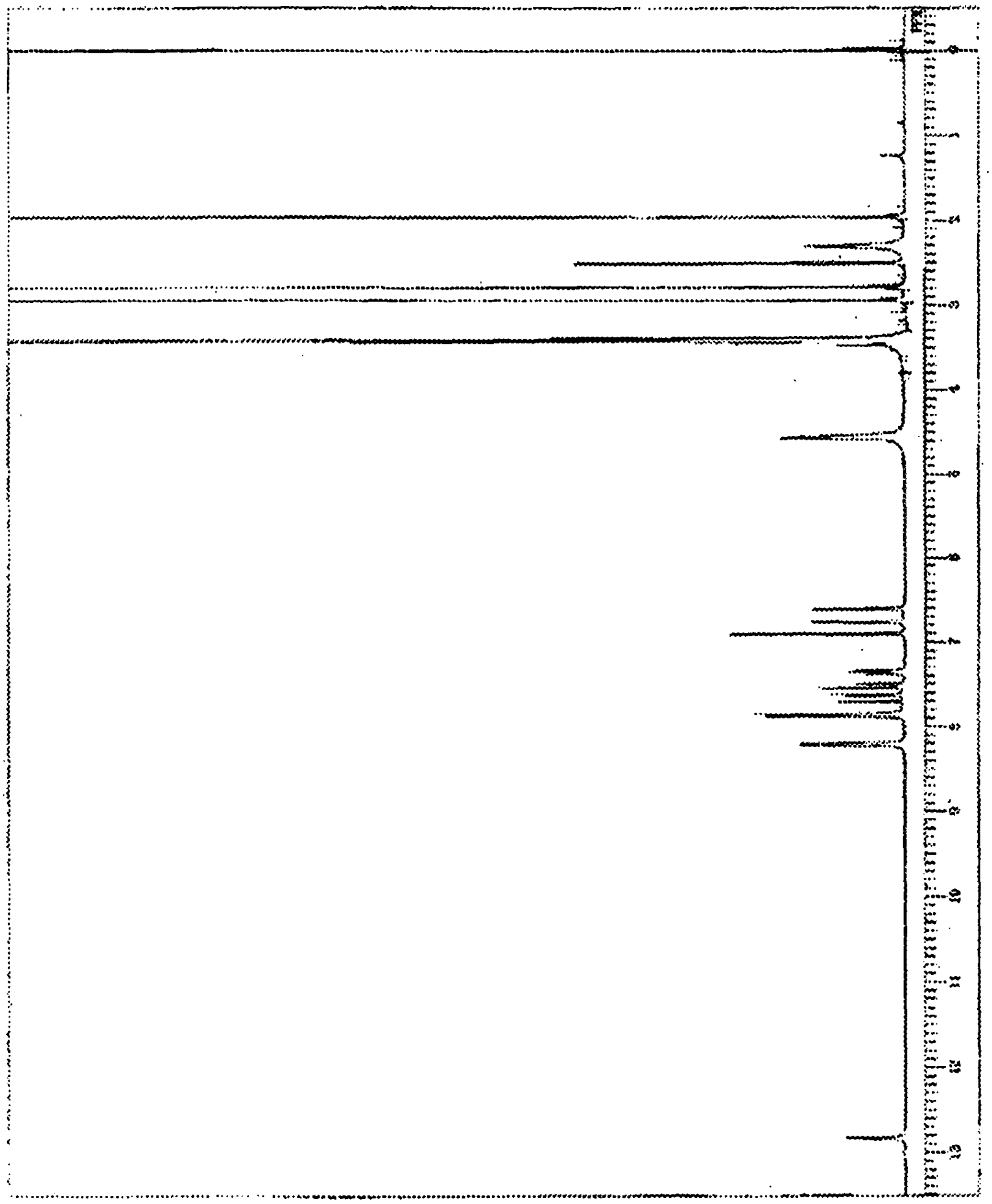

# Figure 2

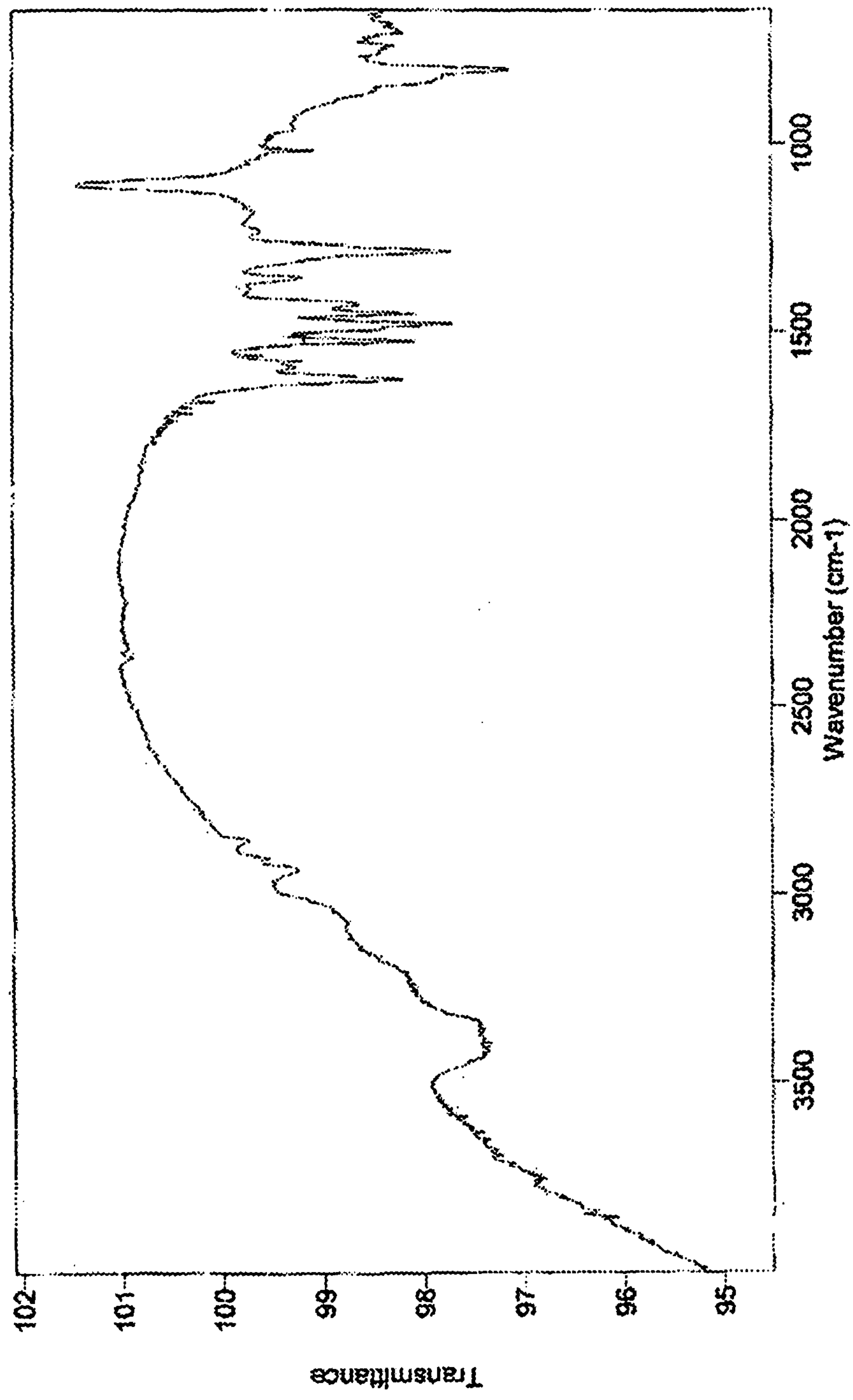
Transmittance
102
101
100
99
98
97
96
95
1000
1500
2000
2500
3000
3500
Wavenumber (cm-1)

# Figure 3

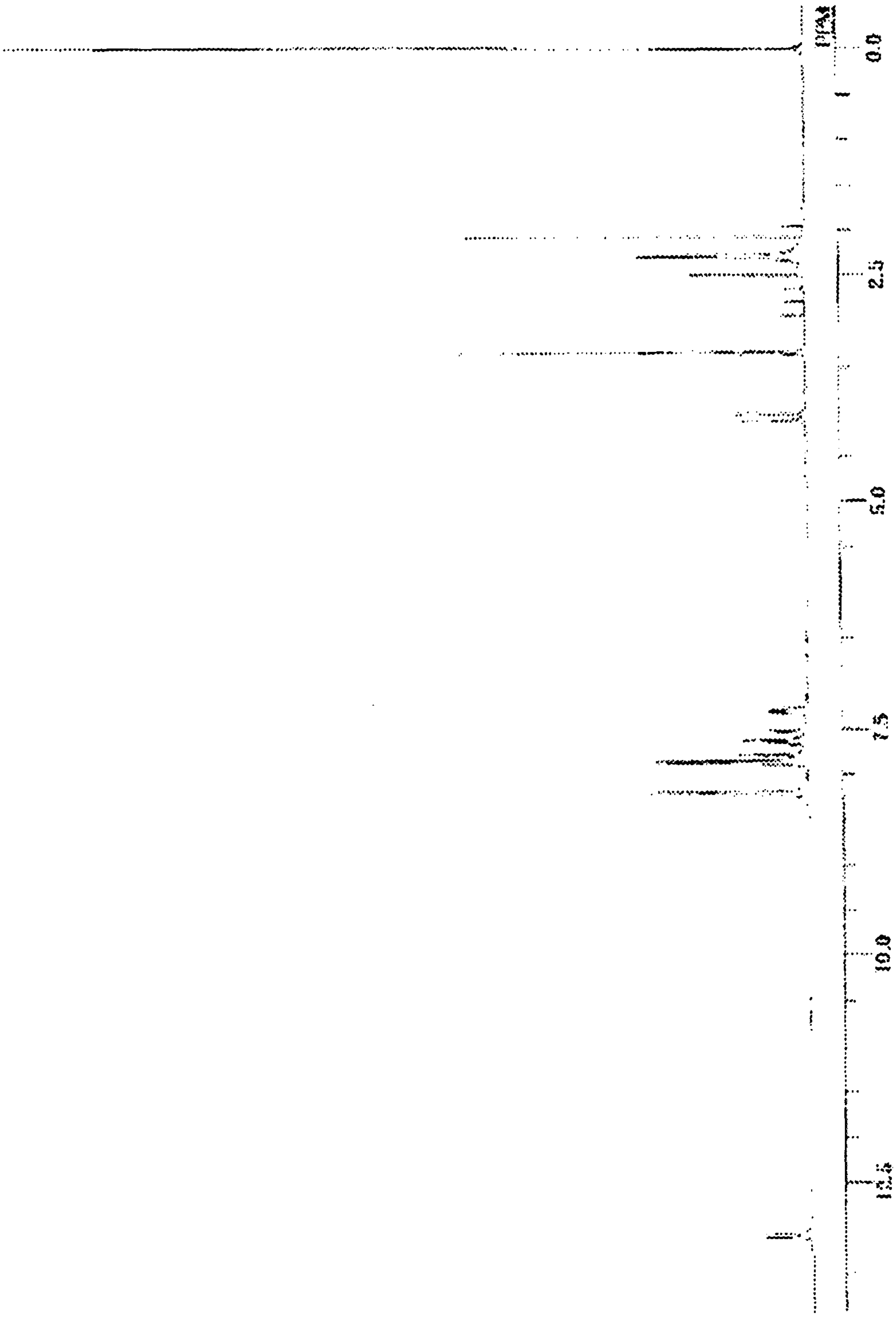

## Figure 4

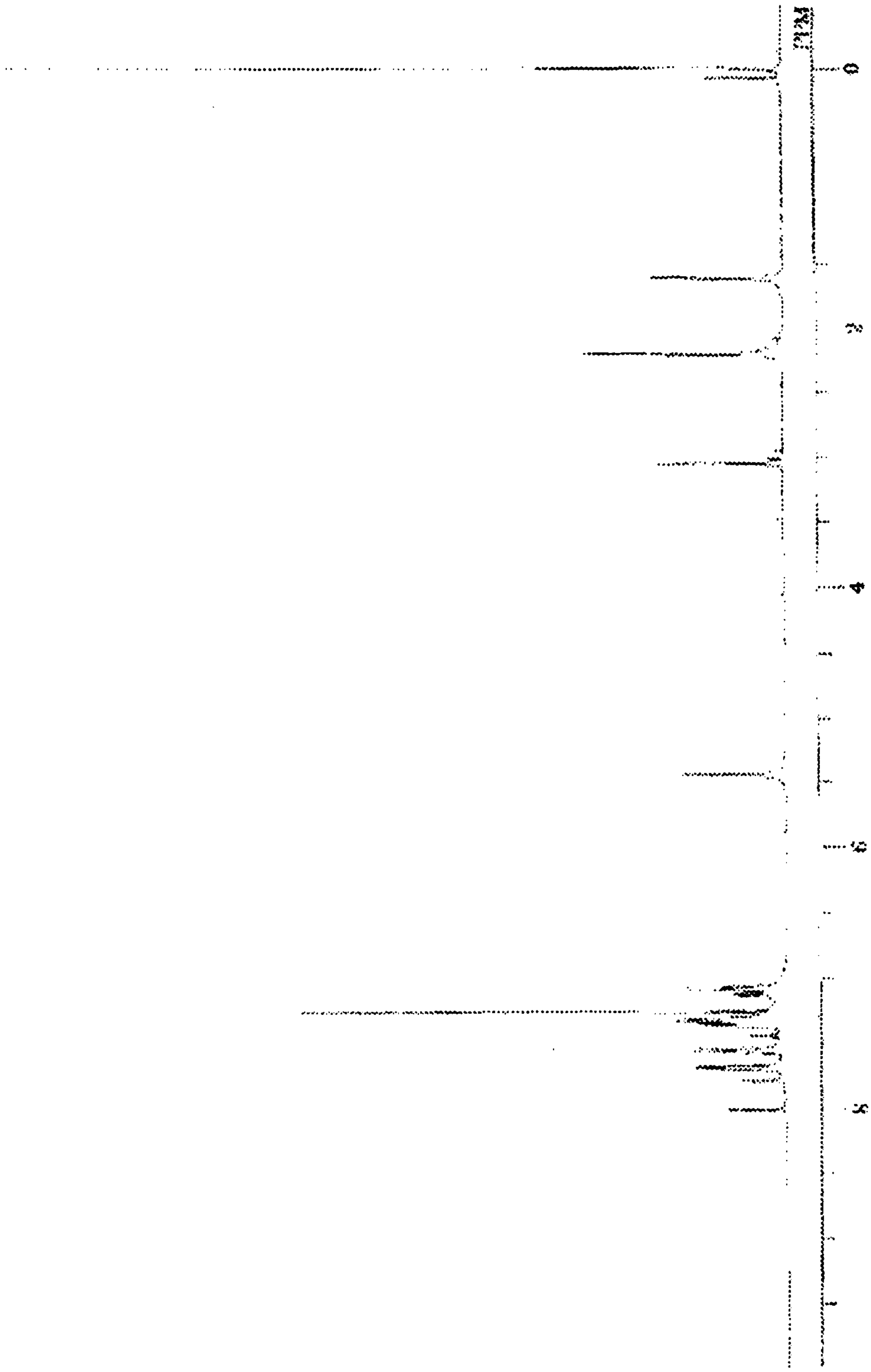

# Figure 5

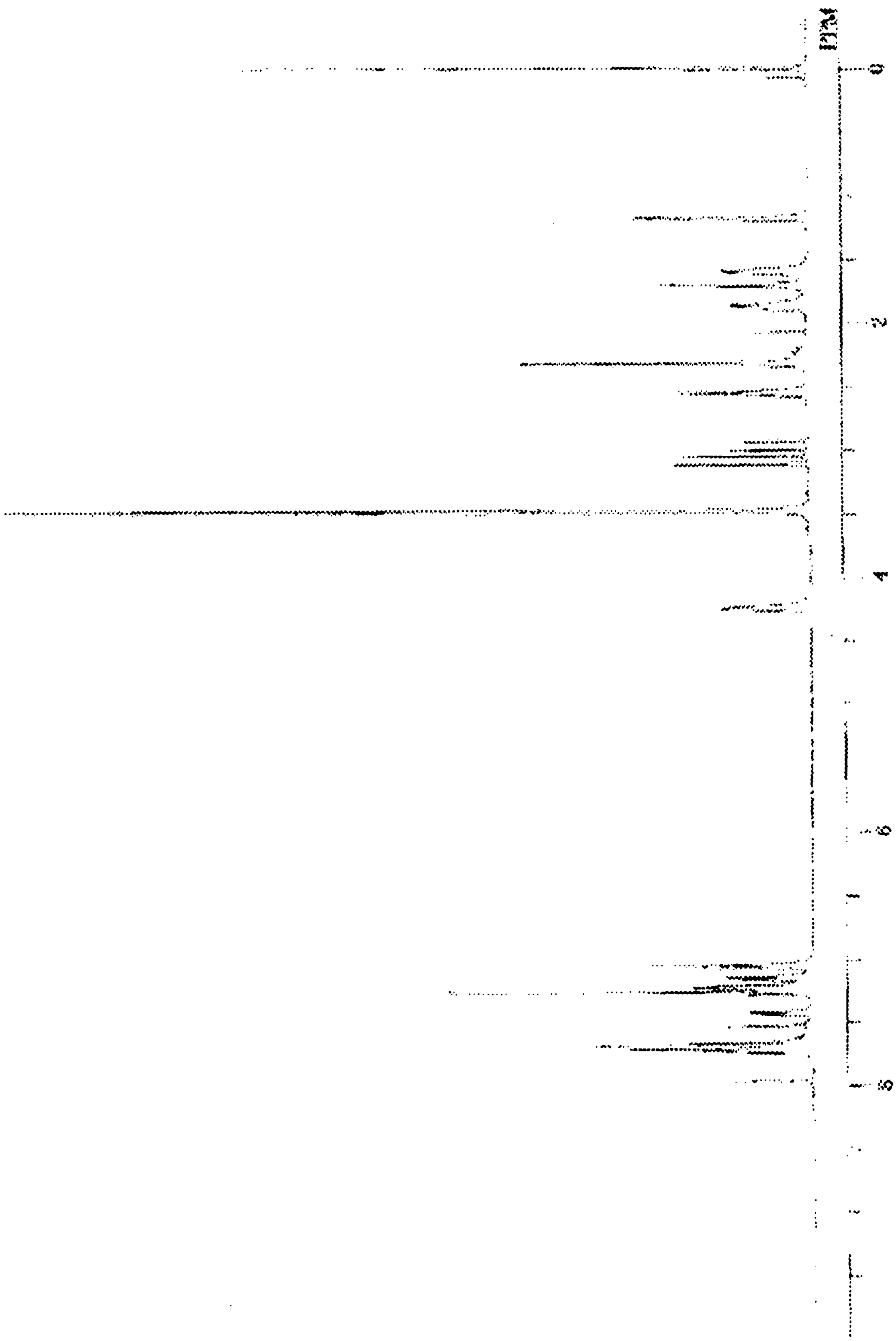

# Figure 6

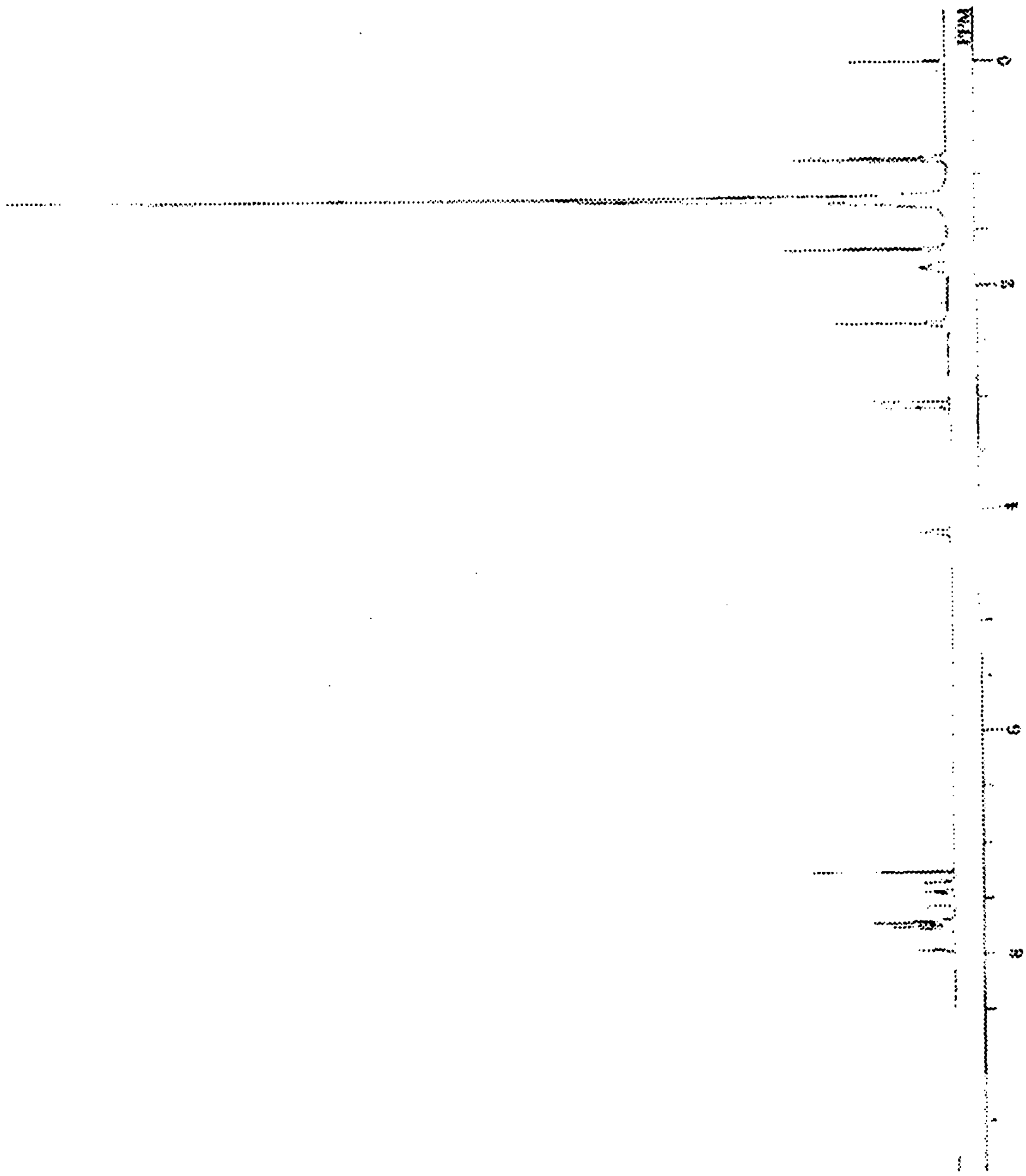

## Figure 7

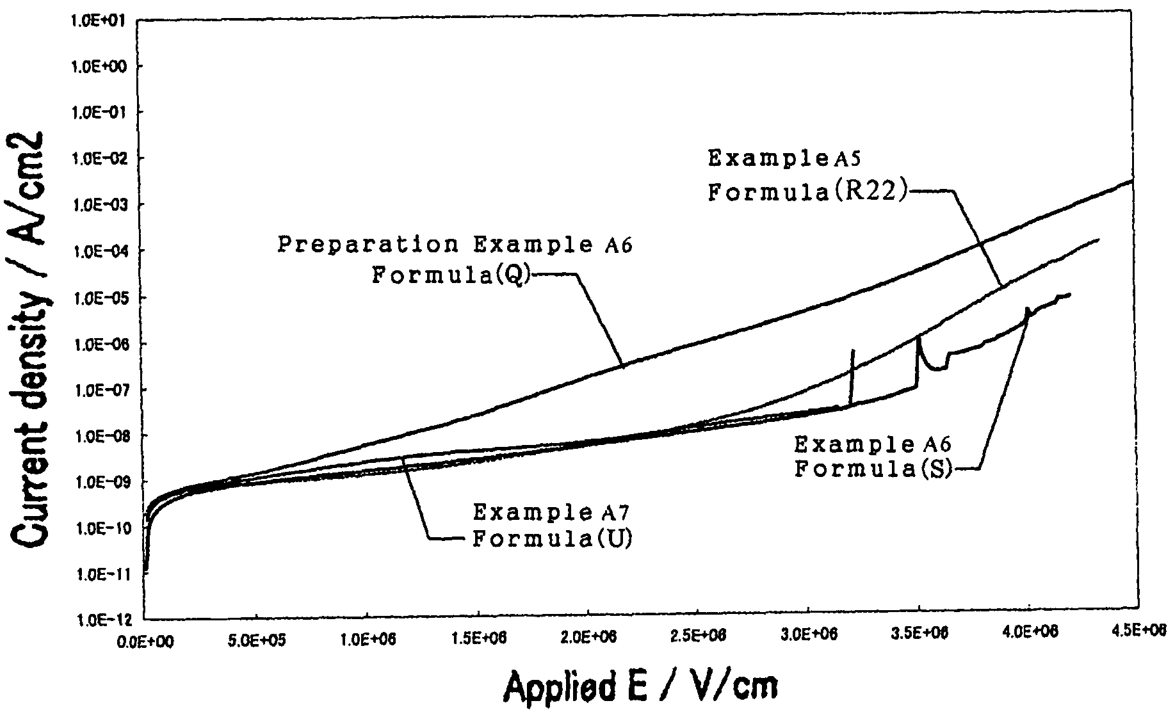
Current density / A/cm2
1.0E+01
1.0E+00
1.0E-01
1.0E-02
1.0E-03
1.0E-04
1.0E-05
1.0E-06
1.0E-07
1.0E-08
1.0E-09
1.0E-10
1.0E-11
1.0E-12
0.0E+00
5.0E+05
1.0E+06
1.5E+06
2.0E+06
2.5E+06
3.0E+06
3.5E+06
4.0E+06
4.5E+06
Applied E / V/cm
Example A5
Formula(R22)
Preparation Example A6
Formula(Q)
Example A6
Formula(S)
Example A7
Formula(U)

Figure 8

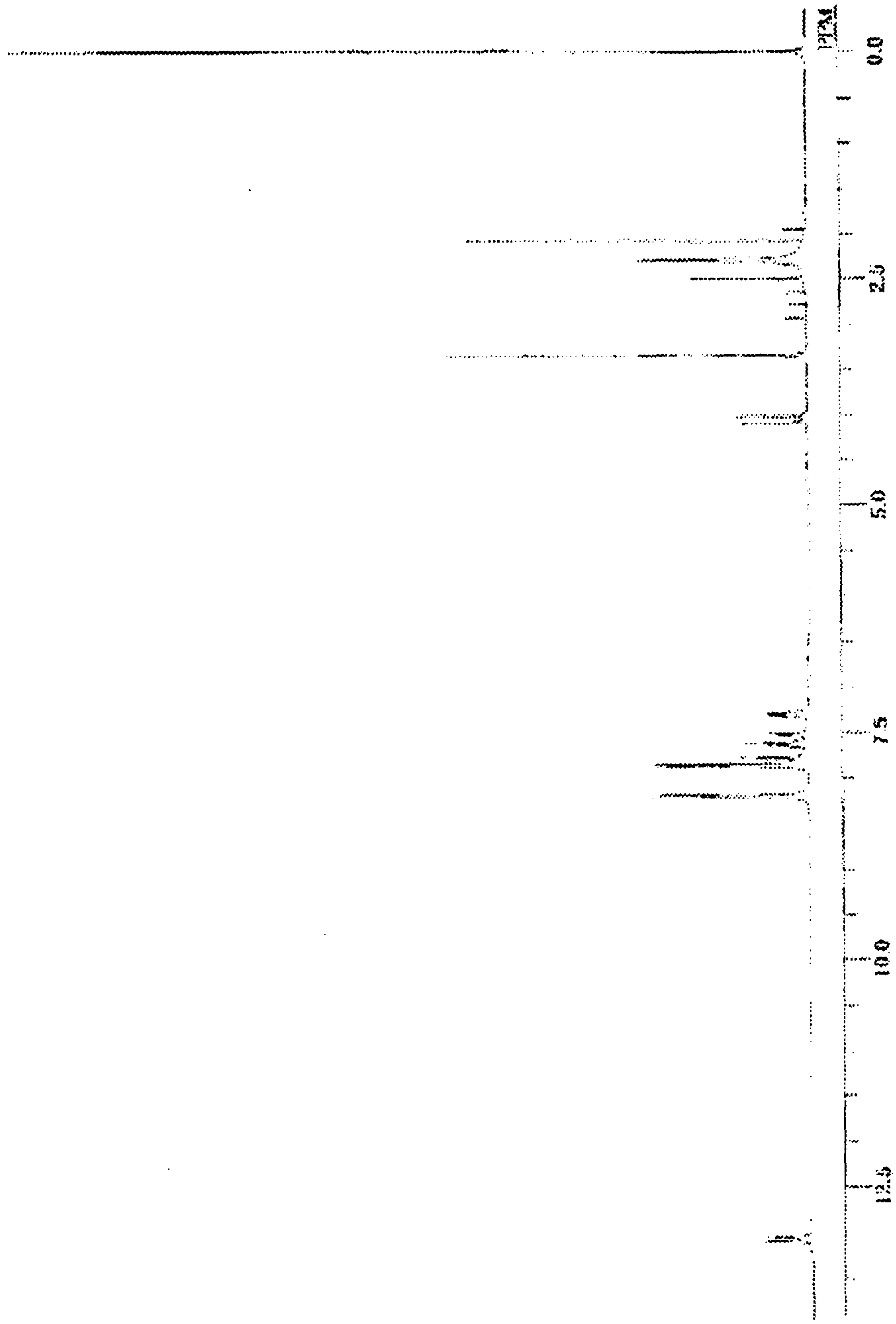

## Figure 9

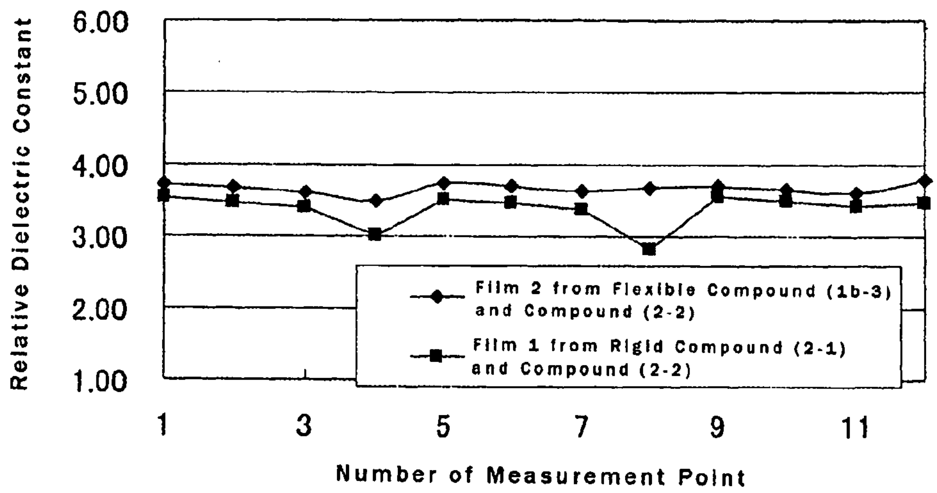


## Figure 10

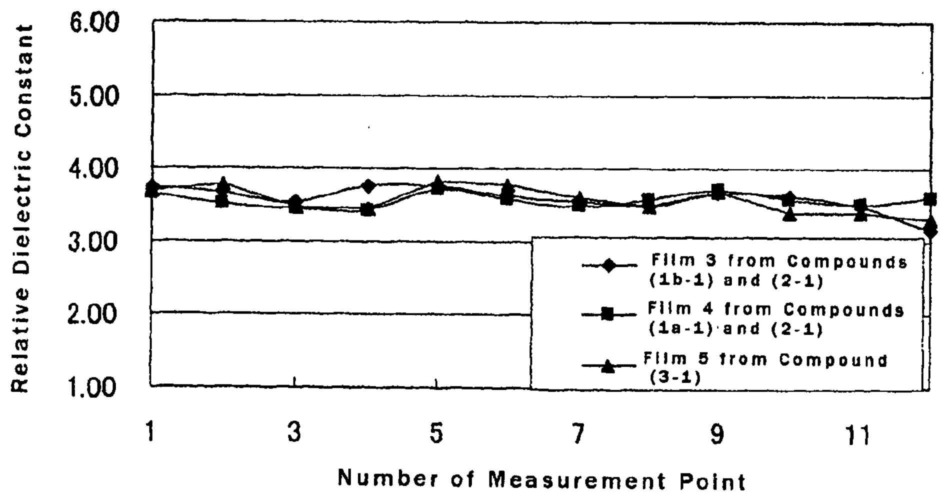

# Figure 11

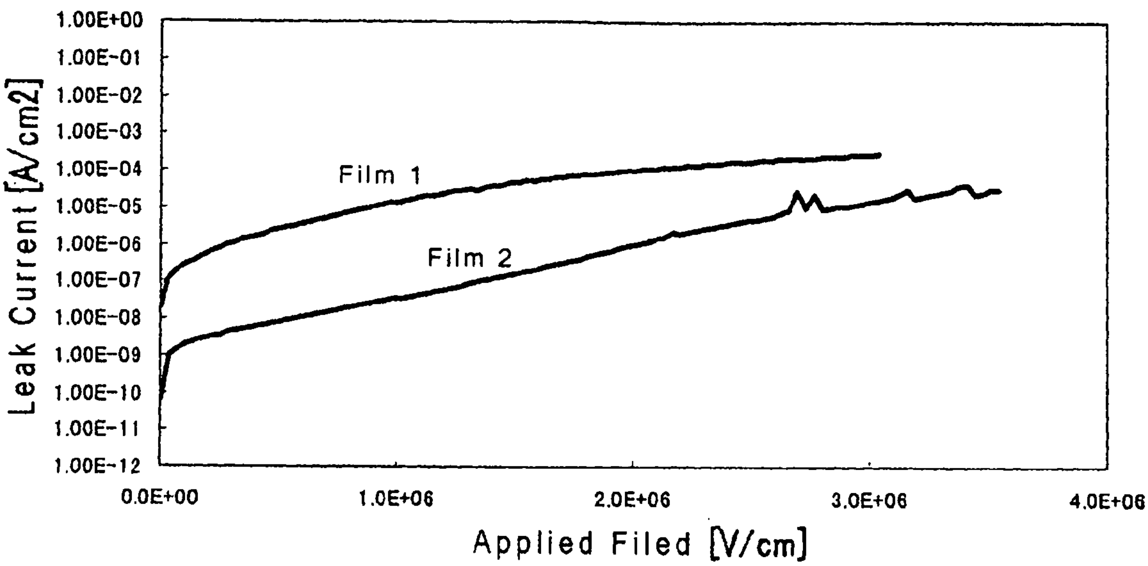
1.00E+00
1.00E-01
1.00E-02
1.00E-03
1.00E-04
1.00E-05
1.00E-06
1.00E-07
1.00E-08
1.00E-09
1.00E-10
1.00E-11
1.00E-12
Leak Current [A/cm2]
Film 1
Film 2
0.0E+00
1.0E+06
2.0E+06
3.0E+06
4.0E+06
Applied Filed [V/cm]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004307804 A